# EUROPEAN PATENT APPLICATION

(11) **EP 2 002 827 A2**
(43) Date of publication of application: **17.12.2008**
(21) Application number: 08008841.2
(22) Date of filing: 31.10.2002
(51) Int. Cl.: A61K 31/00, A61K 31/138, A61P 19/08, A61P 19/10

(54) **Methods and compositions for control of bone formation via modulation of sympathetic tone**

(30) Priority: 05.12.2001 US 337054 P
(62) Divisional of application: 02784350.7
(71) Applicant: THE BAYLOR COLLEGE OF MEDICINE, Houston, TX 77030-3498 (US)
(72) Inventor: Karsenty, Gerard, Houston, TX 77030 (US); Takeda, Shu, Kawasaki-shi, Kanagawa-ken 216-0004 (JP); Elefteriou, Florent, Houston, TX 77030 (US)
(74) Representative: Vossius & Partner

(57) **Abstract**

The invention relates to the use of a β adrenergic antagonist and a leptin antagonist for the preparation of a pharmaceutical composition for treating or preventing a symptom of osteoporosis.

SEQUENCE LISTING

<110> Baylor College of Medicine

<120> METHODS AND COMPOSITIONS FOR CONTROL OF BONE FORMATION VIA
MODULATION OF SYMPATHETIC TONE

<130> 9142-022-228

<140> TBA

<141> 2002-10-31

<150> 60/337,054

<151> 2001-12-05

<160> 20

<170> PatentIn version 3.0

<210> 1

<211> 17

<212> DNA

<213> Homo sapiens

<400> 1
catcttactt cagagaa 17

<210> 2

<211> 24

<212> DNA

<213> Homo sapiens

<400> 2
catcttactt cagagaaagt acac 24

<210> 3

<211> 29

<212> DNA

<213> Homo sapiens

<400> 3
catcttactt cagagaagta cacccataa 29

<210> 4

<211> 35

<212> DNA

<213> Homo sapiens

<400> 4
catcttactt cagagaagta cacccataat cctct 35

<210> 5

<211> 35

<212> DNA

<213> Homo sapiens

<400> 5
aatcatctta cttcagagaa gtacacccat aatcc 35

<210> 6

<211> 29

<212> DNA

<213> Homo sapiens

<400> 6
cttacttcag agaagtacac ccataatcc 29

<210> 7

<211> 23

<212> DNA

<213>  Homo sapiens

<400>  7
tcagagaagt acacccataa tcc                                              23

<210>  8

<211>  17

<212>  DNA

<213>  Homo sapiens

<400>  8
aagtacaccc ataatcc                                                     17

<210>  9

<211>  56

<212>  RNA

<213>  Homo sapiens

<220>

<221>  misc_feature

<223>  n = a, u, g, or c

<400>  9
acagaauuuu ugacaaauca aagcagannn nucugagnag uccuuacuuc agagaa          56

<210>  10

<211>  57

<212>  RNA

<213>  Homo sapiens

<220>

<221>  misc_feature

<223>  n = a, u, g, or c

<400>  10

ggcccgggca gccugcccaa agccggnnnn ccggagnagu cgccagaccg gcucgug 57

<210> 11

<211> 56

<212> RNA

<213> Homo sapiens

<220>

<221> misc_feature

<223> n = a, u, g, or c

<400> 11
uggcaugcaa gacaaagcag gnnnnccuga gnaguccuua aaucuccaag gaguaa 56

<210> 12

<211> 50

<212> RNA

<213> Homo sapiens

<220>

<221> misc_feature

<223> n = a, u, g, or c

<400> 12
uauaugacaa agcugunnnn acagagnagu ccuugugugg uaaagacacg 50

<210> 13

<211> 61

<212> RNA

<213> Homo sapiens

<220>

<221> misc_feature

<223> n = a, u, g, or c

<400> 13
agcaccaauu gaauugaugg ccaaagcggg nnnncccgag nagucaaccg uaacaguaug 60

u 61

<210> 14

<211> 69

<212> RNA

<213> Homo sapiens

<220>

<221> misc_feature

<223> n = a, u, g, or c

<400> 14
ugaaauuguu ucaggcucca aagccggnnn nccggagnag ucaagaagag gaccacaugu 60

cacugaugc 69

<210> 15

<211> 61

<212> RNA

<213> Homo sapiens

<220>

<221> misc_feature

<223> n = a, u, g, or c

<400> 15
gguuucuuca gugaaauuac acaaagcagc nnnngcugag nagucaguua ggucacacau 60

c 61

<210> 16

<211> 53

<212> RNA

<213> Homo sapiens

<220>

<221>  misc_feature

<223>  n = a, u, g or c


<400>  16
acccauuaua acacaaagcu gannnnucag agnagucauc ugaagguuuc uuc          53


<210>  17

<211>  21

<212>  DNA

<213>  Homo sapiens


<400>  17
tggataaacc cttgctcttc a                                             21


<210>  18

<211>  23

<212>  DNA

<213>  Homo sapiens


<400>  18
acactgttaa tttcacacca gag                                           23


<210>  19

<211>  20

<212>  DNA

<213>  Homo sapiens


<400>  19
gttgagagat catctccacc                                               20


<210>  20

<211>  20

<212>  DNA

<213>  Homo sapiens

<400> 20
agcgatgatg aaccaggtta 20

## Description

This invention was made with government support under grant numbers DK58882 and NASA NCC9-58 awarded by the National Institutes of Health. The government may have certain rights in the invention. This application claims the benefit of U.S. Provisional Application No. 60/337,054, filed December 5, 2001, which is incorporated herein by reference in its entirety.

### 1. INTRODUCTION

The present invention relates to compositions and methods for the treatment, diagnosis and prevention of conditions, disorders or diseases involving bone, including, but not limited to, osteoporosis. The invention relates to modulation of the receptor signaling pathway for the polypeptide hormone leptin. More particularly the present invention relates to the modulation of leptin synthesis, leptin receptor synthesis, leptin binding to its receptor, and leptin signaling to bone cells. The invention also relates to the modulation of bone homeostasis via sympathetic nervous system pathways.

The present invention also provides methods for the identification and prophylactic or therapeutic use of compounds in the treatment, prognosis and diagnosis of conditions, disorders, or diseases involving bone. Additionally, methods are provided for the diagnostic monitoring of patients undergoing clinical evaluation for the treatment of conditions or disorders involving bone, for monitoring the efficacy of compounds in clinical trials and for identifying subjects who may be predisposed to such conditions, disorders, or diseases involving bone.

### 2. BACKGROUND OF THE INVENTION

The physiological process of bone remodeling allows constant renewal of bone through two well-defined sequential cellular processes (Karsenty, 1999, Genes and Development, 13:3037-3051). The initial event is resorption of preexisting bone by the osteoclasts, followed by de novo bone formation by the osteoblasts. These two processes in bone remodeling must maintain equilibrium of bone mass within narrow limits between the end of puberty and the arrest of gonadal function. The molecular mechanisms responsible for maintaining a constant bone mass are unknown, yet several lines of evidence suggest that this may be achieved, at least in part, through a complex endocrine regulation. For example, gonadal failure and the concomitant deficiency of the sex steroids stimulates the bone resorption process of bone remodeling and eventually leads to osteopenia (low bone mass) or osteoporosis (low bone mass and high susceptibility to fractures). Likewise, the recent identification of osteoprotegerin in serum and its functional characterization through a systemic route is another indication that secreted molecules affect osteoclastic bone resorption (Simonet et al., 1997, Cell, 89:309-319). This systemic control of bone resorption suggests that other circulating molecules, yet to be identified, could control bone formation via the osteoblasts. The identification of these hormones or growth factors, if they exist, is of paramount importance given the incidence and morbidity of diseases affecting bone remodeling.

One such disease is osteoporosis (Riggs et al., 1998, J. Bone Miner. Res., 13:763-773). Osteoporosis is the most common disorder affecting bone remodeling and the most prevalent-disease in the Western hemisphere. At the physiopathological level, hallmarks of the disease are that bones exhibit a lowered mass, that is, are less dense and, thus, subject to fractures. In addition, the onset of osteoporosis in both sexes is intimately linked to arrest of gonadal function and is rarely observed in obese individuals. At the cellular level, osteoporosis is characterized by a loss in equilibrium of bone remodeling favoring bone resorption over bone formation, which leads to the lowered bone mass and increased bone fractures. At the molecular level, the pathogenesis of osteoporosis remains largely unknown.

Complex anabolic and catabolic effects on bone by β adrenergic receptor activation and inhibition have been described. The anabolic and catabolic effects on bone resulting from activating β adrenergic receptors have been reporter. Potentially conflicting results have been described but may be context-dependent results and may result from the parathyroid hormone (Kellenberger et al., 1998, Bone 22(5):471-478). Further, not only have anabolic effects been reported for β agonists, but also anabolic effects have been reported for β antagonists. Anabolic effects of β agonists formoterol and salbutamol have been observed in bone in an ovariectomized rat (Pataki et al., 1996, Bone 19(3), Supplement 129S-169S). Furthermore, a β₃ antagonist, BRL 35135, has been described to reduce the sizes of bone marrow adipocytes in the lumbar spine, but not in the tibia (Kurabayashi et al., 2001, Calcif Tissue Int 68:248-254). A direct relationship between the effects of clenbuterol, a β₂ agonist, on muscle and bone mass, under conditions of reduced work load was demonstrated and postulated to result from changes in muscle mass caused by clenbuterol, which results in the reduction of bone mineralization due to inactivity (Zeman et al., 1991, Am J. Physiol Endocrin Metab 261:E285-E289). Increased femoral metaphyseal mineral apposition rates were observed in rats treated with propranolol, a nonspecific β blocker, suggesting that propranolol may stimulate bone metabolism or affect osteoblasts through β adrenergic or membrane-stabilizing mechanisms (Minkowitz et al., 1991, J. Orthop Res 9:869-875). Thus, it is unclear what the effects of β agonists and/or antagonists are on bone type and whether the effect of the β agonist and/or antagonist is direct or indirect.

Leptin signaling in the hypothalamus can stimulate sympathetic nerve activity to some tissues, particularly brown adipose tissue, kidney, hindlimb, adrenal gland (Haynes et al., 1997, J. Clin. Investigation 100(2):270-278). The functional outcome of the increased sympathetic activity to brown adipose tissue is increased thermogenic activity, as well as increased gene expression of UCP1 (uncoupling protein 1) and leptin itself (Commins et al., 1999, Endocrinology 140(10):4772-4778). There is also some evidence that leptin may regulate pancreatic endocrine function via sympathetic nerve stimulation (Mizuno et al., 1998, Endocrinology 139(9):3863-3870). Other papers have shown that mammals deficient in leptin expression have decreased sympathetic nerve activity and endocrine defects, and that in some instances, adrenergic agonists are capable of suppressing weight gain in these animals (Commins et al., 2000, J. Biol. Chem. 275(42):33059-33067 and Ozata et al., 1999, J Clin Endocrinol Metab 84(10):3686-95).

### 3. SUMMARY OF THE INVENTION

An object of the present invention is the treatment, diagnosis and/or prevention of bone disease through manipulation of sympathetic nervous system pathways. In accordance with one aspect of the present invention, there is a method for treating or preventing one or more symptoms of osteoporosis comprising the administration of β adrenergic antagonists. A specific embodiment of this aspect includes, but is not limited to, the further administration of a leptin antagonist in combination with β adrenergic antagonists. In another embodiment, there is a method for treating or preventing one or more symptoms of osteopetrosis or osteosclerosis comprising the administration of β adrenergic agonists. A further embodiment includes, but is not limited to, the further administration of a leptin agonist in combination with β adrenergic agonists.

Another object of the present invention relates to a method of modulating leptin effects on bone comprising the administration of a therapeutically effective amount of a pharmaceutical composition that alters the sympathetic tone. Another embodiment of the present invention relates to a method of modulating bone mass comprising the administration of a therapeutically effective amount of a pharmaceutical composition that alters sympathetic tone so that bone mass is modulated. Specific embodiments of the pharmaceutical composition include, but are not limited to, leptin antagonists, leptin agonists, sympathetic nervous system antagonists, sympathetic nervous system agonists, and combinations thereof.

Another object of the present invention relates to methods of treating or preventing one or more symptoms of bone disease comprising the administration of a therapeutically effective amount of a pharmaceutical composition that modulates leptin effects in bone by altering sympathetic tone. Specific embodiments of the pharmaceutical composition include, but are not limited to, leptin antagonists, leptin agonists, sympathetic nervous system antagonists, sympathetic nervous system agonists, and combinations thereof.

Another object of the present invention is the diagnosis or prognosis of a bone disease in a mammal comprising:
(a) measuring sympathetic tone and leptin activity in the mammal suspected of having the bone disease and
(b) comparing values measured in step (a) with corresponding control values.

Another object of the present invention encompasses pharmaceutical compositions comprising any combination of leptin antagonists, leptin agonists, sympathetic nervous system antagonists, sympathetic nervous system agonists. Examples of combinations include, but are not limited to, a leptin antagonist and a sympathetic nervous system antagonist, a leptin agonist and a sympathetic nervous system agonist, a leptin antagonist and a sympathetic nervous system agonist, and a leptin agonist and a sympathetic system antagonist. In another embodiment, the composition further comprises a pharmaceutically acceptable carrier.

Another object of the present invention is the treatment, diagnosis and/or prevention of bone disease through manipulation of the leptin signaling pathway. Bone diseases which can be treated and/or prevented in accordance with the present invention include bone diseases characterized by a decreased bone mass relative to that of corresponding non-diseased bone, including, but not limited to osteoporosis, osteopenia and Paget's disease. Bone diseases which can be treated and/or prevented in accordance with the present invention also include bone diseases characterized by an increased bone mass relative to that of corresponding non-diseased bone, including, but not limited to osteopetrosis, osteosclerosis, osteochondrosis, and pynchodisostosis.

Thus, in accordance with one aspect of the present invention, there is a method of treating a bone disease comprising: administering to a mammal in need of said treatment a therapeutically effective amount of a compound that lowers leptin level in blood serum, wherein the bone disease is characterized by a decreased bone mass relative to that of corresponding non-diseased bone. Specific embodiments of some of these compounds and methods include, but are not limited to ones that inhibit or lower leptin synthesis or increase leptin breakdown. Among such compounds are antisense, ribozyme or triple helix sequences of a leptin-encoding polypeptide.

In accordance with another aspect of the present invention, there is a method of treating a bone disease comprising: administering to a mammal in need of said treatment a therapeutically effective amount of a compound that lowers leptin level in cerebrospinal fluid, wherein the bone disease is characterized by a decreased bone mass relative to that of corresponding non-diseased bone. Specific embodiments of some of these compounds and methods include, but are not limited to ones that inhibit or lower leptin synthesis or increase leptin breakdown, and compounds that bind leptin in blood.

Particular embodiments of the methods of the invention include, for example, a method of treating a bone disease comprising: administering to a mammal in need of said treatment a therapeutically effective amount of a compound, wherein the bone disease is characterized by a decreased bone mass relative to that of corresponding non-diseased bone, and wherein the compound is selected from the group consisting of compounds which bind leptin in blood, including, but not limited to such compounds as an antibody which specifically binds leptin, a soluble leptin receptor polypeptide, an inter-alpha-trypsin inhibitor heavy chain related protein and an alpha 2-macroglobulin protein.

In accordance with another aspect of the present invention, there is a method of treating a bone disease comprising: administering to a mammal in need of said treatment a therapeutically effective amount of a compound that lowers the level of phosphorylated Stat3. polypeptide, wherein the bone disease is characterized by a decreased bone mass relative to that of corresponding non-diseased bone. Specific embodiments of some of these compounds and methods include, but are not limited to ones that inhibit or lower leptin synthesis or increase leptin breakdown, compounds that bind leptin in blood, and leptin receptor antagonist compounds, such as acetylphenol compounds, antibodies which specifically bind leptin, antibodies which specifically bind leptin receptor, and compounds that comprise soluble leptin receptor polypeptide sequences.

In accordance with another aspect of the present invention, there is a method of treating or preventing a bone disease comprising: administering to a mammal in need of said treatment or prevention a therapeutically effective amount of a compound that inhibits the activity of dopamine β hydroxylase ("DBH"), the enzyme necessary for converting dopamine to norepinephrine. Specific embodiments of some of these compounds and methods include, but are not limited to ones that inhibit DBH enzyme activity, DBH gene expression, antibodies which specifically bind DBH, and compounds which are involved in the norephinephrine synthesis pathway. In another embodiment, a DBH inhibitor can be administered together with another antagonist or agonist *(e.g.,* a β adrenergic antagonist and/or a leptin antagonist).

In accordance with another aspect of the present invention, there is a method of treating a bone disease comprising: administering to a mammal in need of said treatment a therapeutically effective amount of a compound that lowers leptin receptor levels in hypothalamus, wherein the bone disease is characterized by a decreased bone mass relative to that of corresponding non-diseased bone. Specific embodiments of some of these compounds and methods include, but are not limited to ones that inhibit or lower leptin receptor synthesis or increase leptin receptor breakdown. Among such compounds are antisense, ribozyme or triple helix sequences of a leptin receptor-encoding polypeptide.

In accordance with yet another aspect of the present invention, there is a method of treating a bone disease comprising: administering to a mammal in need of said treatment a therapeutically effective amount of a compound that increases leptin level in blood serum and/or cerebrospinal fluid, wherein the bone disease is characterized by a increased bone mass relative to that of corresponding non-diseased bone. Specific embodiments of some of these compounds and methods include, but are not limited to ones that increase or induce leptin synthesis or decrease leptin breakdown.

In accordance with another aspect of the present invention, there is a method of treating a bone disease comprising: administering to a mammal in need of said treatment a therapeutically effective amount of a compound that increases the level of phosphorylated Stat3 polypeptide, wherein the bone disease is characterized by a increased bone mass relative to that of corresponding non-diseased bone. Specific embodiments of some of these compounds and methods include, but are not limited to ones that increase or induce leptin synthesis or decrease leptin breakdown, and leptin receptor agonist compounds.

In accordance with another aspect of the present invention, there is a method of treating a bone disease comprising: administering to a mammal in need of said treatment a therapeutically effective amount of a compound that increases leptin receptor levels in hypothalamus, wherein the bone disease is characterized by a increased bone mass relative to that of corresponding non-diseased bone. Specific embodiments of some of these compounds and methods include, but are not limited to ones that increase or induce leptin receptor synthesis or decrease leptin receptor breakdown.

In accordance with yet another aspect of the present invention, there is a method of preventing a bone disease comprising: administering to a mammal at risk for the disease a compound that lowers leptin level in blood serum, at a concentration sufficient to prevent the bone disease, wherein the bone disease is characterized by a decreased bone mass relative to that of corresponding non-diseased bone. Specific embodiments of some of these compounds and methods include, but are not limited to ones that inhibit or lower leptin synthesis or increase leptin breakdown. Among such compounds are antisense, ribozyme or triple helix sequences of a leptin-encoding polypeptide.

In accordance with another aspect of the present invention, there is a method of preventing a bone disease comprising: administering to a mammal at risk for the bone disease a compound that lowers leptin level in cerebrospinal fluid, at a concentration sufficient to prevent the bone disease, wherein the bone disease is characterized by a decreased bone mass relative to that of corresponding non-diseased bone. Specific embodiments of some of these compounds and methods include, but are not limited to ones that inhibit or lower leptin synthesis or increase leptin breakdown, and compounds that bind leptin in blood.

Particular embodiments of the methods of the invention include, for example, a method of preventing a bone disease comprising: administering to a mammal at risk for the bone disease a compound at a concentration sufficient to prevent the bone disease, wherein the bone disease is characterized by a decreased bone mass relative to that of corresponding non-diseased bone, and wherein the compound is selected from the group consisting of compounds which bind leptin in blood, including, but not limited to such compounds as an antibody which specifically binds leptin, a soluble leptin receptor polypeptide, an inter-alpha-trypsin inhibitor heavy chain related protein and an alpha 2-macroglobulin protein.

In accordance with another aspect of the present invention, there is a method of preventing a bone disease comprising: administering to a mammal at risk for the bone disease a compound that lowers the level of phosphorylated Stat3 polypeptide, at a concentration sufficient to prevent the bone disease, wherein the bone disease is characterized by a decreased bone mass relative to that of corresponding non-diseased bone. Specific embodiments of some of these compounds and methods include, but are not limited to ones that inhibit or lower leptin synthesis or increase leptin breakdown, compounds that bind leptin in blood, and leptin receptor antagonist compounds, such as acetylphenol compounds, antibodies which specifically bind leptin, antibodies which specifically bind leptin receptor, and compounds that comprise soluble leptin receptor polypeptide sequences.

In accordance with another aspect of the present invention, there is a method of preventing a bone disease comprising: administering to a mammal at risk for the bone disease a compound that lowers leptin receptor levels in hypothalamus, wherein the bone disease is characterized by a decreased bone mass relative to that of corresponding non-diseased bone. Specific embodiments of some of these compounds and methods include, but are not limited to ones that inhibit or lower leptin receptor synthesis or increase leptin receptor breakdown. Among such compounds are antisense, ribozyme or triple helix sequences of a leptin receptor-encoding polypeptide.

In accordance with yet another aspect of the present invention, there is a method of preventing a bone disease comprising: administering to a mammal at risk for the bone disease a compound that increases leptin level in blood serum and/or cerebrospinal fluid, at a concentration sufficient to prevent the bone disease, wherein the bone disease is characterized by a increased bone mass relative to that of corresponding non-diseased bone. Specific embodiments of some of these compounds and methods include, but are not limited to ones that increase or induce leptin synthesis or decrease leptin breakdown.

In accordance with another aspect of the present invention, there is a method of preventing a bone disease comprising: administering to a mammal at risk for the bone disease a compound that increases the level of phosphorylated Stat3 polypeptide, at a concentration sufficient to prevent the bone disease, wherein the bone disease is characterized by a increased bone mass relative to that of corresponding non-diseased bone. Specific embodiments of some of these compounds and methods include, but are not limited to ones that increase or induce leptin synthesis or decrease leptin breakdown, and leptin receptor agonist compounds.

In accordance with another aspect of the present invention, there is a method of preventing a bone disease comprising: administering to a mammal at risk for the disease a compound that increases leptin receptor levels in hypothalamus, at a concentration sufficient to prevent the bone disease, wherein the bone disease is characterized by a increased bone mass relative to that of corresponding non-diseased bone. Specific embodiments of some of these compounds and methods include, but are not limited to ones that increase or induce leptin receptor synthesis or decrease leptin receptor breakdown.

In accordance with yet another aspect of the present invention, there is a method of diagnosing or prognosing a bone disease in a mammal, such as a human, comprising:
(a) measuring leptin levels in blood serum of a mammal, e.g., a mammal suspected of exhibiting or being at risk for the bone disease; and
(b) comparing the level measured in (a) to the leptin level in control blood serum, so that if the level obtained in (a) is higher than that of the control, the mammal is diagnosed or prognosed as exhibiting or being at risk for the bone disease, wherein the bone disease is characterized by a decreased bone mass relative to that of corresponding non-diseased bone.

In accordance with another aspect of the present invention, there is a method of diagnosing or prognosing a bone disease in a mammal, such as a human, comprising:
(a) measuring leptin levels in cerebrospinal fluid of a mammal, e.g., a mammal suspected of exhibiting or being at risk for the bone disease; and
(b) comparing the level measured in (a) to the leptin level in control cerebrospinal fluid,
so that if the level obtained in (a) is higher than that of the control, the mammal is diagnosed as exhibiting or being at risk for the bone disease, wherein the bone disease is characterized by a decreased bone mass relative to that of corresponding non-diseased bone.

In accordance with yet another aspect of the present invention, there is a method of diagnosing or prognosing a bone disease in a mammal, such as a human, comprising:
(a) measuring leptin levels in blood serum of a mammal, e.g., a mammal suspected of exhibiting or being at risk for the bone disease; and
(b) comparing the level measured in (a) to the leptin level in control blood serum,
so that if the level obtained in (a) is lower than that of the control, the mammal is diagnosed as exhibiting or being at risk for the bone disease, wherein the bone disease is characterized by an increased bone mass relative to that of corresponding non-diseased bone.

In accordance with another aspect of the present invention, there is a method of diagnosing or prognosing a bone disease in a mammal, such as a human, comprising:
(a) measuring leptin levels in cerebrospinal fluid of a mammal, e.g., a mammal suspected of exhibiting or being at risk for the bone disease; and
(b) comparing the level measured in (a) to the leptin level in control cerebrospinal fluid,
so that if the level obtained in (a) is lower than that of the control, the mammal is diagnosed as exhibiting or being at risk for the bone disease, wherein the bone disease is characterized by an increased bone mass relative to that of corresponding non-diseased bone.

In accordance with yet another aspect of the present invention, there is a method of monitoring efficacy of a compound for treating a bone disease in a mammal, such as a human, comprising:
(a) administering the compound to a mammal;
(b) measuring leptin levels in blood serum of the mammal; and
(c) comparing the level measured in (b) to the leptin level in blood serum of the mammal prior to administering the compound,
thereby monitoring the efficacy of the compound, wherein the bone disease is characterized by a decreased bone mass relative to that of corresponding non-diseased bone.

In accordance with another aspect of the present invention, there is a method of monitoring efficacy of a compound for treating a bone disease in a mammal, such as a human, comprising:
(a) administering the compound to a mammal;
(b) measuring leptin levels in cerebrospinal fluid of the mammal; and
(c) comparing the level measured in (b) to the leptin level in cerebrospinal fluid of the mammal prior to administering the compound,
thereby monitoring the efficacy of the compound, wherein the bone disease is characterized by a decreased bone mass relative to that of corresponding non-diseased bone.

In accordance with yet another aspect of the present invention, there is a method of monitoring efficacy of a compound for treating a bone disease in a mammal, such as a human, comprising:
(a) administering the compound to a mammal;
(b) measuring leptin levels in blood serum of the mammal; and
(c) comparing the level measured in (b) to the leptin level in blood serum of the mammal prior to administering the compound,
thereby monitoring the efficacy of the compound, wherein the bone disease is characterized by a increased bone mass relative to that of corresponding non-diseased bone.

In accordance with another aspect of the present invention, there is a method of monitoring efficacy of a compound for treating a bone disease in a mammal, such as a human, comprising:
(a) administering the compound to a mammal;
(b) measuring leptin levels in cerebrospinal fluid of the mammal; and
(c) comparing the level measured in (b) to the leptin level in cerebrospinal fluid of the mammal prior to administering the compound,
thereby monitoring the efficacy of the compound, wherein the bone disease is characterized by a increased bone mass relative to that of corresponding non-diseased bone.

In accordance with another aspect of the present invention, there is a method for identifying a compound to be tested for an ability to modulate (increase or decrease) bone mass in a mammal, comprising:
(a) contacting a test compound with a polypeptide; and
(b) determining whether the test compound binds the polypeptide, so that if the test compound binds the polypeptide, then a compound to be tested for an ability to modulate bone mass is identified, wherein the polypeptide is selected from the group consisting of a leptin polypeptide, leptin receptor polypeptide, and an adrenergic receptor polypeptide.

In accordance with another aspect of the present invention, there is a method for identifying a compound that modulates (increases or decreases) bone mass in a mammal, comprising:
(a) contacting test compounds with a polypeptide;
(b) identifying a test compound that binds the polypeptide; and
(c) administering the test compound in (b) to a non-human mammal, and determining whether the test compound modulates bone mass in the mammal relative to that of a corresponding bone in an untreated control non-human mammal,
wherein the polypeptide is selected from the group consisting of a leptin polypeptide, a leptin receptor polypeptide, and an adrenergic receptor polypeptide so that if the test compound modulates bone mass, then a compound that modulates bone mass in a mammal is identified.

In accordance with yet another aspect of the present invention, there is a method for identifying a compound to be tested for an ability to modulate (increase or decrease) bone mass in a mammal, comprising:
(a) contacting a test compound with a leptin polypeptide and a leptin receptor polypeptide for a time sufficient to form leptin/leptin receptor complexes or catecholamine/adrenergic receptor complexes; and
(b) measuring leptin/leptin receptor or catecholamine/adrenergic receptor complex level, so that if the level measured differs from that measured in the absence of the test compound, then a compound to be tested for an ability to modulate bone mass is identified.

In accordance with another aspect of the present invention, there is a method for identifying a compound to be tested for an ability to decrease bone mass in a mammal, comprising:
(a) contacting a test compound with a cell which expresses a functional leptin receptor or a functional adrenergic receptor; and
(b) determining whether the test compound activates the leptin receptor or adrenergic receptor,
wherein if the compound activates the leptin receptor or the adrenergic receptor a compound to be tested for an ability to decrease bone mass in a mammal is identified.

In accordance with another aspect of the present invention, there is a method for identifying a compound that decreases bone mass in a mammal, comprising:
(a) contacting a test compound with a cell that expresses a functional leptin receptor or adrenergic receptor, and determining whether the test compound activates the leptin receptor or adrenergic receptor;
(b) administering a test compound identified in (a) as activating the leptin receptor or adrenergic receptor to a non-human animal, and determining whether the test compound decreases bone mass of the animal relative to that of a corresponding bone of a control non-human animal, so that if the test compound decreases bone mass, then a compound that decreases bone mass in a mammal is identified.

In accordance with another aspect of the present invention, there is a method for identifying a compound to be tested for an ability to increase bone mass in a mammal, comprising:
(a) contacting a leptin polypeptide and a test compound with a cell that expresses a functional leptin receptor; and
(b) determining whether the test compound lowers activation of the leptin receptor relative to that observed in the absence of the test compound; wherein a test compounds that lowers activation of the leptin receptor is identified as a compound to be tested for an ability to increase bone mass in a mammal.

In accordance with another aspect of the present invention, there is a method for identifying a compound to be tested for an ability to increase bone mass in a mammal, comprising:
(a) contacting a catecholamine and a test compound with a cell that expresses a functional adrenergic receptor; and
(b) determining whether the test compound lowers activation of the adrenergic receptor relative to that observed in the absence of the test compound; wherein a test compounds that lowers activation of the adrenergic receptor is identified as a compound to be tested for an ability to increase bone mass in a mammal.

In accordance with yet another aspect of the present invention, there is a method for identifying a compound that increases bone mass in a mammal, comprising:
(a) contacting a leptin polypeptide and a test compound with a cell that expresses a functional leptin receptor, and determining whether the test compound decreases activation of the leptin receptor;
(b) administering a test compound identified in (a) as decreasing leptin receptor to a non-human animal, and determining whether the test compound increases bone mass of the animal relative to that of a corresponding bone of a control non-human animal, so that if the test compound increases bone mass, then a compound that increases bone mass in a mammal is identified.

In accordance with yet another aspect of the present invention, there is a method for identifying a compound that increases bone mass in a mammal, comprising:
(a) contacting a catecholamine and a test compound with a cell that expresses a functional adrenergic receptor, and determining whether the test compound decreases activation of the adrenergic receptor;
(b) administering a test compound identified in (a) as decreasing adrenergic receptor to a non-human animal, and determining whether the test compound increases bone mass of the animal relative to that of a corresponding bone of a control non-human animal, so that if the test compound increases bone mass, then a compound that increases bone mass in a mammal is identified.

The present invention also provides pharmaceutical compositions which can be used to treat and/or prevent bone diseases.

Other and further objects, features and advantages would be apparent and eventually more readily understood by reading the following specification and by reference to the accompanying drawings forming a part thereof, or any examples of the presently preferred embodiments of the invention are given for the purpose of the disclosure.

### 3.1. Terminology

The following terms used herein shall have the meaning indicated:
Leptin, ("Ob") as used herein, is defined by the endogenous polypeptide product of an *ob* gene, preferably a human ob gene, of which the known activities are mediated through the hypothalamus.
Leptin receptor ("ObR"), as used herein, is defined by the receptor through which the leptin hormone binds to generate its signal; preferably, this term refers to a human leptin receptor.
Catecholamines, as used herein, is defined as being an endogenous amine-containing derivatives of catechol, 1,2-dihydroxybenzene, preferably norepinephrine and epinephrine.
Adrenergic receptor ("AR"), as used herein, is defined by the receptor through which catecholamines bind to generate its signal; preferably, this term refers to a human adrenergic receptor.
NPY, as used herein, is defined as neuropeptide Y, preferably human neuropeptide Y. Neuropeptide Y (NPY) is a member of the pancreatic polypeptide family. It is to be understood that the term NPY, as used herein is intended to encompass not only neuropeptide Y but also its peptide relatives in the pancreatic polypeptide family, e.g., peptide YY (PYY), and pancreatic polypeptide (PP).
Neuropeptide Y receptor ("NPY receptor" or "NPY-R"), as used herein, is defined as a receptor, preferably a human receptor, that binds endogenous NPY under physiological conditions. NPY receptors are G protein-coupled receptors including, but not limited to subtypes known as Y1, Y2, Y3, Y4, or Y5 (or PP).
Ciliary neurotrophic factor ("CNTF"), as used herein, is defined by the endogenous polypeptide product of a CNTF gene, preferably a human CNTF gene, of which the known activities are mediated through the central and peripheral (including autonomous) nervous system.
CNTF receptor, as used herein, is defined by the receptor through which CNTF binds to generate its signal; preferably, this term refers to a human CNTF receptor. In a specific embodiment, the CNTF receptor comprises a multimeric complex of gp130, LIFR, CNTFRα, and any other molecule necessary to transduce the CNTF signal. In an alternate embodiment, the CNTF receptor comprises multimeric complexes of analogs of gp130, LIFR and CNTFRα. Such analogs are known to those of skill in the art to contribute to CNTF receptor function. In one such embodiment, the CNTF receptor specifically comprises a multimeric complex of gp 130, LIFR and sCNTFRα.
CNTF receptor polypeptides, as used herein, are defined as polypeptide components of the CNTF receptor. In specific embodiments of the invention, CNTF receptor polypeptides include gp130, LIFR, CNTFRα and any other polypeptide necessary to transduce the CNTF signal, and functional analogs thereof. Such analogs, including sCNTFRα, are known to those of skill in the art to participate in CNTF signal transduction.
CNTF receptor polynucleotides, as used herein, are defined as polynucleotides encoding polypeptide components of the CNTF receptor. In specific embodiments, CNTF receptor polynucleotides include polynucleotides that encode gp 130, polynucleotides that encode LIFR, polynucleotides that encode CNTFRα and polynucleotides that encode any other component of the CNTF receptor.
A CNTF receptor gene, as used herein is defined as a gene coding for a polypeptide component of the CNTF receptor such as, for example, a gene encoding gp130, LIFR, CNTFRα or any other component of the CNTF receptor.
Soluble CNTFRα ("sCNTFRα"), as used herein, is a molecule comprising the polypeptide component of CNTFRα which is soluble in an extracellular milieu. Soluble CNTFRα typically lacks the GPI membrane anchor of CNTFRα.
Bone disease, as used herein, refers to any bone disease or state which results in or is characterized by loss of health or integrity to bone and includes, but is not limited to, osteoporosis, osteopenia, faulty bone formation or resorption, Paget's disease, fractures and broken bones, bone metastasis, osteopetrosis, osteosclerosis and osteochondrosis. More particularly, bone diseases which can be treated and/or prevented in accordance with the present invention include bone diseases characterized by a decreased bone mass relative to that of corresponding non-diseased bone (e.g., osteoporosis, osteopenia and Paget's disease), and bone diseases characterized by an increased bone mass relative to that of corresponding non-diseased bone *(e.g.,* osteopetrosis, osteosclerosis and osteochondrosis). Prevention of bone disease includes actively intervening as described herein prior to onset to prevent the disease. Treatment of bone disease encompasses actively intervening after onset to slow down, ameliorate symptoms of, or reverse the disease or situation. More specifically, treating, as used herein, refers to a method that modulates bone mass to more closely resemble that of corresponding non-diseased bone (that is a corresponding bone of the same type, *e.g.,* long, vertebral, etc.) in a non-diseased state.
Leptin receptor antagonist, as used herein, refers to a factor which neutralizes or impedes or otherwise reduces the action or effect of a leptin receptor. Such antagonists can include compounds that bind leptin or that bind leptin receptor. Such antagonists can also include compounds that neutralize, impede or otherwise reduce leptin receptor output, that is, intracellular steps in the leptin signaling pathway following binding of leptin to the leptin receptor, *i.e.,* downstream events that affect leptin/leptin receptor signaling, that do not occur at the receptor/ligand interaction level. Leptin receptor antagonists may include, but are not limited to proteins, antibodies, small organic molecules or carbohydrates, such as, for example, acetylphenol compounds, antibodies which specifically bind leptin, antibodies which specifically bind leptin receptor, and compounds that comprise soluble leptin receptor polypeptide sequences.
Leptin receptor agonist, as used herein, refers to a factor which activates, induces or otherwise increases the action or effect of a leptin receptor. Such agonists can include compounds that bind leptin or that bind leptin receptor. Such antagonists can also include compounds that activate, induce or otherwise increase leptin receptor output, that is, intracellular steps in the leptin signaling pathway following binding of leptin to the leptin receptor, *i.e.,* downstream events that affect leptin/leptin receptor signaling, that do not occur at the receptor/ligand interaction level. Leptin receptor agonists may include, but are not limited to proteins, antibodies, small organic molecules or carbohydrates, such as, for example, leptin, leptin analogs, and antibodies which specifically bind and activate leptin.
An agent is said to be administered in a "therapeutically effective amount" if the amount administered results in a desired change in the physiology of a recipient mammal, e.g., results in an increase or decrease in bone mass relative to that of a corresponding bone in the diseased state; that is, results in treatment, *i.e.*, modulates bone mass to more closely resemble that of corresponding non-diseased bone (that is a corresponding bone of the same type, *e.g.,* long, vertebral, *etc.*) in a non-diseased state.
ECD, as used herein, refers to extracellular domain.
TM, as used herein, refers to transmembrane domain.
CD, as used herein, refers to cytoplasmic domain.

### 4. BRIEF DESCRIPTION OF THE FIGURES

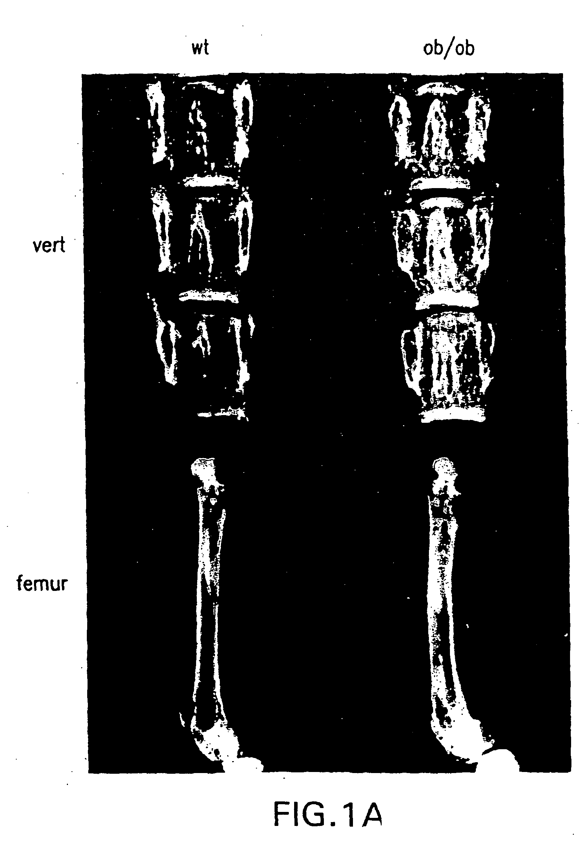

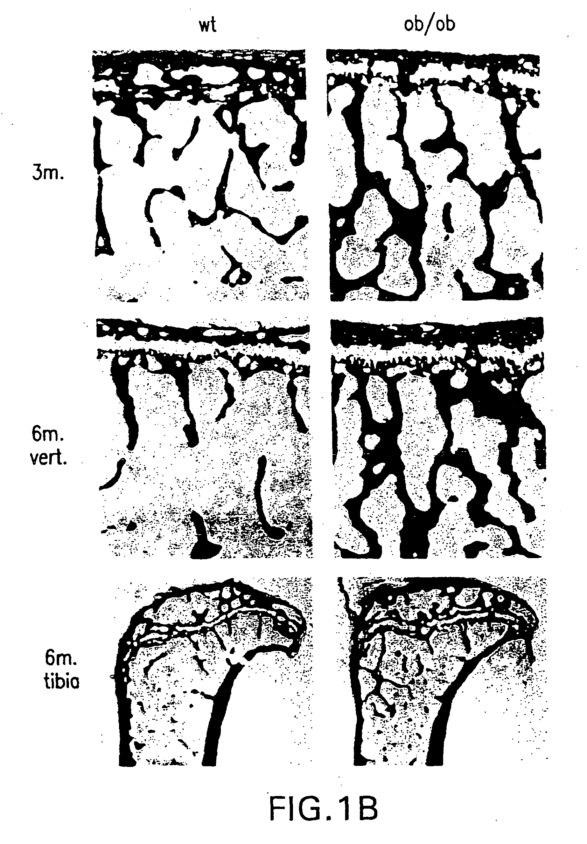

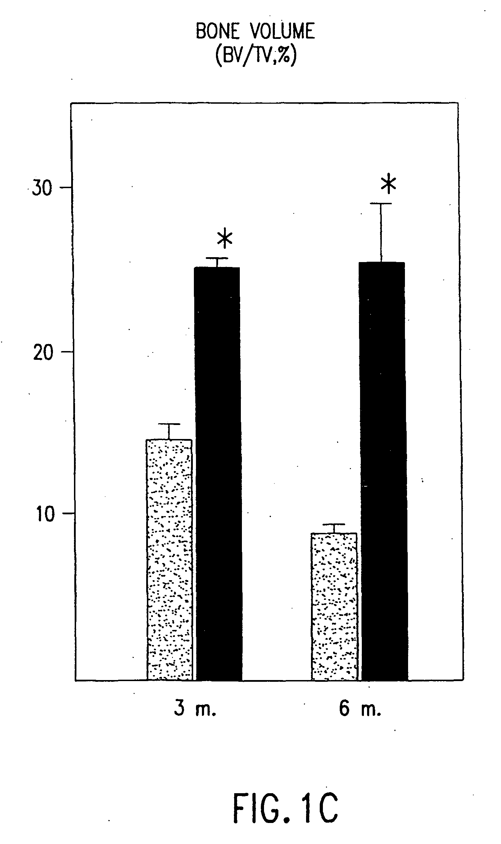

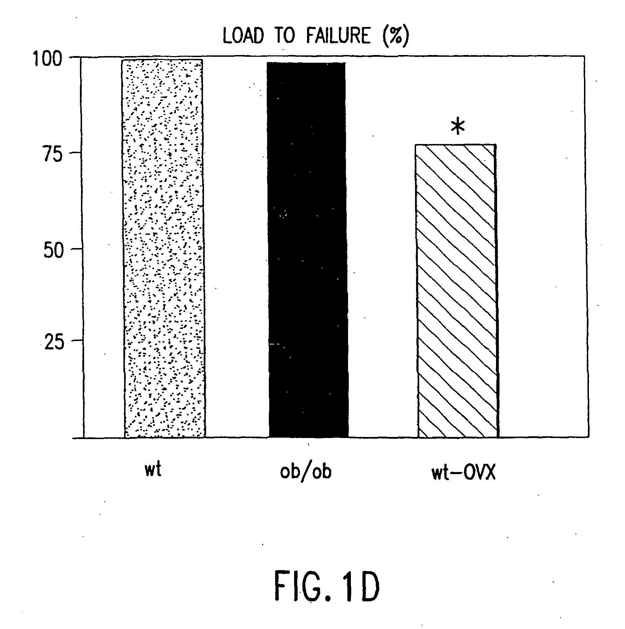

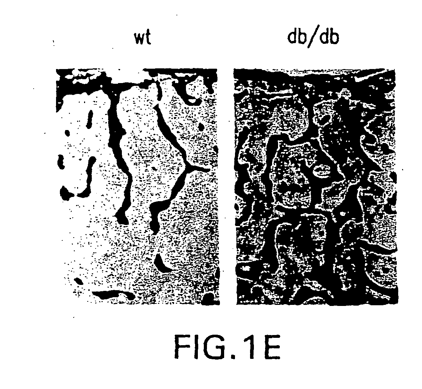

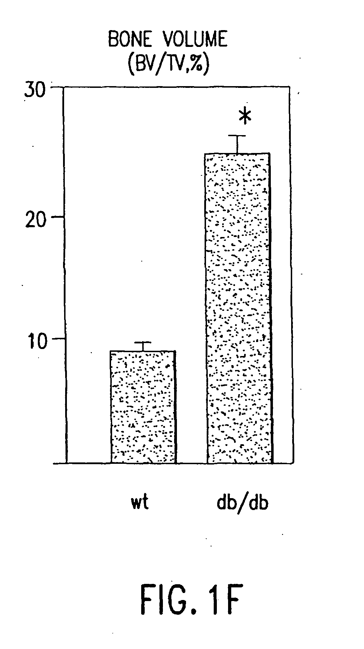

**FIGS. 1A****-1F.** High bone mass phenotype in ob/ob and db/db mice. In FIG. 1A, an X-ray analysis of vertebrae (vert.) and long bones (femurs) of 6 month-old wild-type (wt) and ob/ob mice is shown. FIG. 1B demonstrates a histological analysis of bones of 3 month-old (3m.) and 6 month-old (6m.) wt and ob/ob mice. The two upper panels demonstrate analysis of vertebrae, and the two bottom panels demonstrate long bones. Mineralized bone matrix is stained in black by the von Kossa reagent. FIG. 1C shows quantification of the increase in bone volume in ob/ob mice. BV/TV, bone volume over trabecular volume. Grey bars, wt mice; black bars, ob/ob mice. FIG. 1D illustrates a three points bending analysis of femur from wild-type (wt), ob/ob and wild-type ovariectomized (wt-OVX) mice. FIG. 1E is a histological analysis of vertebrae of 6 month-old wt and db/db mice. FIG. 1F is a quantification of the increase in bone volume in db/db mice. Asterisks indicate a statistically significant difference between two groups of mice (p<0.05). Error bars represent standard error of the mean (SEM).

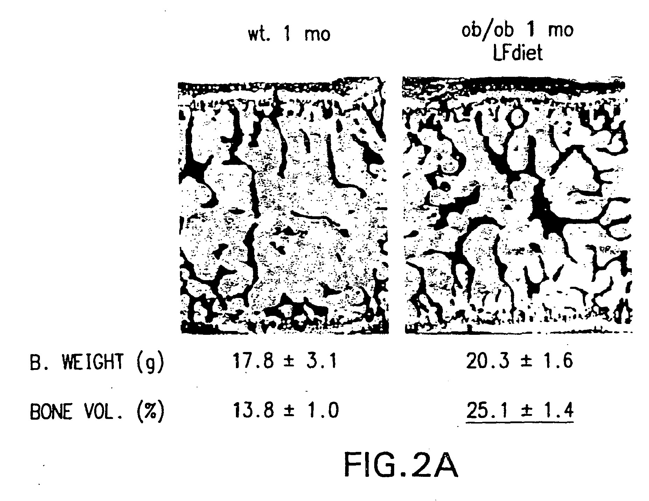

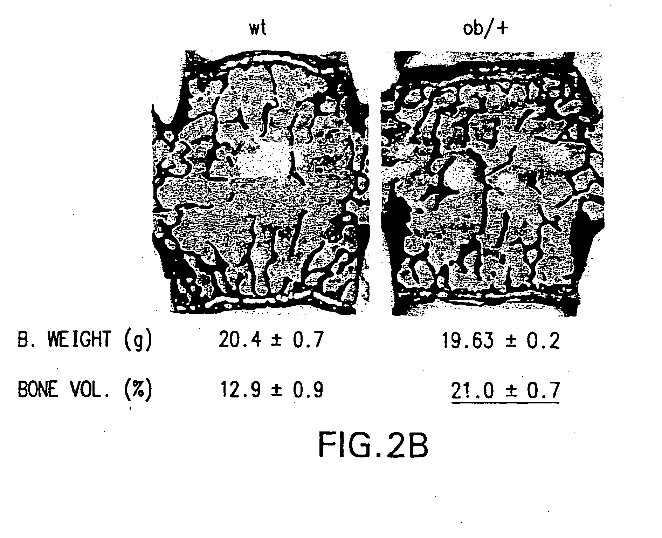

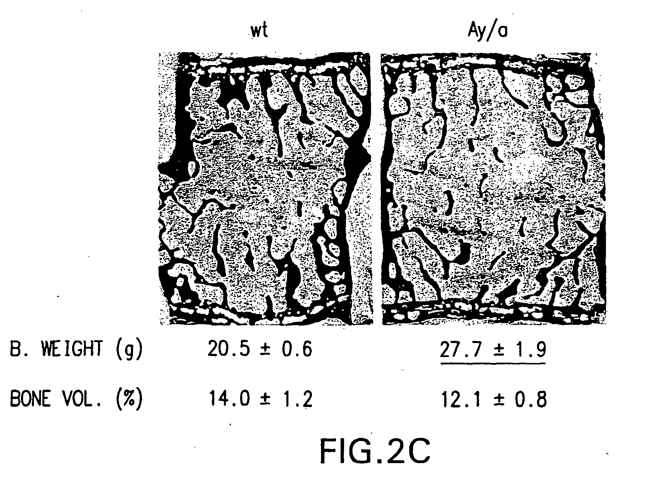

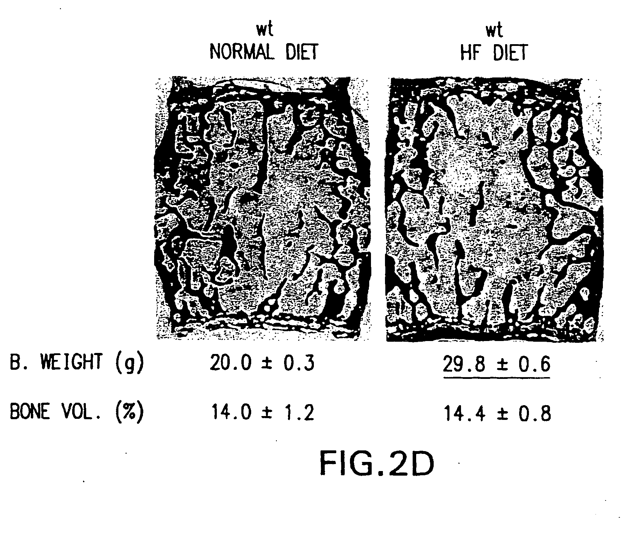

**FIGS. 2A****-2D**. High bone mass phenotype of the ob/ob mice is due to leptin deficiency, not to obesity. FIG. 2A demonstrates histological analysis of vertebrae of 1 month-old wt (wt 1 mo) and ob/ob mice fed a low fat diet (ob/ob 1 mo LF diet). FIG. 2B is a histological analysis of vertebrae of 3 month-old wt and ob/+ mice. FIG. 2C is a histological analysis of vertebrae of 6 month-old wt and Agouti yellow mutant mice (A ^{y}/ₐ). FIG. 2D is a histological analysis of vertebrae of 6 month-old wt mice fed a normal diet or a high fat (HF) diet. Underlined numbers indicate a statistically significant difference between experimental and control groups of mice (p<0.05).

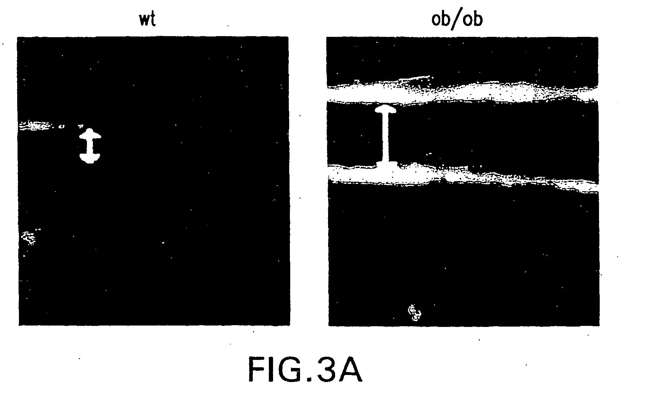

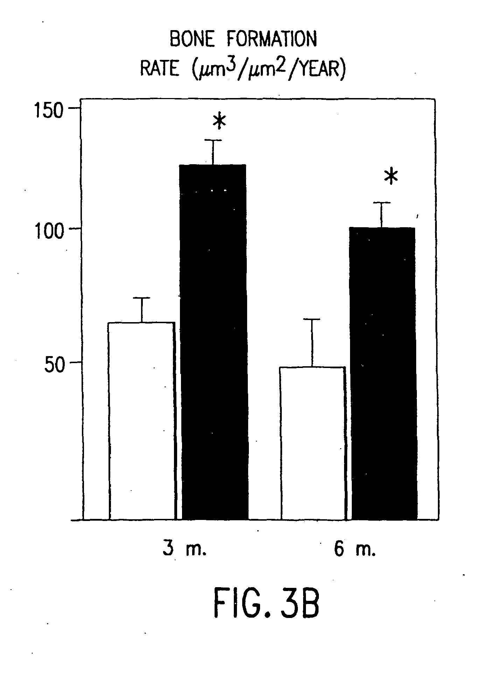

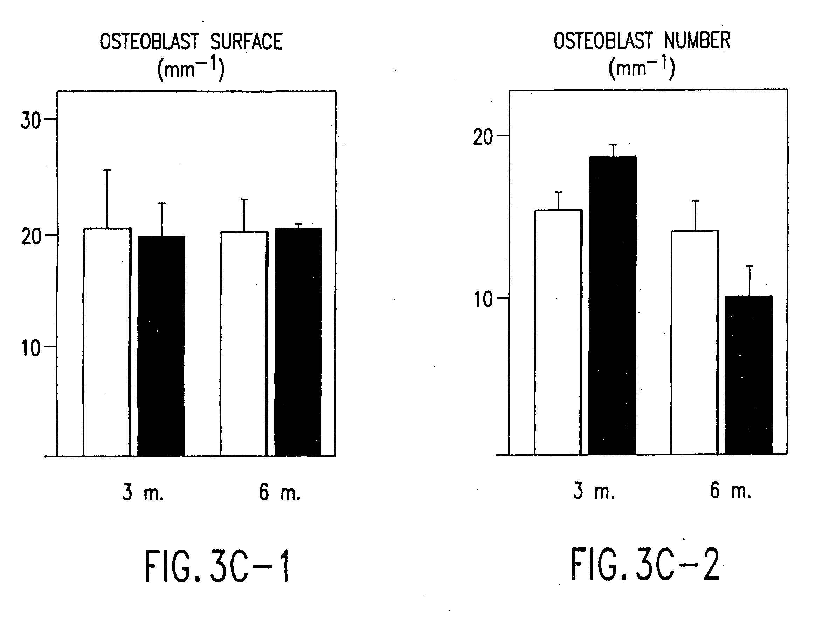

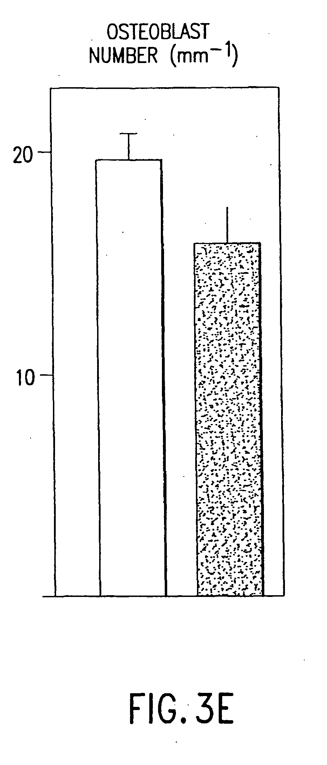

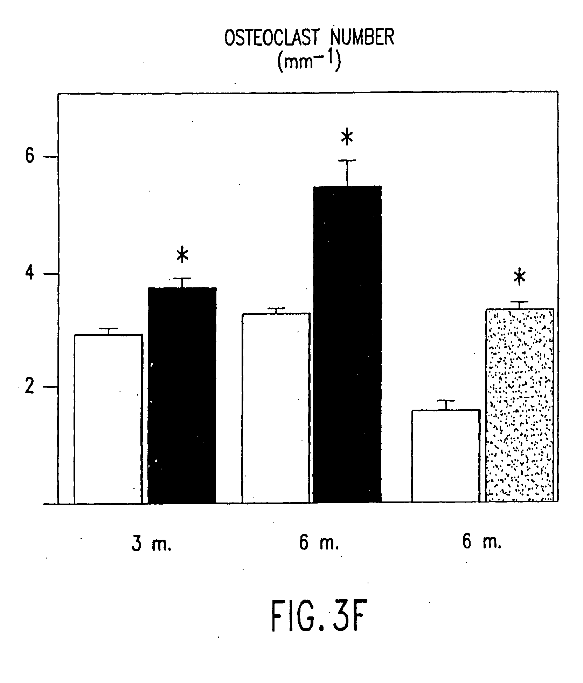

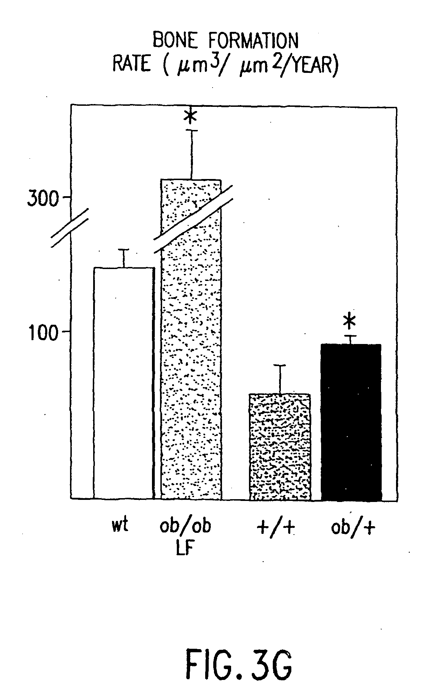

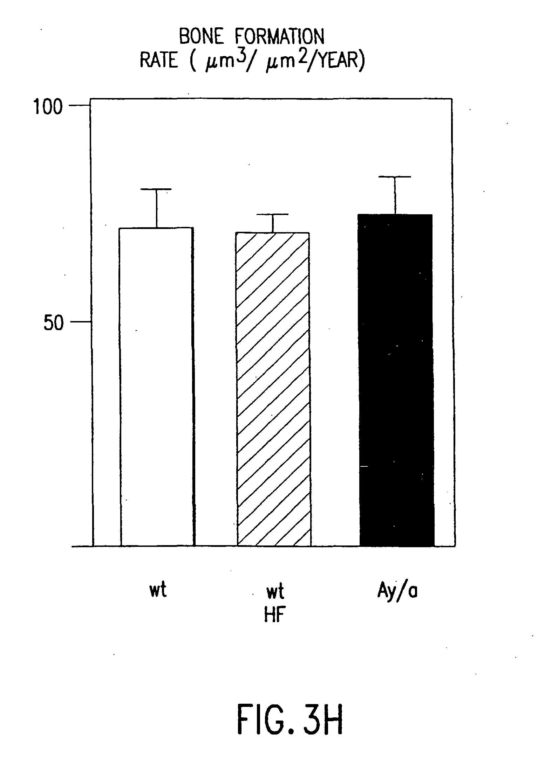

**FIGS. 3A****-3H.** Absence of leptin signaling causes an increase in osteoblast function. In FIG. 3A, calcein double labeling in 3-month-old wild-type (wt) and ob/ob mice is demonstrated. The distance between the two labels (white arrow) represents the rate of bone formation. In FIGS. 3B-3F, the rate of bone formation is increased in ob/ob mice (B) and db/db mice (D) 45% and 70%, respectively, compared to wt littermates. This increase occurs in the presence of a normal number of osteoblasts (FIGS. 3C and 3E), and in spite of the increased number of osteoclasts due to their hypogonadism (FIG. 3F). Empty bars, wt mice; black bars, ob/ob mice; grey bars, db/db mice; 3m., 3-month-old animals; 6m., 6-month-old animals. In FIG. 3G, increased bone formation rate in fat restricted 1-month-old ob/ob mice and heterozygote ob/+ mice, which are not obese (see body weights FIGS. 2A and B) is shown. In FIG. 3H, wt mice fed a high fat diet, or A ^{y}/ₐ mice, that are overweight (see FIGS. 2C and D) but not leptin-deficient have a normal rate of bone formation. Asterisks indicate statistically significant differences compared to control mice (p<0.05). Error bars represent SEM.

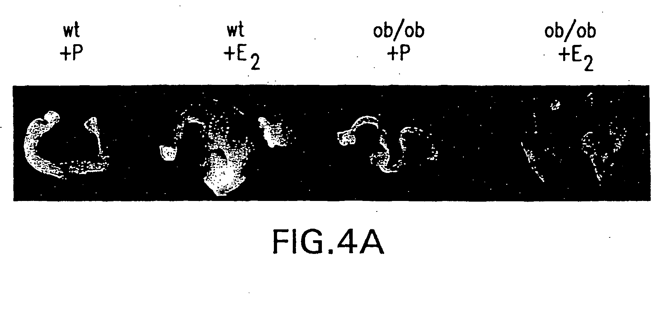

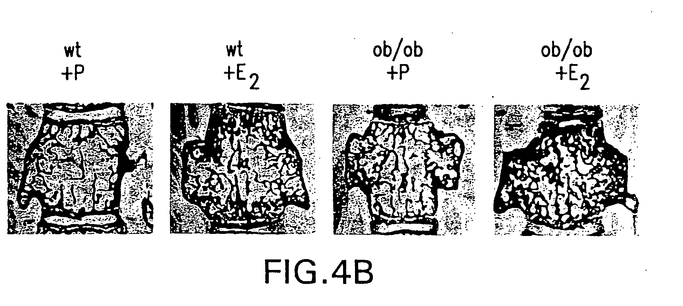

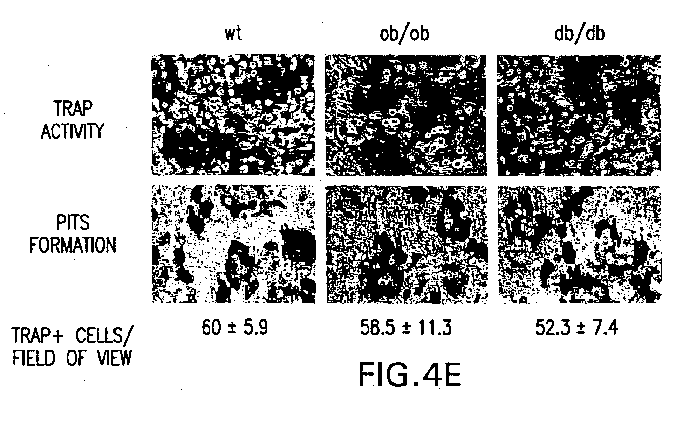

**FIGS. 4A****-4E**. Normal osteoclasts function in absence of leptin signaling. A comparative analyses of wild-type (wt) and ob/ob mice whose hypogonadism has been corrected by 17 β-estradiol treatment (E2) or not corrected (P, placebo) are shown. FIG. 4A demonstrates there is correction of the uterus atrophy of the ob/ob mice by the 17 β-estradiol treatment. In FIGS. 4B through 4D there is histological analysis of vertebrae showing that 17 β-estradiol treatment leads to a significant increase in bone trabeculae in 17 β-estradiol-treated wt and even more in 17 β-estradiol-treated ob/ob mice. Grey bars, wild-type mice; black bars, ob/ob mice; patterned bars, treated mice; solid bars, placebo control mice. Asterisks indicate a statistically significant difference between treated and untreated mice (p<0.05). Error bars represent SEM. In FIG. 4E, there is shown normal differentiation and function of ob/ob and db/db osteoclasts *ex-vivo.* Marrow progenitors derived from wt, ob/ob, and db/db mice differentiate equally well in TRAP positive (TRAP+) osteoclasts (upper panel and bottom line). There is also no difference in their ability to form resorption pits on a dentin slice matrix (bottom panel).

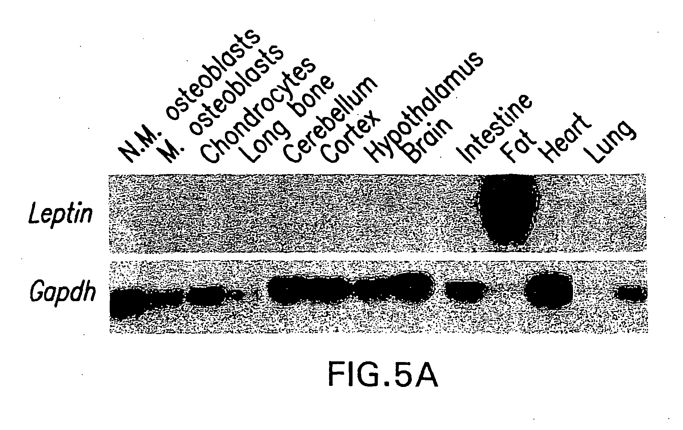

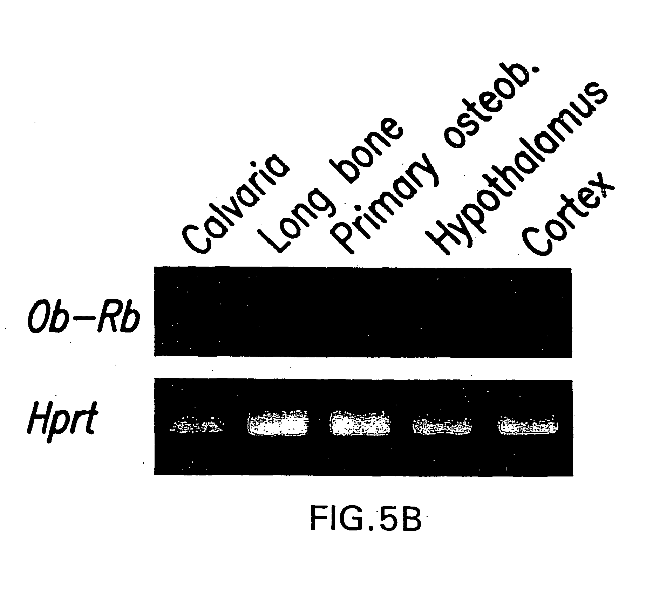

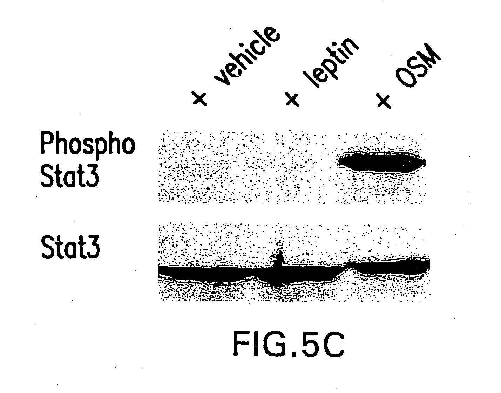

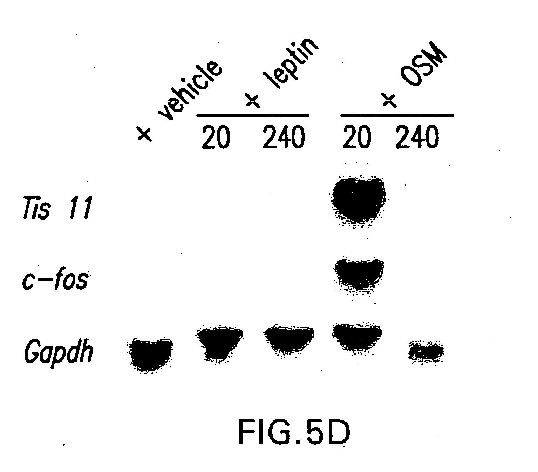

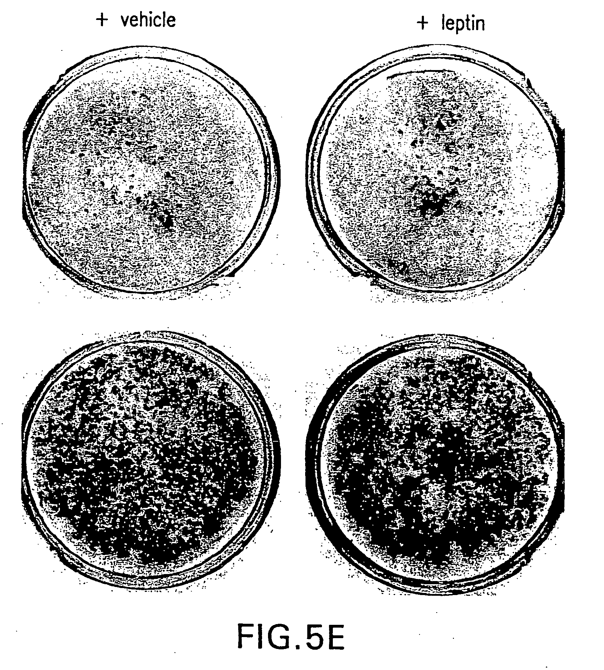

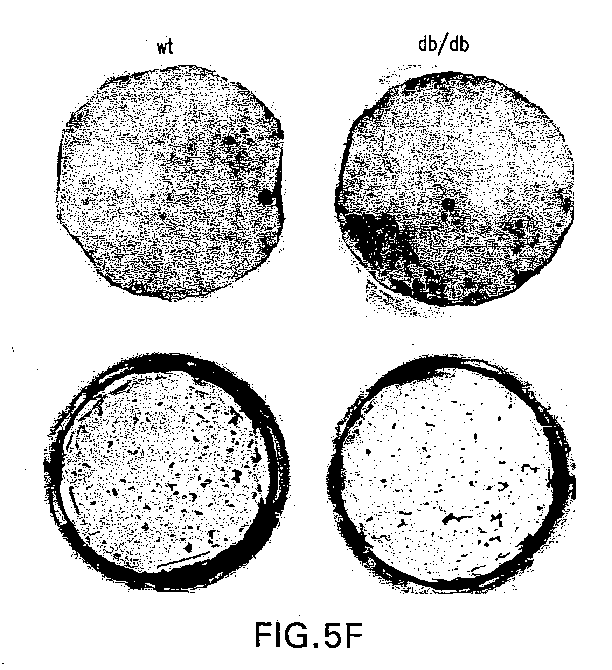

**FIGS. 5A****-5F.** Leptin does not signal in osteoblasts. Northern blot analysis of leptin expression in tissues and primary cells (non-mineralizing (NM) osteoblasts, mineralizing (M) osteoblasts and chondrocytes) (upper panel) is demonstrated in FIG. 5A. GAPDH expression was used as an internal control for loading (lower panel). FIG. 5B shows that *Ob-Rb* (Ob-receptor, type b) transcripts cannot be detected in long bones, Calabria and primary osteoblasts by RT-PCR while this message is detected in hypothalamus. Amplification of Hprt was used as an internal control for cDNA quality. FIG. 5C is a western blot analysis. Induction by oncostatin-M (OSM) was used as a positive control. In FIG. 5D, there is Northern blot analysis of immediate early gene expression *(Tis11* and *c-fos* genes) upon treatment of primary osteoblast cultures with leptin or Oncostatin-M. Gapdh expression was used as an internal control for loading (lower panel). In FIG. 5E *ex-vivo* primary osteoblast cultures from wild-type mice maintained in the absence (vehicle) or presence of leptin are shown. No effect on collagen synthesis (upper panel, van Gieson staining) or matrix mineralization (lower panel, von Kossa staining) can be observed. In FIG. 5F normal function of db/db osteoblasts in *ex vivo* culture experiments is shown. Collagen synthesis (upper panel, van Gieson staining) and matrix mineralization (lower panel, von Kossa staining) between primary osteoblast cultures derived from wt and db/db mice are demonstrated.

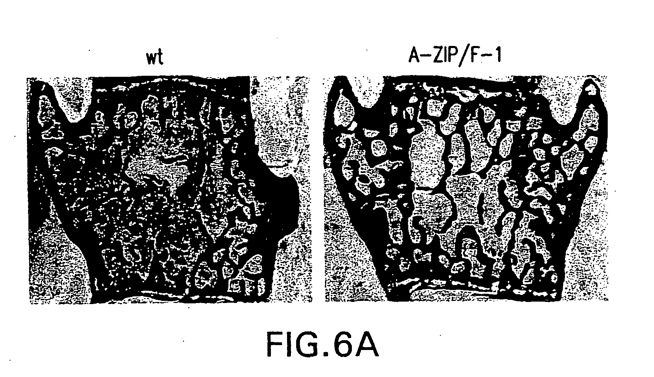

**FIGS. 6A****-6C**. Fat tissue is not required for the appearance of a high bone mass phenotype. In FIG. 6A, there is shown histological analysis of vertebrae of 6 month-old wt and A-ZIP/F-1 transgenic mice, that have no fat tissue. Bone volume (B) and bone formation rate (C) in the transgenic mice are illustrated. Asterisks indicate a statistically significant difference between wt and transgenic mice (p<0.05). Error bars represent SEM.

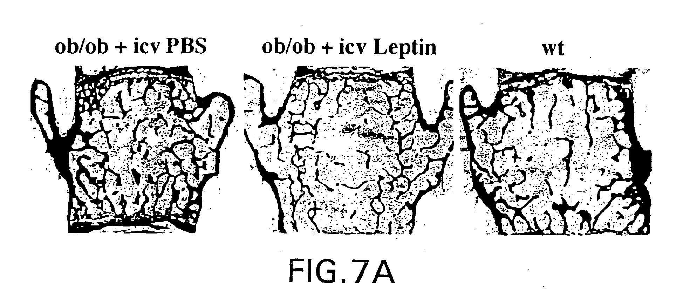

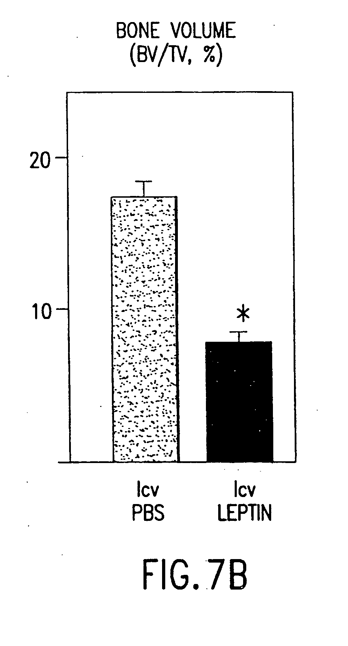

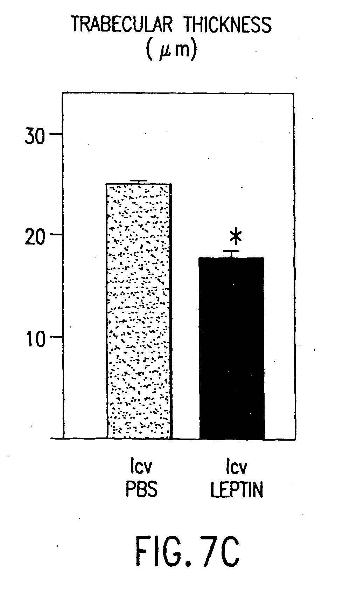

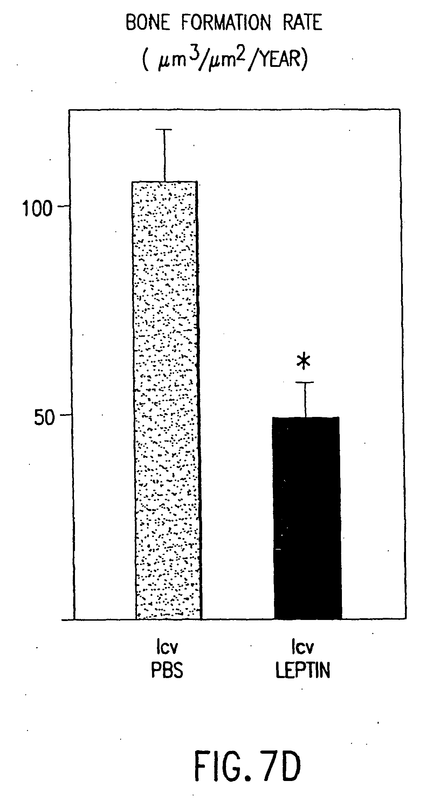

**FIGS. 7A****-7D.** Leptin action on bone formation is mediated by a hypothalamic relay. In FIG. 7A, there is a histological comparison of vertebrae of 4 month-old ob/ob mice infused centrally (third venticule) with PBS or leptin and wt mice. Bone volume (B), trabecular volume (C) and the rate of bone formation (D) are demonstrated. Asterisks indicate a statistically significant difference between PBS-infused and leptin-infused mice (p<O.O5). Error bars represent SEM.

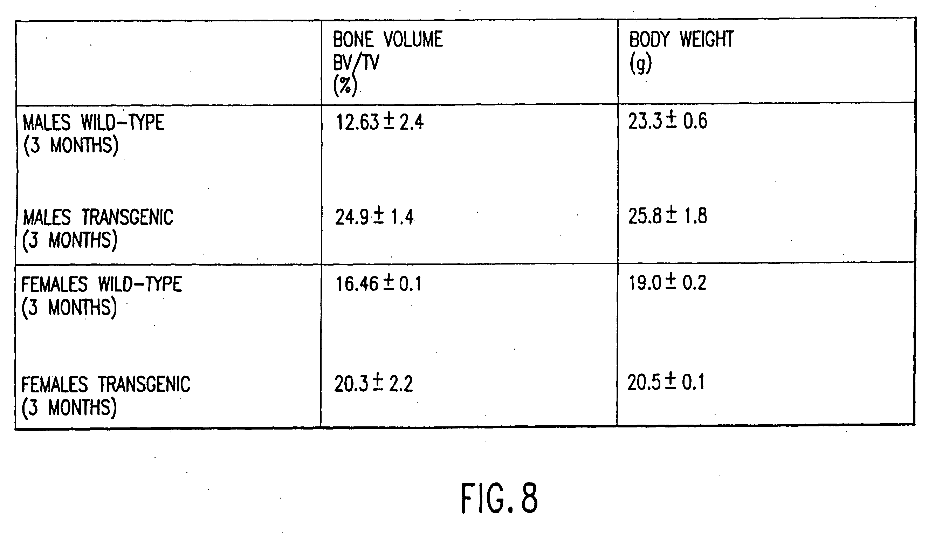

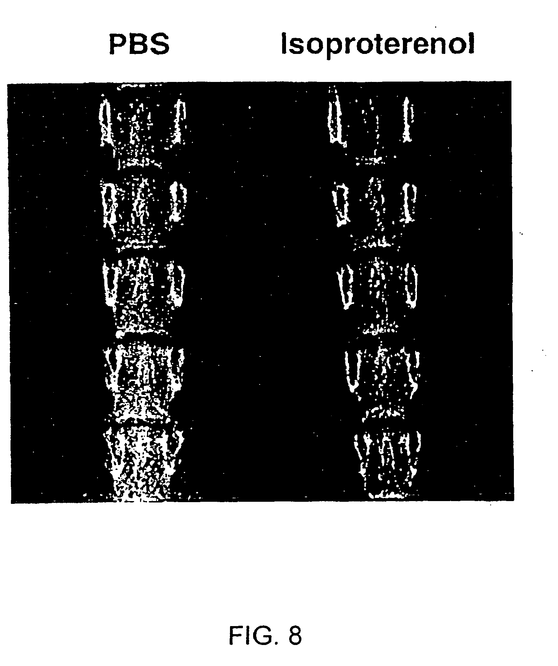

**FIG. 8****.** DBH knockout mice have high bone volume. FIG. 8 demonstrates histological analysis of vertebrae of wt and DBH -/- mice. Asterisks indicate a statistically significant difference between experimental and control groups of mice (p < 0.05).

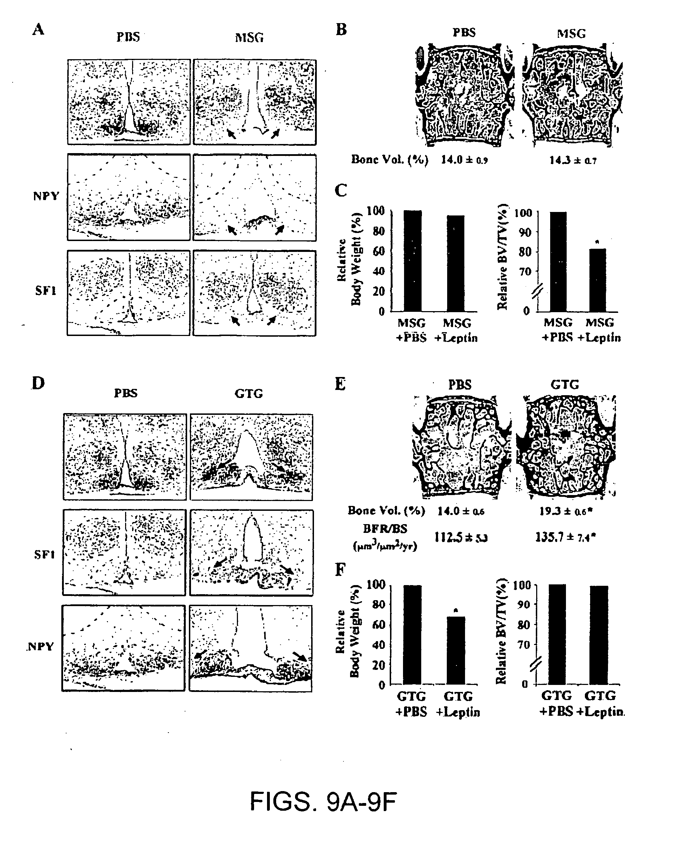

**FIGS. 9A****-9F.** Dissociation of leptin anorexigenic and antiosteogenic actions in the hypothalamus. (FIGS. 9A-9C) MSG lesion. (A) Effect on hypothalamic structure, Cresyl violet staining (top panels) immunohistochemistry for NPY (middle panels) and SF1 (bottom panels). The arcuate nuclei (ARC) and the VMH are encircled by black and red dotted lines, respectively. MSG treatment markedly affected ARC structure and NPY expression (arrows) while VMH structure and SF1-expressing neurons were preserved. (B) Histological analysis of vertebrae of MSG-lesioned mice; bone volume (% Bone vol. / tissue volume) is not affected. (C) Leptin ICV infusion does not affect body weight (left) but decreases bone volume (right) in MSG-lesioned *ob*/*ob* mice (MSG+leptin). (FIGS. 9D-9F) GTG lesion. (D) Effect on hypothalamic structure, Cresyl violet staining (top panels) immunohistochemistry for SF1 (middle panels) and NPY (bottom panels). GTG treatment affected the VMH resulting in a dense scar and markedly altering the distribution of SF-1-expressiong neurons while preserving ARC structure and NPY expression. (E) Histological analysis of vertebrae of GTG-lesioned mice; Bone vol. and bone formation rate (BFR/ BS) are increased. (F) Leptin ICV infusion decreases body weight (left) but does not affect Bone vol. (right) in GTG-treated animals (GTG+leptin). Asterisks indicate statistically significant differences between experimental and control groups (P<0.01).

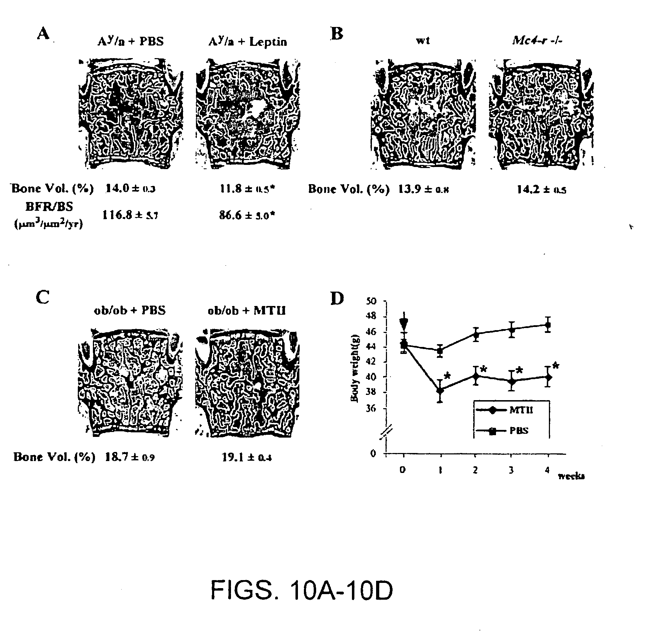

**FIGS. 10A****-10D.** Anorexigenic neuropeptides do not control bone formation. (A) Histological analysis of vertebrae of 4-month-old agouti yellow mutant mice (A^{y}/a) infused ICV with PBS or leptin. ICV leptin infusion causes a decrease in bone volume (Bone vol. %) and bone formation rate (BFR/BS, white arrows). (B) Histological analysis of vertebrae of 3-month-old *Mc4-R*-deficient mice (*MC4-R*-/-); Bone vol. is normal. (C-D) Histological analysis of vertebrae of *ob*/*ob* mice infused ICV with PBS or MTII; bone volume is not affected (C), whereas body weight is decreased (D). Asterisks indicate statistically significant differences between experimental and control groups (P<0.01). Error bars represent SEM.

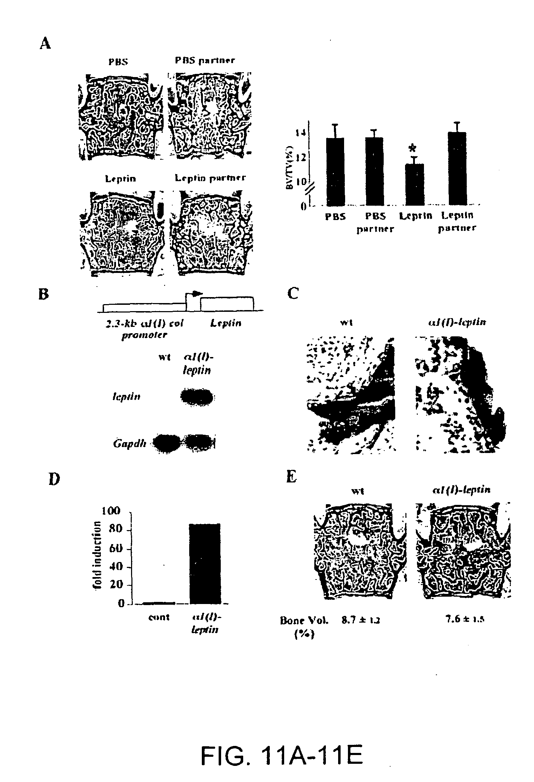

**FIGS. 11A****-11E.** Peripheral mediation of leptin antiosteogenic function. (A) Histological analysis of vertebrae of parabiosed *ob*/*ob* mice subsequently infused with PBS or leptin is shown on the left. Leptin ICV infusion decreases the bone volume (Bone vol. %) of the ipsilateral mouse (Leptin), but not in the contralateral mouse (Leptin partner). No effect was observed with PBS (PBS and PBS partner). (B) Generation of osteoblast-specific leptin (*α1(I)-leptin*) transgenic mice. Top panel, schematic representation of the construct. The mouse *leptin* cDNA is under the control of the 2.3-kb osteoblast-specific fragment of the *al(I) collagen* promoter. Bottom left panel, Northern blot analysis of the transgene expression in bone. Bottom right panel, Leptin immunoreactivity is detectable in bones of *αl-leptin* mice but not of wt mice. (C) Bioactivity analysis of the *αl-leptin* transgene performed in 293 cells expressing *ObRb* cotransfected with the *αl-leptin* transgene and a Stat3-dependent promoter-luc construct. (E) Histological analysis of vertebrae of 12-month-old *αl(I)-leptin* transgenic mice. Leptin expression in osteoblasts does not affect Bone vol. Asterisks indicate statistically significant differences between experimental and control groups (P<0.01). Error bars represent SEM.

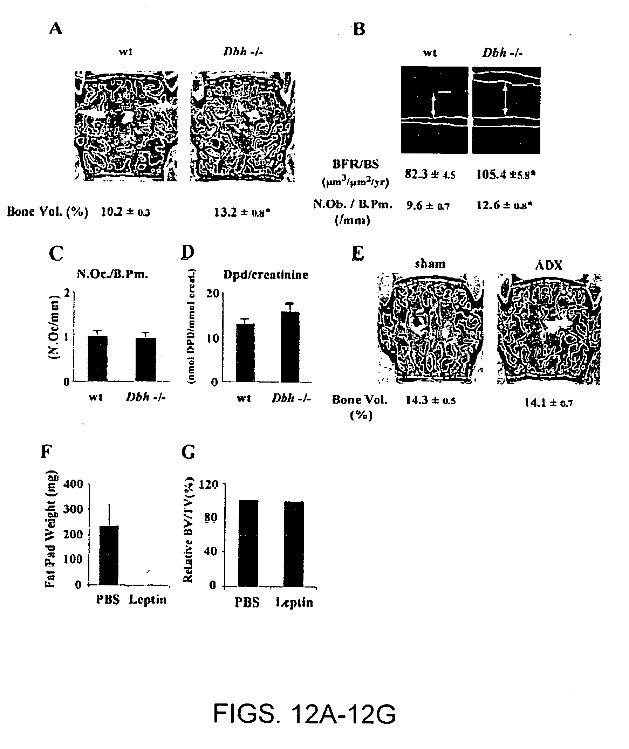

**FIGS. 12A****-12G**. Sympathetic control of bone formation. (A) Histological analysis of vertebrae of 12 month-old *Dbh*-deficient mice (*Dbh* -/-). Bone volume (Bone vol. %) is significantly increased in *Dbh-l-* mice. (B) Calcein double labeling, bone formation rate (BFR/BS, white arrows) and number of osteoblasts (N.Ob/B.Pm) are increased in *Dbh-l-*mice. (C, D) Normal osteoclast number (N.Oc/B.Pm) and deoxypyridinoline crosslinks elimination in *Dbh*-deficient mice. (E) Histological analysis of vertebrae of adrenal medullectomized mice (ADX); Bone vol. is not affected. (F, G) Leptin icv infusion in *Dbh-*/mice. Fat pad weight is decreased (D) while bone volume is not affected (E) by the treatment. Asterisks indicate statistically significant differences between experimental and control groups (P<0.01). Error bars represent SEM.

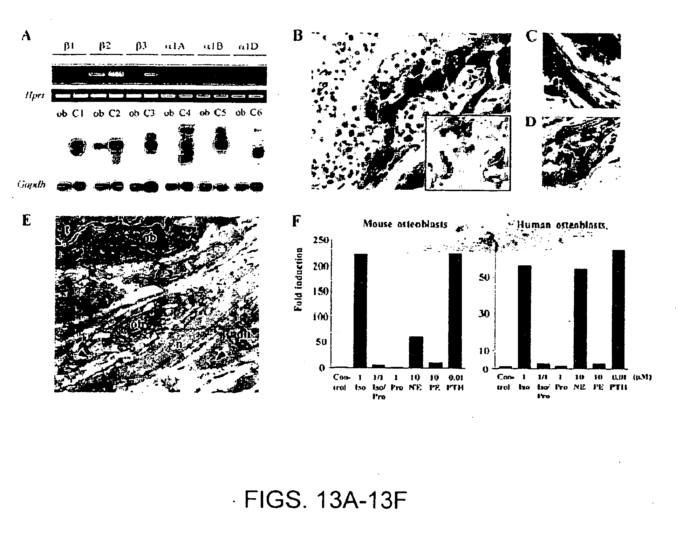

**FIGS. 13A****-13F.** Presence of functional adrenergic receptors on osteoblasts. (A) RT-PCR (top panel) and Northern blot analyses (bottom panel) of α- and β- adrenergic receptor expression. Only β2-adrenergic receptor expression can be detected in osteoblasts. Samples were from primary osteoblasts (ob), heart (C1, C2, C4 and C6), brown adipose tissue (C3), and liver (C5). *Hprt* amplification and *Gapdh* expression were used as controls for loading and RNA integrity. (B) Immunolocalization of β2-adrenergic receptors in bones of wt mice and transgenic mice overexpressing LacZ in osteoblasts (Inset). Mononucleated cells express β2-adrenergic receptors and those cells are X-gal positive *i.e.,* osteoblasts. (C, D) Immunolocalization of neurofilament (C) and tyrosine hydroxylase (D, arrow) adjacent to osteoblasts. (E) Electron micrographs of bone sections of 2-day-old mice. A nerve (n) is located in close vicinity to osteoblasts (ob) and bone trabeculae (t). (F) In murine primary osteoblasts (left) and human SaOS-2 osteoblastic cells (right) cAMP production is induced by β-adrenergic receptor agonists (isoproterenol, iso; norepinephrine, NE) and inhibited by the addition of β-adrenergic antagonist (propranolol, Pro). PTH was used as a positive control.

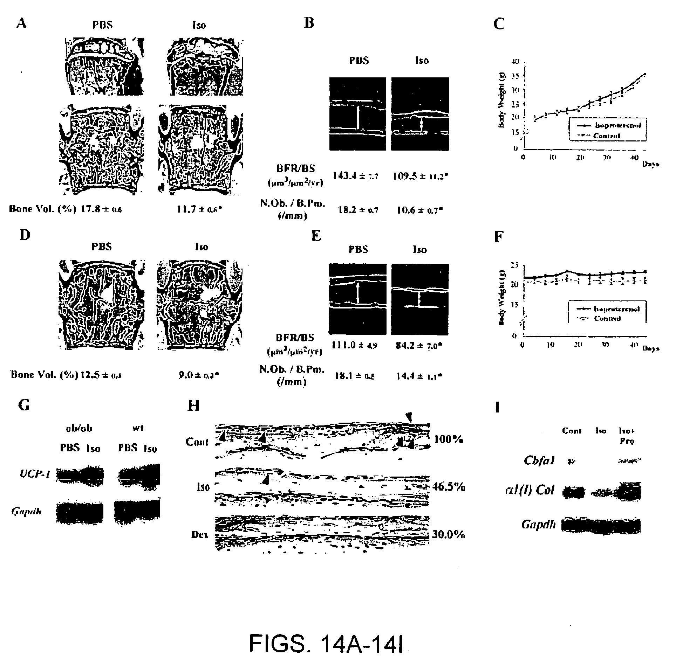

**FIGS. 14A****-14I.** A β-adrenergic agonist inhibits bone formation. Isoproterenol (Iso) treatment of *ob*/*ob* (A-C, G) and wt (D-H) mice (A, D). Histological analysis of vertebrae and tibia (A). Bone volume (Bone vol. %) is decreased by Iso treatment. (B, E) Calcein double labeling (upper panels), bone formation rate (BFR/BS, white arrows) and osteoblast number (N.Oc/B.Pm) are decreased by Iso treatment. (C, F) Body weight analysis. These doses of Iso do not affect body weight. (G) Northern blot analysis. Iso increases *UCP-1* expression in brown adipose tissues (upper panel). (H) Osteoblast proliferation. Immunolocalization of BrdU incorporation (red staining, arrows) in calvariae of Iso, dexamethasone (Dex) or vehicle (Cont.)-treated mice. Percentages of BrdU positive cells relative to control are indicated on the right. (I) Northern blot analysis of *Cbfa1* and *αl(I) collagen* expression in primary osteoblasts treated by Iso, Iso and propranolol (Iso+Pro) or vehicle (Cont). *Gapdh* expression was used as an internal control for all Northern blots. Asterisks indicate statistically significant differences between experimental and control groups (P<0.01).

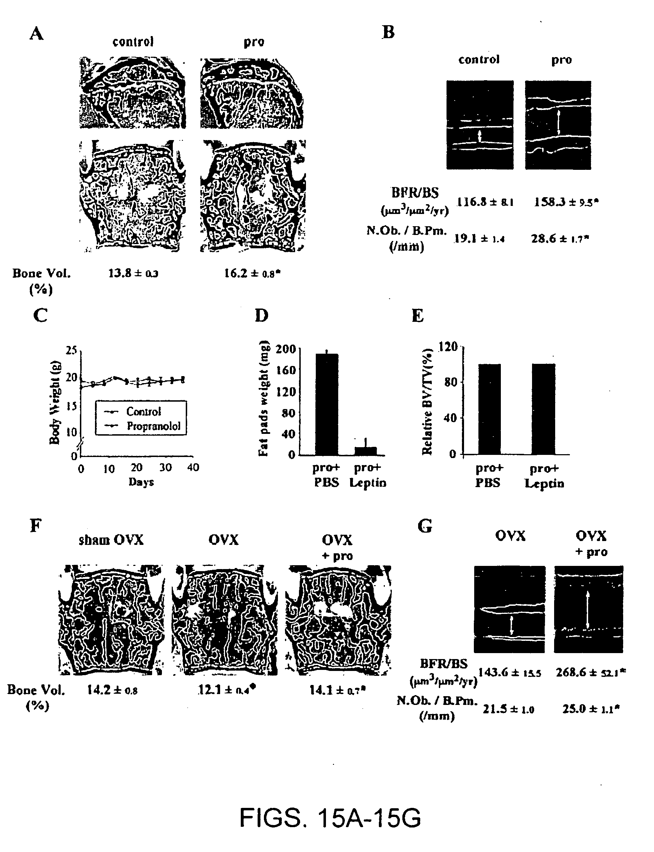

**FIGS. 15A****-15G.** A β-adrenergic antagonist increases bone mass. Propranolol (Pro) treatment of wt (A, C), wt receiving leptin ICV infusion (D, E) ovariectomized (OVX) and sham-operated (Sham OVX) (F, G) mice. Histological analysis of vertebrae (A, B, E-G) and tibiae (A). Bone volume (Bone vol. %) is increased by Pro treatment (A, E, F). Calcein double labeling (upper panels), bone formation rates (BFR/BS, white arrows) and osteoblast numbers (N.Ob/B.Pm) are increased in propranolol-treated mice (B and G). Body weight (C) is not affected. Leptin ICV infusion decreases fat pad weight but not bone mass in protreated wt mice (D and E). Pro-treatment prevents bone loss by increasing bone formation parameters in OVX mice (F and G). Asterisks indicate statistically significant differences between experimental and control groups (P<0.01). Diamond indicates statistically significant differences between OVX and sham operated mice (P<0.01). Error bars represent SEM.

The drawings and figures are not necessarily to scale and certain features mentioned may be exaggerated in scale or shown in schematic form in the interest of clarity and conciseness.

### 5. DETAILED DESCRIPTION OF THE INVENTION

Various aspects of the present invention are presented in detail herein.

### 5.1. Leptin, Leptin Receptor, and Adrenergic Receptor Proteins, Polypeptides and Nucleic Acids

Leptin ("Ob"), leptin receptor ("ObR"), and adrenergic receptor ("AR") proteins and nucleic acids (sense and antisense) can be utilized as part of the therapeutic, diagnostic, prognostic and screening methods of the present invention. For example, Ob, ObR, and/or AR proteins, polypeptides and peptide fragments, mutated, truncated or deleted forms of Ob, ObR, and AR including, but not limited to, soluble derivatives such as peptides or polypeptides corresponding to one or more leptin receptor ECDs; truncated leptin receptor polypeptides lacking one or more ECD or TM; truncated adrenergic receptor polypeptides lacking one or more ECD or TM; and leptin, leptin receptor, and adrenergic receptor fusion protein products (such as leptin receptor-Ig fusion proteins, that is, fusions of the leptin receptor or a domain of the leptin receptor, to an IgFc domain) can be utilized.

Sequences of leptin and leptin receptor, including human leptin and leptin receptors, are well known. For a review of leptin receptor proteins, see, e.g., Friedman and Halaas, 1998, Nature, 395:763-770 and U.S. Patent No. 5,972,621. For leptin sequences, including human leptin coding sequences and leptin gene regulatory sequences, see, e.g., Zhang et al., 1994, Nature 372:425-432; de la Brousse et al., 1996, Proc. Natl. Acad. Sci. USA 93:4096-4101; He et al., 1995, J. Biol. Chem. 270:28887-28891; Hwang et al., 1996, Proc. Natl. Acad. Sci. USA 93:873-877; and Gong et al., 1996, J. Biol Chem 271:3971-3974.

Sequences of adrenergic receptors, include human adrenergic receptors, are well known (see, *e.g.,* U.S. Patent Nos. 6,274,706; 5,994,506; 5,817,477; and 5,595,880).

For example, peptides and polypeptides corresponding to Ob or to one or more domains of the ObR or AR (e.g., ECD, TM or CD), truncated or deleted Ob, ObRs, or ARs (e.g., ObR in which the TM and/or CD is deleted) as well as fusion proteins in which the full length Ob, ObR, or AR, an Ob, ObR, or AR peptide or truncated Ob, ObR, or AR (e.g., an ObR ECD, TM or CD domain) is fused to a heterologous, unrelated protein are also within the scope of the invention and can be utilized and designed on the basis of such Ob, ObR, and AR nucleotide and Ob, ObR, and AR amino acid sequences which are known to those of skill in the art. Preferably, leptin polypeptides can bind leptin receptor under standard physiological and/or cell culture conditions. Likewise, preferably leptin receptor polypeptides can bind leptin under standard physiological and/or cell culture conditions. Similarly, preferably adrenergic receptor polypeptides can bind catecholamines under standard physiological and/or cell conditions. Thus, at a minimum, leptin receptor polypeptides comprise a leptin receptor amino acid sequence sufficient for leptin binding, that is for leptin/leptin receptor complex formation and likewise, at a minimum, leptin receptor polypeptides comprise a leptin receptor ECD sequence sufficient for leptin binding. Similarly, at a minimum, adrenergic receptor polypeptides comprise a adrenergic receptor amino acid sequence sufficient for catecholamine binding, that is for catecholamine/adrenergic receptor complex formation and likewise, at a minimum, adrenergic receptor polypeptides comprise a adrenergic receptor ECD sequence sufficient for catecholamine binding.

With respect to ObR and AR peptides, polypeptides, fusion peptides and fusion polypeptides comprising all or part of an ObR or AR ECD, such peptides include soluble leptin receptor or adrenergic receptor polypeptides. Preferably, such soluble leptin receptor or adrenergic receptor polypeptides can bind leptin or catecholamines, respectively, under standard physiological and/or cell culture conditions. Thus, at a minimum, such soluble leptin receptor or adrenergic receptor polypeptides comprise an ObR or AR ECD sequence sufficient for leptin or catecholamine, respectively, binding.

Fusion proteins include, but are not limited to, IgFc fusions which stabilize the soluble ObR or AR protein or peptide and prolong half-life *in vivo;* or fusions to any amino acid sequence that allows the fusion protein to be anchored to the cell membrane, allowing the ECD to be exhibited on the cell surface; or fusions to an enzyme, fluorescent protein, or luminescent protein which provide a marker or reporter function, useful e.g, in screening and/or diagnostic methods of the invention.

While the Ob, ObR, or AR polypeptides and peptides can be chemically synthesized *(e.g.,* see Creighton, 1983, Proteins: Structures and Molecular Principles, W. H. Freeman & Co., N.Y.), large polypeptides derived from Ob, ObR, and AR and full length Ob, ObR, and AR may advantageously be produced by recombinant DNA technology using techniques well known in the art for expressing nucleic acid containing Ob, ObR, and AR gene sequences and/or coding sequences. Ob, ObR, and AR encoding polynucleotides does not refer only to sequences encoding open reading frames, but also to upstream and downstream sequences within the Ob, ObR, and AR genes. Such methods also can be used to construct expression vectors containing the Ob, ObR, and AR nucleotide sequences. These methods include, for example, *in vitro* recombinant DNA techniques, synthetic techniques, and *in vivo* genetic recombination (see, e.g., Sambrook et al., 1989, Molecular Cloning, A Laboratory Manual, Second Edition, Cold Spring Harbor Press, N.Y., and Ausabel et al., 1989, Current Protocols in Molecular Biology, Green Publishing Associates and Wiley Interscience, N.Y., each of which is incorporated herein by reference in its entirety). Alternatively, RNA capable of encoding Ob, ObR, and AR nucleotide sequences may be chemically synthesized using, for example, synthesizers (see, e.g., the techniques described in "Oligonucleotide Synthesis", 1984, Gait, M. J. ed., IRL Press, Oxford, which is incorporated by reference herein in its entirety).

A variety of host-expression vector systems may be utilized to express the Ob, ObR, and AR nucleotide sequences of the invention. Where the Ob, ObR, and AR peptide or polypeptide is a soluble derivative (e.g., ObR or AR peptides corresponding to the ECD; truncated or deleted ObR in which the TM and/or CD are deleted) the peptide or polypeptide can be recovered from the culture, *i.e*., from the host cell in cases where the ObR or AR peptide or polypeptide is not secreted, and from the culture media in cases where the ObR or AR peptide or polypeptide is secreted by the cells. However, the expression systems also encompass engineered host cells that express Ob, ObR, and AR or functional equivalents in *situ, i.e.,* anchored in the cell membrane. Purification or enrichment of Ob, ObR, or AR from such expression systems can be accomplished using appropriate detergents and lipid micelles and methods well known to those skilled in the art. However, such engineered host cells themselves may be used in situations where it is important not only to retain the structural and functional characteristics of Ob, ObR, and AR, but to assess biological activity, e.g., in drug screening assays.

The expression systems that may be used for purposes of the invention include, but are not limited to, microorganisms such as bacteria (e.g., E. coli, B. subtilis) transformed with recombinant bacteriophage DNA, plasmid DNA or cosmid DNA expression vectors containing Ob, ObR, or AR nucleotide sequences; yeast (e.g., Saccharomyces, Pichia) transformed with recombinant yeast expression vectors containing the nucleotide sequences; insect cell systems infected with recombinant virus expression vectors (e.g., baculovirus) containing the sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing the nucleotide sequences; or mammalian cell systems (*e.g.,* COS, CHO, BHK, 293, 3T3) harboring recombinant expression constructs containing promoters derived from the genome of mammalian cells (e.g., metallothionein promoter) or from mammalian viruses (e.g., the adenovirus late promoter; the vaccinia virus 7.5K promoter).

In bacterial systems, a number of expression vectors may be advantageously selected depending upon the use intended for the Ob, ObR, or AR gene product being expressed. For example, when a large quantity of such a protein is to be produced, for the generation of pharmaceutical compositions of Ob, ObR, or AR protein or for raising antibodies to Ob, ObR, or AR protein, for example, vectors which direct the expression of high levels of fusion protein products that are readily purified may be desirable. Such vectors include, but are not limited to, the E. coli expression vector pUR278 (Ruther et al., 1983, EMBO J. 2:1791), in which the Ob or ObR coding sequence may be ligated individually into the vector in frame with the lacZ coding region so that a fusion protein is produced; pIN vectors (Inouye & Inouye, 1985, Nucleic Acids Res. 13:3101-3109; Van Heeke & Schuster, 1989, J. Biol. Chem. 264:5503-5509); and the like. pGEX vectors may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target gene product can be released from the GST moiety.

In an insect system, Autographa californica nuclear polyhidrosis virus (AcNPV) is used as a vector to express foreign genes. The virus grows in Spodoptera frugiperda cells. The Ob, ObR, or AR gene coding sequence may be cloned individually into non-essential regions (for example the polyhedrin gene) of the virus and placed under control of an AcNPV promoter (for example the polyhedrin promoter). Successful insertion of an Ob, ObR, or AR gene coding sequence will result in inactivation of the polyhedrin gene and production of non-occluded recombinant virus, *(i.e.,* virus lacking the proteinaceous coat coded for by the polyhedrin gene). These recombinant viruses are then used to infect Spodoptera frugiperda cells in which the inserted gene is expressed (see, e.g., Smith et al., 1983, J. Virol. 46: 584 and U.S. Pat. No. 4,215,051).

In mammalian host cells, a number of viral-based expression systems may be utilized. In cases where an adenovirus is used as an expression vector, the Ob, ObR, or AR nucleotide sequence of interest may be ligated to an adenovirus transcription/translation control complex, e.g., the late promoter and tripartite leader sequence. This chimeric gene may then be inserted in the adenovirus genome by *in vitro* or *in vivo* recombination. Insertion in a non-essential region of the viral genome (*e.g.,* region E1 or E3) will result in a recombinant virus that is viable and capable of expressing the Ob, ObR, or AR gene product in infected hosts (see, e.g., Logan & Shenk, 1984, Proc. Natl. Acad. Sci. USA 81:3655-3659). Specific initiation signals may also be required for efficient translation of inserted Ob, ObR, or AR nucleotide sequences. These signals include the ATG initiation codon and adjacent sequences. In cases where entire Ob, ObR, or AR genes or cDNAs, including their own initiation codons and adjacent sequences, are inserted into the appropriate expression vector, no additional translational control signals may be needed. However, in cases where only a portion of the coding sequence is inserted, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, *etc*. (See Bittner et al., 1987, Methods in Enzymol. 153:516-544).

In addition, a host cell strain may be chosen which modulates the expression of the inserted sequences, or modifies and processes the gene product in the specific fashion desired. Such modifications (e.g., glycosylation) and processing (e.g., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERO, BHK, HeLa, COS, MDCK, 293, 3T3, WI38, and in particular, choroid plexus cell lines.

For long-term, high-yield production of recombinant proteins, stable expression is preferred. For example, cell lines which stably express the Ob, ObR, or AR sequences may be engineered. Rather than using expression vectors which contain viral origins of replication, host cells can be transformed with DNA controlled by appropriate expression control elements (e.g., promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the Ob, ObR, or AR gene products. Such engineered cell lines may be particularly useful in screening and evaluation of compounds that affect the endogenous activity of Ob, ObR, and AR gene products.

A number of selection systems may be used, including but not limited to, the herpes simplex virus thymidine kinase (Wigler et al., 1977, Cell 11:223), hypoxanthine-guanine phosphoribosyltransferase (Szybalska & Szybalski, 1962, Proc. Natl. Acad. Sci. USA 48:2026), and adenine phosphoribosyltransferase (Lowy et al., 1980, Cell 22:817) genes can be employed in tk⁻, hgprt⁻ or aprt⁻ cells, respectively. Also, antimetabolite resistance can be used as the basis of selection for the following genes: dhfr, which confers resistance to methotrexate (Wigler et al., 1980, Natl. Acad. Sci. USA 77:3567; O'Hare et al., 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 (Colberre-Garapin et al., 1981, J. Mol. Biol. 150:1); and hygro, which confers resistance to hygromycin (Santerre et al., 1984, Gene 30:147).

The Ob, ObR, and AR gene products can also be expressed in transgenic animals. Animals of any species, including, but not limited to, mice, rats, rabbits, guinea pigs, pigs, micro-pigs, goats, and non-human primates, e.g., baboons, monkeys, and chimpanzees may be used to generate the transgenic animals.

Any technique known in the art may be used to introduce the Ob, ObR, or AR transgene into animals or to "knock-out" or inactivate endogenous Ob, ObR, or AR to produce the founder lines of transgenic animals. Such animals can be utilized as part of the screening methods of the invention, and cells and/or tissues from such animals can be obtained for generation of additional compositions (e.g., cell lines, tissue culture systems) that can also be utilized as part of the screening methods of the invention.

Techniques for generation of such animals are well known to those of skill in the art and include, but are not limited to, pronuclear microinjection (Hoppe & Wagner, 1989, U.S. Pat. No. 4,873,191); retrovirus mediated gene transfer into germ lines (Van der Putten et al., 1985, Proc. Natl. Acad. Sci., USA 82:6148-6152); gene targeting in embryonic stem cells (Thompson et al., 1989, Cell 56:313-321); electroporation of embryos (Lo, 1983, Mol Cell. Biol. 3:1803-1814); and sperm-mediated gene transfer (Lavitrano et a]., 1989, Cell 57:717-723); *etc.* For a review of such techniques, see Gordon, 1989, Transgenic Animals, Intl. Rev. Cytol. 115:171-229, which is incorporated by reference herein in its entirety.

With respect to transgenic animals containing a transgenic Ob, ObR, and/or AR, such animals can carry an Ob, ObR, or AR transgene in all their cells. Alternatively, such animals can carry the transgene or transgenes in some, but not all their cells, *i.e*., mosaic animals. The transgene may be integrated as a single transgene or in concatamers, e.g., head-to-head tandems or head-to-tail tandems. The transgene may also be selectively introduced into and activated in a particular cell type by following, for example, the teaching of Lasko et al., 1992, Proc. Natl.Acad. Sci. USA 89: 6232-6236. The regulatory sequences required for such a cell-type specific activation will depend upon the particular cell type of interest, and will be apparent to those of skill in the art. When it is desired that the transgene be integrated into the chromosomal site of the endogenous gene, gene targeting is preferred. Briefly, when such a technique is to be utilized, vectors containing some nucleotide sequences homologous to the endogenous Ob, ObR, or AR gene are designed for the purpose of integrating, via homologous recombination with chromosomal sequences, into and disrupting the function of the nucleotide sequence of the endogenous Ob, ObR, or AR gene, respectively. The transgene may also be selectively introduced into a particular cell type, thus inactivating the endogenous Ob, ObR, or AR gene in only that cell type, by following, for example, the teaching of Gu et al., 1994, Science 265: 103-106. The regulatory sequences required for such a cell-type specific inactivation will depend upon the particular cell type of interest, and will be apparent to those of skill in the art.

Once transgenic animals have been generated, the expression of the recombinant gene may be assayed utilizing standard techniques. Initial screening may be accomplished by Southern blot analysis or PCR techniques to analyze animal tissues to assay whether integration of the transgene has taken place. The level of mRNA expression of the transgene in the tissues of the transgenic animals may also be assessed using techniques which include, but are not limited to, Northern blot analysis of tissue samples obtained from the animal, *in situ* hybridization analysis, and RT-PCR. Samples of Ob, ObR, and AR gene-expressing tissue, may also be evaluated immunocytochemically using antibodies specific for the transgene product.

### 5.1.1. Antibodies to Ob, ObR, and AR Proteins

Antibodies that specifically recognize and bind to one or more epitopes of Ob, ObR, or AR or epitopes of conserved variants of Ob, ObR, or AR, or peptide fragments of Ob, ObR, or AR can be utilized as part of the methods of the present invention. Such antibodies include, but are not limited to, polyclonal antibodies, monoclonal antibodies (mAbs), human, humanized or chimeric antibodies, single chain antibodies, Fab fragments, F(ab')₂ fragments, fragments produced by a Fab expression library, anti-idiotypic (anti-Id) antibodies and epitope-binding fragments of any of the above.

Such antibodies may be used, for example, as part of the diagnostic or prognostic methods of the invention for diagnosing a bone disease in a mammal by measuring leptin levels in the mammal, e.g., leptin levels in blood serum or cerebrospinal fluid of the mammal. Such antibodies may also be utilized in conjunction with, for example, compound screening schemes, as described below, for the evaluation of the effect of test compounds on expression and/or activity of the Ob, ObR, or AR gene product. Additionally, such antibodies can be used in therapeutic and preventative methods of the invention. For example, such antibodies can correspond to leptin or adrenergic receptor agonists or antagonists. Further, such antibodies can be administered to lower leptin levels in the brain, as assayed by leptin levels in cerebrospinal fluid. In addition, such antibodies can be utilized to lower leptin levels by increasing the rate at which leptin is removed from circulation (e.g., can speed leptin breakdown), or can be used to lower leptin receptor levels, including lowering cells expressing leptin receptor, by increasing the rate at which leptin receptor (and cells expressing leptin receptor) breaks down or is degraded.

For the production of antibodies, various host animals may be immunized by injection with Ob, ObR, or AR; or ObR or AR peptide (e.g., for ObR, one corresponding with a functional domain of the receptor, such as ECD, TM or CD), truncated Ob, ObR, or AR polypeptides (e.g., for ObR or AR, in which one or more domains, e.g., the TM or CD, has been deleted), functional equivalents of Ob, ObR, or AR or mutants of Ob, ObR, or AR. Such host animals may include, but are not limited to, rabbits, mice, and rats, to name but a few. Various adjuvants may be used to increase the immunological response, depending on the host species, including but not limited to, Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and *Corynebacterium parvum.* Polyclonal antibodies are heterogeneous populations of antibody molecules derived from the sera of the immunized animals.

Monoclonal antibodies, which are homogeneous populations of antibodies to a particular antigen, may be obtained by any technique which provides for the production of antibody molecules by continuous cell lines in culture. These include, but are not limited to, the hybridoma technique of Kohler & Milstein, 1975, Nature 256:495-497 and U.S. Pat. No. 4,376,110, the human B-cell hybridoma technique (Kosbor et al., 1983, Immunology Today 4:72; Cole et al., 1983, Proc. Natl. Acad. Sci. USA 80:2026-2030), and the EBV-hybridoma technique (Cole et al., 1985, Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated *in vitro* or *in vivo.* Production of high titers of mAbs *in vivo* makes this the presently preferred method of production.

Additionally, recombinant antibodies, such as chimeric and humanized monoclonal antibodies, comprising both human and non-human portions, which can be made using standard recombinant DNA techniques, are within the scope of the invention. A chimeric antibody is a molecule in which different portions are derived from different animal species, such as those having a variable region derived from a murine mAb and a human immunoglobulin constant region (see, e.g., U.S. Patent Nos. 4,816,567 and 4,816397, which are incorporated herein by reference in their entireties). Humanized antibodies are antibody molecules from non-human species having one or more complementarily determining regions (CDRs) from the non-human species and a framework region from a human immunoglobulin molecule (see, e.g., U.S. Patent No. 5,585,089, which is incorporated herein by reference in its entirety). Such chimeric and humanized monoclonal antibodies can be produced by recombinant DNA techniques known in the art, for example using methods described in PCT Publication No. WO 87/02671; European Patent Application No. 184,187; European Patent Application No. 171,496; European Patent Application No. 173,494; PCT Publication No. WO 86/01533; U.S. Patent No. 4,816,567; European Patent Application No. 125,023; Better et al., 1988, Science 240:1041-1043; Liu et al., 1987, Proc. Natl. Acad. Sci. USA 84:3439-3443; Liu et al., 1987, J. Immunol. 139:3521-3526; Sun et al., 1987, Proc. Natl. Acad. Sci. USA 84:214-218; Nishimura et al., 1987, Canc. Res. 47:999-1005; Wood et al., 1985, Nature 314:446-449; and Shaw et al., 1988, J. Natl. Cancer Inst. 80:1553-1559; Morrison, 1985, Science 229:1202-1207; Oi et al., 1986, Bio/Techniques 4:214; U.S. Patent 5,225,539; Jones et al., 1986, Nature 321:552-525; Verhoeyan et al., 1988, Science 239:1534; and Beidler et al., 1988, J. Immunol. 141:4053-4060.

Completely human antibodies are particularly desirable for therapeutic treatment of human patients. Such antibodies can be produced, for example, using transgenic mice which are incapable of expressing endogenous immunoglobulin heavy and light chains genes, but which can express human heavy and light chain genes. The transgenic mice are immunized in the normal fashion with a selected antigen, e.g., all or a portion of a polypeptide of the invention. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA and IgE antibodies. For an overview of this technology for producing human antibodies, see Lonberg and Huszar , 1995, Int. Rev. Immunol. 13:65-93. For a detailed discussion of this technology for producing human antibodies and human monoclonal antibodies and protocols for producing such antibodies, see, e.g., U.S. Patent Nos. 5,625,126; 5,633,425; 5,569,825; 5,661,016; and 5,545,806. In addition, companies such as Abgenix, Inc. (Fremont, CA), can be engaged to provide human antibodies directed against a selected antigen using technology similar to that described above.

Completely human antibodies which recognize a selected epitope can be generated using a technique referred to as "guided selection." In this approach a selected non-human monoclonal antibody, e.g., a mouse antibody, is used to guide the selection of a completely human antibody recognizing the same epitope (Jespers et al., 1994, Bio/technology 12:899-903).

Alternatively, techniques described for the production of single chain antibodies (U.S. Pat. No. 4,946,778; Bird, 1988, Science 242:423-426; Huston et al., 1988, Proc. Natl. Acad. Sci. USA 85:5879-5883; and Ward et al., 1989, Nature 334:544-546) can be adapted to produce single chain antibodies against Ob and ObR gene products. Single chain antibodies are formed by linking the heavy and light chain fragments of the Fv region via an amino acid bridge, resulting in a single chain polypeptide.

Antibody fragments which recognize specific epitopes may be generated by known techniques. For example, such fragments include, but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (Huse et al., 1989, Science, 246:1275-1281) to allow rapid and easy identification of monoclonal Fab fragments with the desired specificity.

Antibodies to Ob, ObR, or AR can, in turn, be utilized to generate anti-idiotype antibodies that "mimic" Ob, ObR, or AR using techniques well known to those skilled in the art (see, e.g., Greenspan & Bona, 1993, FASEB J 7(5):437-444; and Nissinoff, 1991, J. Immunol. 147(8):2429-2438). For example, antibodies which bind to the ObR ECD and competitively inhibit the binding of Ob to the ObR can be used to generate anti-idiotypes that "mimic" the ECD and, therefore, bind and neutralize Ob. Such neutralizing anti-idiotypes or Fab fragments of such anti-idiotypes can be used in therapeutic regimens to neutralize Ob and treat bone disease characterized by a decreased bone mass relative to a corresponding non-diseased bone.

### 5.2. Diagnosis and Prognosis of Bone Disease and Compound/Patient Monitoring

A variety of methods can be employed for the diagnostic and prognostic evaluation of bone diseases or states, including, but not limited to, osteoporosis, osteopenia, faulty bone formation or resorption, Paget's disease, fractures and broken bones, bone metastasis, osteopetrosis, osteosclerosis and osteochondrosis and for the identification of subjects having a predisposition to such diseases or states.

In particular, bone diseases which can be diagnosed or prognosed in accordance with the present invention include bone diseases characterized by a decreased bone mass relative to that of corresponding non-diseased bone, including, but not limited to osteoporosis, osteopenia and Paget's disease.

An object of the present invention is the diagnosis or prognosis of a bone disease in a mammal comprising:
(a) measuring sympathetic tone and leptin activity in the mammal suspected of having the bone disease and
(b) comparing values measured in step (a) with corresponding control values.

Thus, in accordance with this aspect of the present invention, there is a method of diagnosing or prognosing a bone disease in a mammal, such as a human, comprising:
(a) measuring leptin levels in blood serum of a mammal, e.g., a mammal suspected of exhibiting or being at risk for the bone disease; and
(b) comparing the level measured in (a) to the leptin level in control blood serum,
so that if the level obtained in (a) is higher than that of the control, the mammal is diagnosed as exhibiting or being at risk for the bone disease, wherein the bone disease is characterized by a decreased bone mass relative to that of corresponding non-diseased bone.

Alternatively, there is a method of diagnosing or prognosing a bone disease in a mammal, such as a human, comprising:
(a) measuring leptin levels in cerebrospinal fluid of a mammal, *e.g.,* a mammal suspected of exhibiting or being at risk for the bone disease; and
(b) comparing the level measured in (a) to the leptin level in control cerebrospinal fluid,
so that if the level obtained in (a) is higher than that of the control, the mammal is diagnosed as exhibiting or being at risk for the bone disease, wherein the bone disease is characterized by a decreased bone mass relative to that of corresponding non-diseased bone.

Further, bone diseases which can be diagnosed or prognosed in accordance with the present invention also include bone diseases characterized by an increased bone mass relative to that of corresponding non-diseased bone, including, but not limited to osteopetrosis, osteosclerosis and osteochondrosis.

Thus, in accordance with this aspect of the present invention, there is a method of diagnosing or prognosing a bone disease in a mammal, such as a human, comprising:
(a) measuring leptin levels in blood serum of a mammal, e.g., a mammal suspected of exhibiting or being at risk for the bone disease; and
(b) comparing the level measured in (a) to the leptin level in control blood serum,
so that if the level obtained in (a) is lower than that of the control, the mammal is diagnosed as exhibiting or being at risk for the bone disease, wherein the bone disease is characterized by an increased bone mass relative to that of corresponding non-diseased bone.

Alternatively, there is a method of diagnosing or prognosing a bone disease in a mammal, such as a human, comprising:
(a) measuring leptin levels in cerebrospinal fluid of a mammal, e.g., a mammal suspected of exhibiting or being at risk for the bone disease; and
(b) comparing the level measured in (a) to the leptin level in control cerebrospinal fluid,
so that if the level obtained in (a) is lower than that of the control, the mammal is diagnosed as exhibiting or being at risk for the bone disease, wherein the bone disease is characterized by an increased bone mass relative to that of corresponding non-diseased bone.

Additionally, methods are provided for the diagnostic monitoring of patients undergoing clinical evaluation for the treatment of bone disease, and for monitoring the efficacy of compounds in clinical trials.

Thus, yet another aspect of the present invention, there is a method of monitoring efficacy of a compound for treating a bone disease in a mammal, such as a human, comprising:
(a) administering the compound to a mammal;
(b) measuring leptin levels in blood serum of the mammal; and
(c) comparing the level measured in (b) to the leptin level in blood serum of the mammal prior to administering the compound,
thereby monitoring the efficacy of the compound, wherein the bone disease is characterized by a decreased bone mass relative to that of corresponding non-diseased bone. Preferred compounds are ones that increase leptin levels relative to that observed prior to administration.

In accordance with another aspect of the present invention, there is a method of monitoring efficacy of a compound for treating a bone disease in a mammal, such as a human, comprising:
(a) administering the compound to a mammal;
(b) measuring leptin levels in cerebrospinal fluid of the mammal; and
(c) comparing the level measured in (b) to the leptin level in cerebrospinal fluid of the mammal prior to administering the compound,
thereby monitoring the efficacy of the compound, wherein the bone disease is characterized by a decreased bone mass relative to that of corresponding non-diseased bone. Preferred compounds are ones that increase leptin levels relative to that observed prior to administration.

In accordance with yet another aspect of the present invention, there is a method of monitoring efficacy of a compound for treating a bone disease in a mammal, such as a human, comprising:
(a) administering the compound to a mammal;
(b) measuring leptin levels in blood serum of the mammal; and
(c) comparing the level measured in (b) to the leptin level in blood serum of the mammal prior to administering the compound,
thereby monitoring the efficacy of the compound, wherein the bone disease is characterized by a increased bone mass relative to that of corresponding non-diseased bone. Preferred compounds are ones that decrease leptin levels relative to that observed prior to administration.

In accordance with another aspect of the present invention, there is a method of monitoring efficacy of a compound for treating a bone disease in a mammal, such as a human, comprising:
(a) administering the compound to a mammal;
(b) measuring leptin levels in cerebrospinal fluid of the mammal; and
(c) comparing the level measured in (b) to the leptin level in cerebrospinal fluid of the mammal prior to administering the compound,
thereby monitoring the efficacy of the compound, wherein the bone disease is characterized by a increased bone mass relative to that of corresponding non-diseased bone. Preferred compounds are ones that decrease leptin levels relative to that observed prior to administration.

Methods such as these can also be utilized for monitoring of patients undergoing clinical evaluation for treatment of bone disease. Generally, such methods further include a monitoring of bone mass relative to a corresponding non-diseased bone.

Methods described herein may, for example, utilize reagents such as the Ob and ObR nucleotide sequences described above and known to those of skill in the art (*See, e.g.,* U.S. Patent No. 5,972,621), and Ob and ObR antibodies, as described, in Section 5.1.1. Ob is typically expressed within adipocytes, and lower levels are also found in the stomach and in lymphocytes. ObR is typically expressed in the brain within the hypothalamus (Friedman & Halaas, 1998, Nature, 395:763-770). As such, such reagents may be used, for example, for: (1) the detection of the presence of Ob and ObR gene mutations, or the detection of either over- or under-expression of Ob or ObR mRNA relative to the non-bone diseased states, e.g., in a mammal's blood serum or in cerebrospinal fluid; (2) the detection of either an over- or an under-abundance of Ob or ObR gene product relative to the non-bone diseased states, e.g., in a mammal's blood serum or in cerebrospinal fluid; and (3) the detection of perturbations or abnormalities in the signal transduction pathway mediated by Ob or ObR. Alternatively, levels of phosphorylation of Stat3 protein can be measured relative to levels observed in a corresponding control sample or mammal. Stat3 phosphorylation is a biochemical event which occurs following binding of leptin to the leptin receptor (Devos et al., 1997, J. Biol. Chem. 272:18304-18310). In another embodiment, the methods involved herein also involve the measurement of the activity of dopamine β hydroxylase ("DBH"), the enzyme necessary for converting dopamine to norepinephrine.

The methods described herein may involve the measurement of the sympathetic tone of a mammal. Sympathetic tone relates to the relative state of activation or "tension" of the sympathetic nervous system. Sympathetic activity controls a variety of functions, and are measured in a number of ways, including, but not limited to, direct measurements of nerve firing rates (see, *e.g.,* Esler & Kay, 2000, J. Cardiovasc. Pharmacol. 35(7 Suppl 4):S1-7), levels of plasma catecholamines (see, *e.g.,* Urban et al., 1995, European Journal of Pharmacology, 282:29-37), variations in heat rate (see, *e.g.,* Boutouyrie et al., 1994, Am. J. Physiol. 267 (4 Pt 2):H1368-76), or renal sympathetic nerve activity (see, *e.g.,* Feng et al., 1992, Journal of Pharmacology and Experimental Therapeutics, 261:1129-1135). The methods described herein may be performed in conjunction with, prior to, or subsequent to techniques for measuring bone mass. For example, upon identifying a mammal (*e.g.,* human) exhibiting higher or lower levels of leptin (*e.g.,* in blood serum or cerebospinal fluid) relative to that of a corresponding control sample, bone mass of the individual can be measured to further clarify whether the mammal exhibits increased or decreased bone mass relative to a corresponding non-diseased bone. If no abnormal bone mass is observed, the mammal can be considered to be at risk for developing disease, while is an abnormal bone mass is observed, the mammal exhibits the bone disease.

Among the techniques well known to those of skill in the art for measuring bone mass are ones that include, but are not limited to, skeletal X-ray, which shows the lucent level of bone (the lower the lucent level, the higher the bone mass); classical bone histology (e.g., bone volume, number and aspects of trabiculi/trabiculations, numbers of osteoblast relative to controls and/or relative to osteoclasts); and dual energy X-ray absorptiometry (DEXA) (Levis & Altman, 1998, Arthritis and Rheumatism, 41:577-587) which measures bone mass and is commonly used in osteoporosis.

The methods described herein may further be used to diagnose individuals at risk for bone disease. Such individuals include, but are not limited to, peri-menopausal women (as used herein, this tem is meant to encompass a time frame from approximately 6 months prior to the onset of menopause to approximately 18 months subsequent to menopause) and patients undergoing treatment with corticosteroids, especially long-term corticosteroid treatment.

The methods described herein may be performed, for example, by utilizing pre-packaged diagnostic kits comprising at least one specific Ob , ObR, or AR nucleotide sequence or Ob, ObR, or AR antibody reagent, which may be conveniently used, e.g., in clinical settings, to diagnose patients exhibiting bone diseases.

For the detection of Ob, ObR. or AR mutations, any nucleated cell can be used as a starting source for genomic nucleic acid. For the detection of Ob, ObR, or AR gene expression or gene products, any cell type or tissue in which the Ob, ObR, or AR gene is expressed, such as, for example, choroid plexus cells for the ObR or AR, may be utilized.

Nucleic acid-based detection techniques are described below, in Section 5.2.1. Peptide detection techniques are described below, in Section 5.2.2.

### 5.2.1. Detection of Ob, ObR, and AR Gene and Transcripts

Mutations within the Ob, ObR, and AR gene can be detected by utilizing a number of techniques. Nucleic acid from any nucleated cell can be used as the starting point for such assay techniques, and may be isolated according to standard nucleic acid preparation procedures which are well known to those of skill in the art.

DNA may be used in hybridization or amplification assays of biological samples to detect abnormalities involving Ob, ObR, or AR gene structure, including point mutations, insertions, deletions and chromosomal rearrangements. Such assays may include, but are not limited to, Southern analyses, single stranded conformational polymorphism analyses (SSCP), and PCR analyses.

Such diagnostic methods for the detection of Ob, ObR, or AR gene-specific mutations can involve for example, contacting and incubating nucleic acids including recombinant DNA molecules, cloned genes or degenerate variants thereof, obtained from a sample, e.g., derived from a patient sample or other appropriate cellular source, with one or more labeled nucleic acid reagents including recombinant DNA molecules, cloned genes or degenerate variants thereof, under conditions favorable for the specific annealing of these reagents to their complementary sequences within the Ob, ObR, or AR gene, respectively. Preferably, the lengths of these nucleic acid reagents are at least 15 to 30 nucleotides. After incubation, all non-annealed nucleic acids are removed from the nucleic acid:Ob/ObR/AR molecule hybrid. The presence of nucleic acids which have hybridized, if any such molecules exist, is then detected. Using such a detection scheme, the nucleic acid from the cell type or tissue of interest can be immobilized, for example, to a solid support such as a membrane, or a plastic surface such as that on a microtiter plate or polystyrene beads. In this case, after incubation, non-annealed, labeled nucleic acid reagents are easily removed. Detection of the remaining, annealed, labeled Ob, ObR. or AR nucleic acid reagents is accomplished using standard techniques well-known to those in the art. The Ob, ObR, or AR gene sequences to which the nucleic acid reagents have annealed can be compared to the annealing pattern expected from a normal Ob, ObR, or AR gene sequence in order to determine whether an Ob, ObR, or AR gene mutation is present.

Alternative diagnostic methods for the detection of Ob, ObR, or AR gene specific nucleic acid molecules, in patient samples or other appropriate cell sources, may involve their amplification, *e.g.,* by PCR (the experimental embodiment set forth in U.S. Pat. No. 4,683,202), followed by the detection of the amplified molecules using techniques well known to those of skill in the art. The resulting amplified sequences can be compared to those which would be expected if the nucleic acid being amplified contained only normal copies of the Ob, ObR, or AR gene in order to determine whether an Ob, ObR, or AR gene mutation exists.

Additionally, well-known genotyping techniques can be performed to identify individuals carrying Ob, ObR, or AR gene mutations. Such techniques include, for example, the use of restriction fragment length polymorphisms (RFLPs), which involve sequence variations in one of the recognition sites for the specific restriction enzyme used.

Additionally, improved methods for analyzing DNA polymorphisms which can be utilized for the identification of Ob, ObR, or AR gene mutations have been described which capitalize on the presence of variable numbers of short, tandemly repeated DNA sequences between the restriction enzyme sites. For example, U.S. Pat. No. 5,075,217, which is incorporated herein by reference in its entirety, describes a DNA marker based on length polymorphisms in blocks of (dC-dA)n-(dG-dT)n short tandem repeats. The average separation of (dC-dA)n-(dG-dT)n blocks. is estimated to be 30,000-60,000 bp. Markers which are so closely spaced exhibit a high frequency co-inheritance, and are extremely useful in the identification of genetic mutations, such as, for example, mutations within the Ob or ObR gene, and the diagnosis of diseases and disorders related to Ob or ObR mutations.

Also, U.S. Pat. No. 5,364,759, which is incorporated herein by reference in its entirety, describe a DNA profiling assay for detecting short tri and tetra nucleotide repeat sequences. The process includes extracting the DNA of interest, such as the Ob, ObR, or AR gene, amplifying the extracted DNA, and labeling the repeat sequences to form a genotypic map of the individual's DNA.

The level of Ob, ObR, or AR gene expression can also be assayed by detecting and measuring Ob, ObR, or AR transcription, respectively. For example, RNA from a cell type or tissue known, or suspected to express the Ob, ObR, or AR gene, such as brain, especially choroid plexus cells for Ob and ObR, may be isolated and tested utilizing hybridization or PCR techniques such as are described, above. The isolated cells can be derived from cell culture or from a patient. The analysis of cells taken from culture may be a necessary step in the assessment of cells to be used as part of a cell-based gene therapy technique or, alternatively, to test the effect of compounds on the expression of the Ob, ObR, or AR gene. Such analyses may reveal both quantitative and qualitative aspects of the expression pattern of the Ob, ObR, or AR gene, including activation or inactivation of Ob, ObR, or AR gene expression.

In one embodiment of such a detection scheme, cDNAs are synthesized from the RNAs of interest *(e.g.,* by reverse transcription of the RNA molecule into cDNA). A sequence within the cDNA is then used as the template for a nucleic acid amplification reaction, such as a PCR amplification reaction, or the like. The nucleic acid reagents used as synthesis initiation reagents (e.g., primers) in the reverse transcription and nucleic acid amplification steps of this method are chosen from among Ob, ObR, and AR nucleic acid reagents which are well known to those of skill in the art. The preferred lengths of such nucleic acid reagents are at least 9-30 nucleotides. For detection of the amplified product, the nucleic acid amplification may be performed using radioactively or non-radioactively labeled nucleotides. Alternatively, enough amplified product may be made such that the product may be visualized by standard ethidium bromide staining or by utilizing any other suitable nucleic acid staining method.

Additionally, it is possible to perform such Ob, ObR, and AR gene expression assays *"in situ", i.e.,* directly upon tissue sections (fixed and/or frozen) of patient tissue obtained from biopsies or resections, such that no nucleic acid purification is necessary. Nucleic acid reagents which are well known to those of skill in the art may be used as probes and/or primers for such *in situ* procedures (see, *e.g.,* Nuovo, 1992, "PCR *In situ* Hybridization: Protocols And Applications", Raven Press, NY).

Alternatively, if a sufficient quantity of the appropriate cells can be obtained, standard Northern analysis can be performed to determine the level of mRNA expression of the Ob, ObR, and AR gene.

### 5.2.2. Detection of Ob, ObR, and AR Gene Products

Antibodies directed against wild type or mutant Ob, ObR, or AR gene products or conserved variants or peptide fragments thereof, which are discussed, above, in Section 5.1.1, may also be used as diagnostics and prognostics for bone disease, as described herein. Such diagnostic methods may be used to detect abnormalities in the level of Ob, ObR, or AR gene expression, or abnormalities in the structure and/or temporal, tissue, cellular, or subcellular location of Ob, ObR, or AR and may be performed *in vivo* or *in vitro,* such as, for example, on biopsy tissue.

For example, antibodies directed to epitopes of the AR ECD, ObR ECD, or Ob can be used *in vivo* to detect the pattern and level of expression of the AR, ObR, or Ob in the body. Such antibodies can be labeled, e.g., with a radio-opaque or other appropriate compound and injected into a subject in order to visualize binding to AR, ObR, or Ob expressed in the body using methods such as X-rays, CAT-scans, or MRI. Labeled antibody fragments, e.g., the Fab or single chain antibody comprising the smallest portion of the antigen binding region, are preferred for this purpose to promote crossing the blood-brain barrier and permit labeling ObRs expressed in the brain.

Additionally, any Ob, ObR, or AR fusion protein or Ob, ObR. or AR conjugated protein whose presence can be detected, can be administered. For example, Ob, ObR, or AR fusion or conjugated proteins labeled with a radio-opaque or other appropriate compound can be administered and visualized *in vivo,* as discussed, above for labeled antibodies. Further such fusion proteins can be utilized for *in vitro* diagnostic procedures.

Alternatively, immunoassays or fusion protein detection assays, as described above, can be utilized on biopsy and autopsy samples *in vitro* to permit assessment of the expression pattern of Ob, ObR, or AR. Such assays are not confined to the use of antibodies that define any particular epitope of Ob, ObR, or AR. The use of these labeled antibodies will yield useful information regarding translation and intracellular transport of Ob, ObR, and AR to the cell surface, and can identify defects in processing.

The tissue or cell type to be analyzed will generally include those which are known, or suspected, to express the Ob, ObR, or AR gene, such as, for example, the hypothalamus and choroid plexus cells for ObR; and adipocytes for Ob. The protein isolation methods employed herein may, for example, be such as those described in Harlow & Lane, 1988, "Antibodies: A Laboratory Manual", Cold Spring Harbor Laboratory Press, Cold Spring Harbor, N.Y., which is incorporated herein by reference in its entirety. The isolated cells can be derived from cell culture or from a patient. The analysis of cells taken from culture may be a necessary step in the assessment of cells that could be used as part of a cell-based gene therapy technique or, alternatively, to test the effect of compounds on the expression of the Ob, ObR, or AR gene.

For example, antibodies, or fragments of antibodies, such as those described, above, in Section 5.1.1, useful in the present invention may be used to quantitatively or qualitatively detect the presence of Ob, ObR, or AR gene products or conserved variants or peptide fragments thereof. This can be accomplished, for example, by immunofluorescence techniques employing a fluorescently labeled antibody (see below, this Section) coupled with light microscopic, flow cytometric, or fluorimetric detection. Such techniques are especially preferred if such Ob or ObR gene products are expressed on the cell surface.

The antibodies (or fragments thereof) or Ob, ObR, or AR fusion or conjugated proteins useful in the present invention may, additionally, be employed histologically, as in immunofluorescence, immunoelectron microscopy or non-immuno assays, for *in situ* detection of Ob, ObR, and AR gene products or conserved variants or peptide fragments thereof, or for Ob binding (in the case of labeled Ob fusion protein).

*In situ* detection may be accomplished by removing a histological specimen from a patient, and applying thereto a labeled antibody or fusion protein of the present invention. The antibody (or fragment) or fusion protein is preferably applied by overlaying the labeled antibody (or fragment) onto a biological sample. Through the use of such a procedure, it is possible to determine not only the presence of the Ob, ObR, or AR gene product, or conserved variants or peptide fragments, or Ob binding or catecholamine binding, but also its distribution in the examined tissue. Using the present invention, those of ordinary skill will readily perceive that any of a wide variety of histological methods (such as staining procedures) can be modified in order to achieve such *in situ* detection.

Immunoassays and non-immunoassays for Ob, ObR, and AR gene products or conserved variants or peptide fragments thereof will typically comprise incubating a sample, such as a biological fluid (e.g., blood serum or cerebrospinal fluid), a tissue extract, freshly harvested cells, or lysates of cells which have been incubated in cell culture, in the presence of a detectably labeled antibody capable of identifying Ob, ObR, or AR gene products or conserved variants or peptide fragments thereof, and detecting the bound antibody by any of a number of techniques well-known in the art.

The biological sample may be brought in contact with and immobilized onto a solid phase support or carrier such as nitrocellulose, or other solid support which is capable of immobilizing cells, cell particles or soluble proteins. The support may then be washed with suitable buffers followed by treatment with the detectably labeled Ob, ObR, or AR antibody or Ob, ObR, or AR fusion protein. The solid phase support may then be washed with the buffer a second time to remove unbound antibody or fusion protein. The amount of bound label on solid support may then be detected by conventional means.

By "solid phase support or carrier" is intended any support capable of binding an antigen or an antibody. Well-known supports or carriers include glass, polystyrene, polypropylene, polyethylene, dextran, nylon, amylases, natural and modified celluloses, polyacrylamides, gabbros, and magnetite. The nature of the carrier can be either soluble to some extent or insoluble for the purposes of the present invention. The support material may have virtually any possible structural configuration so long as the coupled molecule is capable of binding to an antigen or antibody. Thus, the support configuration may be spherical, as in a bead, or cylindrical, as in the inside surface of a test tube, or the external surface of a rod. Alternatively, the surface may be flat such as a sheet, test strip, etc. Preferred supports include polystyrene beads. Those skilled in the art will know many other suitable carriers for binding antibody or antigen, or will be able to ascertain the same by use of routine experimentation.

The binding activity of a given lot of Ob, ObR, or AR antibody or Ob, ObR, or AR fusion protein may be determined according to well known methods. Those skilled in the art will be able to determine operative and optimal assay conditions for each determination by employing routine experimentation.

With respect to antibodies, one of the ways in which the Ob, ObR, or AR antibody can be detectably labeled is by linking the same to an enzyme and use in an enzyme immunoassay (EIA) (Voller, "The Enzyme Linked Immunosorbent Assay (ELISA)", 1978, Diagnostic Horizons 2:1-7, Microbiological Associates Quarterly Publication, Walkersville, Md.); Voller et al., 1978, J. Clin. Pathol. 31:507-520; Butler, 1981, Meth. Enzymol. 73:482-523; Maggio (ed.), 1980, Enzyme Immunoassay, CRC Press, Boca Raton, Fla.,; Ishikawa et al., (eds.), 1981, Enzyme Immunoassay, Kgaku Shoin, Tokyo). The enzyme which is bound to the antibody will react with an appropriate substrate, preferably a chromogenic substrate, in such a manner as to produce a chemical moiety which can be detected, for example, by spectrophotometric, fluorimetric or by visual means. Enzymes which can be used to detectably label the antibody include, but are not limited to, malate dehydrogenase, staphylococcal nuclease, delta-5-steroid isomerase, yeast alcohol dehydrogenase, alphaglycerophosphate, dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase and acetylcholinesterase. The detection can be accomplished by calorimetric methods which employ a chromogenic substrate for the enzyme. Detection may also be accomplished by visual comparison of the extent of enzymatic reaction of a substrate in comparison with similarly prepared standards.

Detection may also be accomplished using any of a variety of other immunoassays. For example, by radioactively labeling the antibodies or antibody fragments, it is possible to detect Ob, ObR, or AR through the use of a radioimmunoassay (RIA) (see, e.g., Weintraub, Principles of Radioimmunoassays, Seventh Training Course on Radioligand Assay Techniques, The Endocrine Society, March, 1986, which is incorporated by reference herein). The radioactive isotope can be detected by such means as the use of a gamma counter or a scintillation counter or by autoradiography.

It is also possible to label the antibody with a fluorescent compound. When the fluorescently labeled antibody is exposed to light of the proper wave length, its presence can then be detected due to fluorescence. Among the most commonly used fluorescent labeling compounds are fluorescein isothiocyanate, rhodamine, phycoerythrin, phycocyanin, allophycocyanin, o-phthaldehyde and fluorescamine.

The antibody can also be detectably labeled using fluorescence emitting metals such as ¹⁵²Eu, or others of the lanthanide series. These metals can be attached to the antibody using such metal chelating groups as diethylenetriaminepentacetic acid (DTPA) or ethylenediaminetetraacetic acid (EDTA).

The antibody also can be detectably labeled by coupling it to a chemiluminescent compound. The presence of the chemiluminescent-tagged antibody is then determined by detecting the presence of luminescence that arises during the course of a chemical reaction: Examples of particularly useful chemiluminescent labeling compounds are luminol, isoluminol, theromatic acridinium ester, imidazole, acridinium salt and oxalate ester.

Likewise, a bioluminescent compound may be used to label the antibody of the present invention. Bioluminescence is a type of chemiluminescence found in biological systems in, which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

### 5.3. Screening Assays for Compounds Useful in the Treatment, Diagnosis and Prevention of Bone Disease

The present invention also provides screening methods (*e.g.,* assays) for the identification of compounds which affect bone disease. The invention further encompasses agonists and antagonists of leptin and leptin receptors and catecholamines and adrenergic receptors, including small molecules, large molecules, and antibodies, as well as nucleotide sequences that can be used to inhibit leptin, leptin receptor, and adrenergic receptor gene expression (e.g., antisense and ribozyme molecules), and gene or regulatory sequence replacement constructs designed to enhance leptin, leptin receptor, or adrenergic gene expression (e.g., expression constructs that place the leptin, leptin receptor, or adrenergic receptor gene under the control of a strong promoter system). Such compounds may be used to treat bone diseases.

In particular, cellular and non-cellular assays are described that can be used to identify compounds that interact with leptin, leptin receptors. or adrenergic receptors, e.g., modulate the activity of leptin and leptin receptors or catecholamines and adrenergic receptors and/or bind to the leptin receptor or adrenergic receptor. The cell based assays can be used to identify compounds or compositions that affect the signal-transduction activity of leptin and leptin receptors and/or catecholamines and adrenergic receptors, whether they bind to the leptin receptor or catecholamine receptor, or act on intracellular factors involved in the leptin signal transduction pathway or adrenergic signal transduction pathway. Such cell-based assays of the invention utilize cells, cell lines, or engineered cells or cell lines that express leptin, leptin receptors, or adrenergic receptors. The cells can be further engineered to incorporate a reporter molecule linked to the signal transduced by the activated leptin receptor or adrenergic receptor to aid in the identification of compounds that modulate leptin and leptin receptors signaling activity and/or adrenergic signaling activity.

The invention also encompasses the use of cell-based assays or cell-lysate assays (*e.g., in vitro* transcription or translation assays) to screen for compounds or compositions that modulate leptin, leptin receptor, and adrenergic receptor gene expression. To this end, constructs containing a reporter sequence linked to a regulatory element of the leptin or leptin receptor genes can be used in engineered cells, or in cell lysate extracts, to screen for compounds that modulate the expression of the reporter gene product at the level of transcription. For example, such assays could be used to identify compounds that modulate the expression or activity of transcription factors involved in leptin, leptin receptor, and adrenergic receptor gene expression, or to test the activity of triple helix polynucleotides. Alternatively, engineered cells or translation extracts can be used to screen for compounds (including antisense and ribozyme constructs) that modulate the translation of leptin, leptin receptors, and adrenergic receptors from mRNA transcripts, and therefore, affect expression of the leptin receptor and/or the adrenergic receptor.

The following assays are designed to identify compounds that interact with (e.g., bind to) Ob, ObR, or AR (including, but not limited to, the ECD or CD of ObR or AR), compounds that interact with (e.g., bind to) intracellular proteins that interact with Ob, ObR, or AR (including, but not limited to, the TM and CD of ObR or AR), compounds that interfere with the interaction of Ob, ObR, or AR with transmembrane or intracellular proteins involved in ObR-mediated signal transduction or adrenergic signal transduction, and to compounds which modulate the activity of Ob, ObR, or AR gene expression or modulate the level of Ob, ObR, or AR. Assays may additionally be utilized which identify compounds which bind to Ob, ObR, or AR gene regulatory sequences (e.g., promoter sequences) and which may modulate Ob, ObR, or AR gene expression (see *e.g.,* Platt, 1994, J. Biol. Chem. 269:28558-28562). Upon identification, compounds can further be tested for an ability to modulate leptin signaling in vitro or in vivo, and can still further be tested for an ability to modulate bone mass (that is, increase or decrease bone mass) and to treat a bone disease characterized by a decreased or an increased bone mass relative to a corresponding non-diseased bone.

Thus, in accordance with this aspects of the present invention, there is a method for identifying a compound to be tested for an ability to modulate (increase or decrease) bone mass in a mammal, comprising:
(a) contacting a test compound with a polypeptide; and
(b) determining whether the test compound binds the polypeptide, so that if the test compound binds the polypeptide, then a compound to be tested for an ability to modulate bone mass is identified, wherein the polypeptide is selected from the group consisting of a leptin polypeptide, a leptin receptor polypeptide, and an adrenergic receptor polypeptide.

Alternatively, there is a method for identifying a compound that modulates (increases or decreases) bone mass in a mammal, comprising:
(a) contacting test compounds with a polypeptide;
(b) identifying a test compound that binds the polypeptide; and
(c) administering the test compound in (b) to a non-human mammal, and determining whether the test compound modulates bone mass in the mammal relative to that of a corresponding bone in an untreated control non-human mammal, wherein the polypeptide is selected from the group consisting of a leptin polypeptide , a leptin receptor polypeptide, and an adrenergic receptor polypeptide, so that if the test compound modulates bone mass, then a compound that modulates bone mass in a mammal is identified.

In accordance with this, and other aspects of the present invention, a control non-human mammal, as used herein, is intended to mean a corresponding mammal that has not been administered the test compound.

In accordance with yet another aspect of the present invention, there is a method for identifying a compound to be tested for an ability to modulate (increase or decrease) bone mass in a mammal, comprising:
(a) contacting a test compound with a leptin polypeptide and a leptin receptor polypeptide for a time sufficient to form leptin/leptin receptor complexes; and
(b) measuring leptin/leptin receptor complex level, so that if the level measured differs from that measured in the absence of the test compound, then a compound to be tested for an ability to modulate bone mass is identified.

In accordance with yet another aspect of the present invention, there is a method for identifying a compound to be tested for an ability to modulate (increase or decrease) bone mass in a mammal, comprising:
(a) contacting a test compound with a catecholamine and an adrenergic receptor polypeptide for a time sufficient to form catecholamine/adrenergic receptor complexes; and
(b) measuring catecholamine/adrenergic receptor complex level, so that if the level measured differs from that measured in the absence of the test compound, then a compound to be tested for an ability to modulate bone mass is identified.

In accordance with this, and other aspects of the present invention, leptin/leptin receptor and/or catecholamine/adrenergic receptor complex formation can be measured by, for example, isolating the complex and determining the amount complex formation by various assays well known to those of skill in the art, e.g., Western Blot.

In accordance with another aspect of the present invention, there is a method for identifying a compound to be tested for an ability to decrease bone mass in a mammal, comprising:
(a) contacting a test compound with a cell which expresses a functional leptin receptor; and
(b) determining whether the test compound activates the leptin receptor,
wherein if the compound activates the leptin receptor a compound to be tested for an ability to decrease bone mass in a mammal is identified.

In accordance with this, and other aspects of the present invention, a functional leptin receptor is a leptin receptor which is capable of signal transduction following ligand binding to the active site of the receptor. Activation of the leptin receptor, as used herein, is any increase in the activity (*i.e.,* signal transduction) of the leptin receptor.

In accordance with another aspect of the present invention, there is a method for identifying a compound to be tested for an ability to decrease bone mass in a mammal, comprising:
(a) contacting a test compound with a cell which expresses a functional adrenergic receptor; and
(b) determining whether the test compound activates the adrenergic receptor,
wherein if the compound activates the adrenergic receptor a compound to be tested for an ability to decrease bone mass in a mammal is identified.

In accordance with this, and other aspects of the present invention, a functional adrenergic receptor is an adrenergic receptor which is capable of signal transduction following ligand binding to the active site of the receptor. Activation of the adrenergic receptor, as used herein, is any increase in the activity (*i.e*., signal transduction) of the adrenergic receptor.

In accordance with another aspect of the present invention, there is a method for identifying a compound that decreases bone mass in a mammal, comprising:
(a) contacting a test compound with a cell that expresses a functional leptin receptor, and determining whether the test compound activates the leptin receptor;
(b) administering a test compound identified in (a) as activating the leptin receptor to a non-human animal, and determining whether the test compound decreases bone mass of the animal relative to that of a corresponding bone of a control non-human animal, so that if the test compound decreases bone mass, then a compound that decreases bone mass in a mammal is identified.

In accordance with another aspect of the present invention, there is a method for identifying a compound that decreases bone mass in a mammal, comprising:
(a) contacting a test compound with a cell that expresses a functional adrenergic receptor, and determining whether the test compound activates the adrenergic receptor;
(b) administering a test compound identified in (a) as activating the adrenergic receptor to a non-human animal, and determining whether the test compound decreases bone mass of the animal relative to that of a corresponding bone of a control non-human animal, so that if the test compound decreases bone mass, then a compound that decreases bone mass in a mammal is identified.

In accordance with another aspect of the present invention, there is a method for identifying a compound to be tested for an ability to increase bone mass in a mammal, comprising:
(a) contacting a leptin polypeptide and a test compound with a cell that expresses a functional leptin receptor; and
(b) determining whether the test compound lowers activation of the leptin receptor relative to that observed in the absence of the test compound; wherein a test compounds that lowers activation of the leptin receptor is identified as a compound to be tested for an ability to increase bone mass in a mammal.

In accordance with another aspect of the present invention, there is a method for identifying a compound to be tested for an ability to increase bone mass in a mammal, comprising:
(a) contacting a catecholamine and a test compound with a cell that expresses a functional adrenergic receptor; and
(b) determining whether the test compound lowers activation of the adrenergic receptor relative to that observed in the absence of the test compound; wherein a test compounds that lowers activation of the adrenergic receptor is identified as a compound to be tested for an ability to increase bone mass in a mammal.

In accordance with yet another aspect of the present invention, there is a method for identifying a compound that increases bone mass in a mammal, comprising:
(a) contacting a leptin polypeptide and a test compound with a cell that expresses a functional leptin receptor, and determining whether the test compound decreases activation of the leptin receptor;
(b) administering a test compound identified in (a) as decreasing leptin receptor to a non-human animal, and determining whether the test compound increases bone mass of the animal relative to that of a corresponding bone of a control non-human animal, so that if the test compound increases bone mass, then a compound that increases bone mass in a mammal is identified.

In accordance with yet another aspect of the present invention, there is a method for identifying a compound that increases bone mass in a mammal, comprising:
(a) contacting a catecholamine and a test compound with a cell that expresses a functional adrenergic receptor, and determining whether the test compound decreases activation of the adrenergic receptor;
(b) administering a test compound identified in (a) as decreasing adrenergic receptor to a non-human animal, and determining whether the test compound increases bone mass of the animal relative to that of a corresponding bone of a control non-human animal, so that if the test compound increases bone mass, then a compound that increases bone mass in a mammal is identified.

In accordance with yet another aspect of the invention, there is a method in which activation of a leptin receptor is determined by measuring levels of phosphorylated Stat3 polypeptide. Stat3 polypeptide, a downstream effector of leptin signaling in its target cells (Tartaglia et al., 1995, Cell 83, 1263-1271; Baumann et al., 1996, Proc Natl Acad Sci USA 93, 8374-8378; Ghilardi et al., 1996, Proc Natl Acad Sci USA 93, 6231-6235; Vaisse et al., 1996, Nat Genet 14:95-97), is phosphorylated following activation of the leptin receptor by leptin.

The compounds which may be screened in accordance with the invention include, but are not limited to, peptides, antibodies and fragments thereof, and other organic compounds (e.g., peptidomimetics) that bind to Ob, ObR, or AR and either mimic the activity triggered by the natural ligand *(i.e.,* agonists) or inhibit the activity triggered by the natural ligand (*i.e.,* antagonists); as well as peptides, antibodies or fragments thereof, and other organic compounds that mimic the ECD of the ObR or AR (or a portion thereof) and bind to and "neutralize" natural ligand. Additional compounds which may be screened in accordance with the invention include, but are not limited to, compounds which interact with Ob and prevent the transport of Ob across the blood-brain barrier, thereby preventing Ob from activating the ObR.

Such compounds may include, but are not limited to, peptides such as, for example, soluble peptides, including but not limited to members of random peptide libraries; (see, *e.g.,* Lam, K. S. et al., 1991, Nature 354:82-84; Houghten, R. et al., 1991, Nature 354:84-86), and combinatorial chemistry-derived molecular library made of D- and/or L- configuration amino acids, phosphopeptides (including, but not limited to, members of random or partially degenerate, directed phosphopeptide libraries; see, *e.g.,* Songyang et a]., 1993, Cell 72:767-778), antibodies (including, but not limited to, polyclonal, monoclonal, human, humanized, anti-idiotypic, chimeric or single chain antibodies, and FAb, F(ab')₂ and FAb expression library fragments, and epitope-binding fragments thereof), and small organic or inorganic molecules.

Other compounds which can be screened in accordance with the invention include, but are not limited to, small organic molecules that are able to cross the blood-brain barrier, gain entry into an appropriate cell (e.g., in the choroid plexus or in the hypothalamus) and affect the expression of the Ob, ObR, or AR gene or some other gene involved in the ObR signal transduction pathway or adrenergic signal transduction pathway (e.g., by interacting with the regulatory region or transcription factors involved in gene expression); or such compounds that affect the activity of the ObR or the AR (e.g., by inhibiting or enhancing the enzymatic activity of the CD) or the activity of some other intracellular factor involved in the ObR signal transduction pathway, such as, for example, gp130.

Computer modeling and searching technologies permit identification of compounds, or the improvement of already identified compounds, that can modulate Ob, ObR, or AR expression or activity. Having identified such a compound or composition, the active sites or regions are identified. Such active sites might typically be ligand binding sites, such as the interaction domains of Ob with ObR itself. The active site can be identified using methods known in the art including, for example, from the amino acid sequences of peptides, from the nucleotide sequences of nucleic acids, or from study of complexes of the relevant compound or composition with its natural ligand. In the latter case, chemical or X-ray crystallographic methods can be used to find the active site by finding where on the factor the complexed ligand is found. Next, the three dimensional geometric structure of the active site is determined. This can be done by known methods, including X-ray crystallography, which can determine a complete molecular structure. On the other hand, solid or liquid phase NMR can be used to determine certain intra-molecular distances. Any other experimental method of structure determination can be used to obtain partial or complete geometric structures. The geometric structures may be measured with a complexed ligand, natural or artificial, which may increase the accuracy of the active site structure determined.

If an incomplete or insufficiently accurate structure is determined, the methods of computer based numerical modeling can be used to complete the structure or improve its accuracy. Any recognized modeling method may be used, including parameterized models specific to particular biopolymers such as proteins or nucleic acids, molecular dynamics models based on computing molecular motions, statistical mechanics models based on thermal ensembles, or combined models. For most types of models, standard molecular force fields, representing the forces between constituent atoms and groups, are necessary, and can be selected from force fields known in physical chemistry. The incomplete or less accurate experimental structures can serve as constraints on the complete and more accurate structures computed by these modeling methods.

Finally, having determined the structure of the active site, either experimentally, by modeling, or by a combination, candidate modulating compounds can be identified by searching databases containing compounds along with information on their molecular structure. Such a search seeks compounds having structures that match the determined active site structure and that interact with the groups defining the active site. Such a search can be manual, but is preferably computer assisted. These compounds found from this search are potential Ob, ObR, or AR modulating compounds.

Alternatively, these methods can be used to identify improved modulating compounds from an already known modulating compound or ligand. The composition of the known compound can be modified and the structural effects of modification can be determined using the experimental and computer modeling methods described above applied to the new composition. The altered structure is then compared to the active site structure of the compound to determine if an improved fit or interaction results. In this manner, systematic variations in composition, such as by varying side groups, can be quickly evaluated to obtain modified modulating compounds or ligands of improved specificity or activity.

Further experimental and computer modeling methods useful to identify modulating compounds based upon identification of the active sites of Ob, ObR, AR, and related transduction and transcription factors will be apparent to those of skill in the art.

Examples of molecular modeling systems are the CHARMm and QUANTA programs (Polygen Corporation, Waltham, Mass.). CHARMm performs the energy minimization and molecular dynamics functions. QUANTA performs the construction, graphic modelling and analysis of molecular structure. QUANTA allows interactive construction, modification, visualization, and analysis of the behavior of molecules with each other.

A number of articles review computer modeling of drugs interactive with specific-proteins, such as Rotivinen et al., 1988, Acta Pharmaceutical Fennica 97:159-166; Ripka, New Scientist 54-57 (Jun. 16, 1988); McKinaly and Rossmann, 1989, Annu. Rev. Pharmacol. Toxiciol. 29:111-122; Perry & Davies, OSAR: Quantitative Structure-Activity Relationships in Drug Design pp. 189-193 (Alan R. Liss, Inc. 1989); Lewis & Dean, 1989 Proc. R. Soc. Lond. 236:125-140 and 141-162; and, with respect to a model receptor for nucleic acid components, Askew, et al., 1989, J. Am. Chem. Soc. 111:1082-1090. Other computer programs that screen and graphically depict chemicals are available from companies such as BioDesign, Inc. (Pasadena, Calif.), Allelix, Inc. (Mississauga, Ontario, Canada), and Hypercube, Inc. (Cambridge, Ontario). Although these are primarily designed for application to drugs specific to particular proteins, they can be adapted to design of drugs specific to regions of DNA or RNA, once that region is identified.

Although described above with reference to design and generation of compounds which could alter binding, one could also screen libraries of known compounds, including natural products or synthetic chemicals, and biologically active materials, including proteins, for compounds which are inhibitors or activators.

Compounds identified via assays such as those described herein may be useful, for example, in elaborating the biological function of the Ob, ObR, or AR gene product, and for ameliorating bone diseases. Assays for testing the effectiveness of compounds, identified by, for example, techniques such as those described in Section 5.3.1 through 5.3.3, are discussed, below, in Section 5.3.4.

### 5.3.1. In vitro Screening Assays for Compounds that Bind to Ob, ObR, and AR

*In vitro* systems may be designed to identify compounds capable of interacting with (*e*.*g*., binding to) Ob, ObR, and AR (including, but not limited to, the ECD or CD of ObR and AR). Compounds identified may be useful, for example, in modulating the activity of wild type and/or mutant Ob, ObR, and AR gene products; may be useful in elaborating the biological function of Ob, ObR, or AR; may be utilized in screens for identifying compounds that disrupt normal Ob, ObR, and AR interactions; or may in themselves disrupt such interactions.

The principle of the assays used to identify compounds that bind to Ob, ObR, or AR involves preparing a reaction mixture of Ob, ObR, or AR and the test compound under conditions and for a time sufficient to allow the two components to interact and bind, thus forming a complex which can be removed and/or detected in the reaction mixture. The Ob, ObR, or AR species used can vary depending upon the goal of the screening assay. For example, where agonists of the natural ligand are sought, the full length ObR, or a soluble truncated ObR, e.g., in which the TM and/or CD is deleted from the molecule, a peptide corresponding to the ECD or a fusion protein containing the ObR ECD fused to a protein or polypeptide that affords advantages in the assay system (e.g., labeling, isolation of the resulting complex, etc.) can be utilized. Where compounds that interact with the ObR cytoplasmic domain are sought to be identified, peptides corresponding to the ObR CD and fusion proteins containing the ObR CD can be used. In addition, where compounds which will prevent Ob entry across the blood-brain barrier are sought, Ob, or soluble forms of Ob, can be used. Similar approaches using a full length AR or a soluble truncated AR can also be used.

The screening assays can be conducted in a variety of ways. For example, one method to conduct such an assay would involve anchoring the Ob, ObR, or AR protein, polypeptide, peptide or fusion protein or the test substance onto a solid phase and detecting Ob, ObR, or AR/test compound complexes anchored on the solid phase at the end of the reaction. In one embodiment of such a method, the Ob, ObR, or AR reactant may be anchored onto a solid surface, and the test compound, which is not anchored, may be labeled, either directly or indirectly.

In practice, microtiter plates may conveniently be utilized as the solid phase. The anchored component may be immobilized by non-covalent or covalent attachments. Non-covalent, attachment may be accomplished by simply coating the solid surface with a solution of the protein and drying. Alternatively, an immobilized antibody, preferably a monoclonal antibody, specific for the protein to be immobilized may be used to anchor the protein to the solid surface. The surfaces may be prepared in advance and stored.

In order to conduct the assay, the nonimmobilized component is added to the coated surface containing the anchored component. After the reaction is complete, unreacted components are removed (*e.g*., by washing) under conditions such that any complexes formed will remain immobilized on the solid surface. The detection of complexes anchored on the solid surface can be accomplished in a number of ways. Where the previously nonimmobilized component is pre-labeled, the detection of label immobilized on the surface indicates that complexes were formed. Where the previously nonimmobilized component is not pre-labeled, an indirect label can be used to detect complexes anchored on the surface; e.g., using a labeled antibody specific for the previously nonimmobilized component (the antibody, in turn, may be directly labeled or indirectly labeled with a labeled anti-Ig antibody).

Alternatively, a reaction can be conducted in a liquid phase, the reaction products separated from unreacted components, and complexes detected; e.g., using an immobilized antibody specific for Ob, ObR, or AR protein, polypeptide, peptide or fusion protein or the test compound to anchor any complexes formed in solution, and a labeled antibody specific for the other component of the possible complex to detect anchored complexes.

Alternatively, cell-based assays can be used to identify compounds that interact with Ob, ObR, or AR. To this end, cell lines that express Ob, ObR, or AR, or cell lines (e.g., COS cells, CHO cells, fibroblasts, etc.) that have been genetically engineered to express Ob, ObR, or AR (e.g., by transfection or transduction of Ob, ObR, or AR DNA) can be used. Interaction of the test compound with, for example, the ECD of ObR expressed by the host cell can be determined by comparison or competition with native Ob.

### 5.3.2. Assays for Proteins that Interact with Ob, ObR, and AR

Any method suitable for detecting protein-protein interactions may be employed for identifying transmembrane proteins or intracellular proteins that interact with Ob, ObR, or AR. Among the traditional methods which may be employed are co-immunoprecipitation, crosslinking and co-purification through gradients or chromatographic columns of cell lysates or proteins obtained from cell lysates and Ob, ObR, or AR to identify proteins in the lysate that interact with Ob, ObR, or AR. For these assays, the Ob, ObR, or AR component used can be full length, a soluble derivative lacking the membrane-anchoring region (e.g., a truncated ObR or AR in which the TM is deleted resulting in a truncated molecule containing the ECD fused to the CD), a peptide corresponding to the CD or a fusion protein containing Ob or the CD of ObR or AR. Once isolated, such an intracellular protein can be identified and can, in turn, be used in conjunction with standard techniques, to identify proteins with which it interacts. For example, at least a portion of the amino acid sequence of an intracellular protein which interacts with Ob, ObR, or AR can be ascertained using techniques well known to those of skill in the art, such as via the Edman degradation technique (see, e.g., Creighton, 1983, "Proteins: Structures and Molecular Principles", W.H. Freeman & Co., N.Y., pp.34-49). The amino acid sequence obtained may be used as a guide for the generation of oligonucleotide mixtures that can be used to screen for gene sequences encoding such intracellular proteins. Screening may be accomplished, for example, by standard hybridization or PCR techniques. Techniques for the generation of oligonucleotide mixtures and the screening are well-known (see, e.g., Ausubel, supra., and PCR Protocols: A Guide to Methods and Applications, 1990, Innis, M. et al., eds. Academic Press, Inc., New York).

Additionally, methods may be employed which result in the simultaneous identification of genes which encode the transmembrane or intracellular proteins interacting with ObR, Ob, or AR. These methods include, for example, probing expression libraries in a manner similar to the well known technique of antibody probing of λgt11 libraries, using labeled Ob, ObR, or AR protein, or an Ob, ObR, or AR polypeptide, peptide or fusion protein, *e.g*., an Ob, ObR, or AR polypeptide or an Ob, ObR, or AR domain fused to a marker (e.g., an enzyme, fluor, luminescent protein, or dye), or an Ig-Fc domain.

One method which detects protein interactions *in vivo,* the two-hybrid system, is described in detail for illustration only and not by way of limitation. One version of this system has been described (Chien et al., 1991, Proc. Natl. Acad. Sci. USA, 88:9578-9582) and is commercially available from Clontech (Palo Alto, Calif.).

Briefly, utilizing such a system, plasmids are constructed that encode two hybrid proteins: one plasmid consists of nucleotides encoding the DNA-binding domain of a transcription activator protein fused to an Ob, ObR, or Ar nucleotide sequence encoding Ob, ObR, or AR, an Ob, ObR, or AR polypeptide; peptide or fusion protein, and the other plasmid consists of nucleotides encoding the transcription activator protein's activation domain fused to a cDNA encoding an unknown protein which has been recombined into this plasmid as part of a cDNA library. The DNA-binding domain fusion plasmid and the cDNA library are transformed into a strain of the yeast *Saccharomyces cerevisiae* that contains a reporter gene (e.g., HBS or lacZ) whose regulatory region contains the transcription activator's binding site. Either hybrid protein alone cannot activate transcription of the reporter gene: the DNA-binding domain hybrid cannot because it does not provide activation function and the activation domain hybrid cannot because it cannot localize to the activator's binding sites. Interaction of the two hybrid proteins reconstitutes the functional activator protein and results in expression of the reporter gene, which is detected by an assay for the reporter gene product.

The two-hybrid system or related methodology may be used to screen activation domain libraries for proteins that interact with the "bait" gene product. By way of example, and not by way of limitation, Ob, ObR, or AR may be used as the bait gene product. Total genomic or cDNA sequences are fused to the DNA encoding an activation domain. This library and a plasmid encoding a hybrid of a bait Ob, ObR, or AR gene product fused to the DNA-binding domain are cotransformed into a yeast reporter strain, and the resulting transformants are screened for those that express the reporter gene. For example, and not by way of limitation, a bait Ob, ObR, or AR gene sequence, such as the open reading frame of Ob, ObR, or AR (or a domain of ObR or AR), can be cloned into a vector such that it is translationally fused to the DNA encoding the DNA-binding domain of the GAL4 protein. These colonies are purified and the library plasmids responsible for reporter gene expression are isolated. DNA sequencing is then used to identify the proteins encoded by the library plasmids.

A cDNA library of the cell line from which proteins that interact with bait Ob, ObR, or AR gene product are to be detected can be made using methods routinely practiced in the art. According to the particular system described herein, for example, the cDNA fragments can be inserted into a vector such that they are translationally fused to the transcriptional activation domain of GAL4. This library can be co-transformed along with the bait Ob, ObR, or AR gene-GAL4 fusion plasmid into a yeast strain which contains a lacZ gene driven by a promoter which contains GAL4 activation sequence. A cDNA encoded protein, fused to GAL4 transcriptional activation domain, that interacts with bait Ob, ObR, or AR gene product will reconstitute an active GAL4 protein and thereby drive expression of the HIS3 gene. Colonies which express HIS3 can be detected by their growth on petri dishes containing semi-solid agar based media lacking histidine. The cDNA can then be purified from these strains, and used to produce and isolate the bait Ob, ObR, or AR gene-interacting protein using techniques routinely practiced in the art.

### 5.3.3. Assays for Compounds that Interfere with Ob and ObR or Catecholamine and AR Macromolecule Interactions

The macromolecules that interact with Ob or ObR or catecholamines and AR are referred to, for purposes of this discussion, as "binding partners". These binding partners are likely to be involved in the ObR signal transduction pathway or adrenergic signal transduction pathway, and therefore, in the role of Ob or ObR or catecholamines or AR in regulation of bone disorders. Therefore, it is desirable to identify compounds that interfere with or disrupt the interaction of such binding partners with Ob or catecholamines which may be useful in regulating the activity of the ObR or AR, respectively, and control bone disorders associated with ObR or adrenergic activity.

The basic principle of the assay systems used to identify compounds that interfere with the interaction between Ob or ObR, catecholamines or AR, and their binding partner or partners involves preparing a reaction mixture containing Ob, ObR, catecholamine, or AR protein, polypeptide, peptide or fusion protein as described above, and the binding partner under conditions and for a time sufficient to allow the two to interact and bind, thus forming a complex. In order to test a compound for inhibitory activity, the reaction mixture is prepared in the presence and absence of the test compound. The test compound may be initially included in the reaction mixture, or may be added at a time subsequent to the addition of the Ob or ObR moiety, catecholamine or AR moiety, and its respective binding partner. Control reaction mixtures are incubated without the test compound or with a placebo. The formation of any complexes between the Ob or ObR moiety, catecholamine and AR moiety, and the respective binding partner is then detected. The formation of a complex in the control reaction, but not in the reaction mixture containing the test compound, indicates that the compound interferes with the interaction of Ob or ObR, catecholamine or AR, and the respective interactive binding partner. Additionally, complex formation within reaction mixtures containing the test compound and normal Ob or ObR protein (or normal catecholamine or AR protein) may also be compared to complex formation within reaction mixtures containing the test compound and a mutant Ob or ObR (or mutant catecholamine or AR). This comparison may be important in those cases wherein it is desirable to identify compounds that disrupt interactions of mutant but not normal Ob or ObRs (or catecholamine or ARs).

The assay for compounds that interfere with the interaction of Ob or ObR, catecholamine or AR, and binding partners can be conducted in a heterogeneous or homogeneous format. Heterogeneous assays involve anchoring either the Ob or ObR moiety, catecholamine or AR moiety, product or the binding partner onto a solid phase and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction by competition can be identified by conducting the reaction in the presence of the test substance; *i.e.,* by adding the test substance to the reaction mixture prior to or simultaneously with the Ob or ObR moiety, catecholamine or AR moiety, and interactive binding partner. Alternatively, test compounds that disrupt preformed complexes, e.g. compounds with higher binding constants that displace one of the components from the complex, can be tested by adding the test compound to the reaction mixture after complexes have been formed. The various formats are described briefly below.

In a heterogeneous assay system, either the Ob or ObR moiety, catecholamine or AR moiety, or the interactive binding partner, is anchored onto a solid surface, while the non-anchored species is labeled, either directly or indirectly. In practice, microtiter plates are conveniently utilized. The anchored species may be immobilized by non-covalent or covalent attachments. Non-covalent attachment may be accomplished simply by coating the solid surface with a solution of the Ob, ObR, or AR gene product or binding partner and drying. Alternatively, an immobilized antibody specific for the species to be anchored may be used to anchor the species to the solid surface. The surfaces may be prepared in advance and stored.

In order to conduct the assay, the partner of the immobilized species is exposed to the coated surface with or without the test compound. After the reaction is complete, unreacted components are removed (e.g., by washing) and any complexes formed will remain immobilized on the solid surface. The detection of complexes anchored on the solid surface can be accomplished in a number of ways. Where the non-immobilized species is pre-labeled, the detection of label immobilized on the surface indicates that complexes were formed. Where the non-immobilized species is not pre-labeled, an indirect label can be used to detect complexes anchored on the surface; e.g., using a labeled antibody specific for the initially non-immobilized species (the antibody, in turn, may be directly labeled or indirectly labeled with a labeled anti-Ig antibody). Depending upon the order of addition of reaction components, test compounds which inhibit complex formation or which disrupt preformed complexes can be detected.

Alternatively, the reaction can be conducted in a liquid phase in the presence or absence of the test compound, the reaction products separated from unreacted components, and complexes detected; *e.g.,* using an immobilized antibody specific for one of the binding components to anchor any complexes formed in solution, and a labeled antibody specific for the other partner to detect anchored complexes. Again, depending upon the order of addition of reactants to the liquid phase, test compounds which inhibit complex or which disrupt preformed complexes can be identified.

In an alternate embodiment of the invention, a homogeneous assay can be used. In this approach, a preformed complex of the Ob or ObR moiety, catecholamine or AR moiety, and the interactive binding partner is prepared in which either Ob or ObR or its binding partners is labeled, but the signal generated by the label is quenched due to formation of the complex (see, *e.g*., U.S. Pat. No. 4,109,496 which utilizes this approach for immunoassays). The addition of a test substance that competes with and displaces one of the species from the preformed complex will result in the generation of a signal above background. In this way, test substances which disrupt Ob or ObR/intracellular binding partner interaction and or catecholamine or AR/intracellular binding partner interaction can be identified.

In a particular embodiment, an Ob, ObR, or AR fusion can be prepared for immobilization. For example, the Ob, ObR, or AR or a peptide fragment, e.g., corresponding to the ObR CD, can be fused to a glutathione-S-transferase (GST) gene using a fusion vector, such as pGEX-5X-1, in such a manner that its binding activity is maintained in the resulting fusion protein. The interactive binding partner can be purified and used to raise a monoclonal antibody, using methods routinely practiced in the art. This antibody can be labeled with the radioactive isotope ¹²⁵I, for example, by methods routinely practiced in the art. In a heterogeneous assay, e.g., the GST-ObR fusion protein (or GST-AR fusion protein) can be anchored to glutathione-agarose beads. The interactive binding partner can then be added in the presence or absence of the test compound in a manner that allows interaction and binding to occur. At the end of the reaction period, unbound material can be washed away, and the labeled monoclonal antibody can be added to the system and allowed to bind to the complexed components. The interaction between the Ob or ObR gene product (or the catecholamine or AR gene product) and the interactive binding partner can be detected by measuring the amount of radioactivity that remains associated with the glutathione-agarose beads. A successful inhibition of the interaction by the test compound will result in a decrease in measured radioactivity.

Alternatively, the GST-Ob/ObR fusion protein and the interactive binding partner can be mixed together in liquid in the absence of the solid glutathione-agarose beads. The test compound can be added either during or after the species are allowed to interact. This mixture can then be added to the glutathione-agarose beads and unbound material is washed away. Again the extent of inhibition of the Ob or ObR/binding partner interaction can be detected by adding the labeled antibody and measuring the radioactivity associated with the beads.

In another embodiment of the invention, these same techniques can be employed using peptide fragments that correspond to the binding domains of Ob, ObR, or AR and/or the interactive or binding partner (in cases where the binding partner is a protein), in place of one or both of the full length proteins. Any number of methods routinely practiced in the art can be used to identify and isolate the binding sites. These methods include, but are not limited to, mutagenesis of the gene encoding one of the proteins and screening for disruption of binding in a co-immunoprecipitation assay. Compensating mutations in the gene encoding the second species in the complex can then be selected. Sequence analysis of the genes encoding the respective proteins will reveal the mutations that correspond to the region of the protein involved in interactive binding. Alternatively, one protein can be anchored to a solid surface using methods described above, and allowed to interact with and bind to its labeled binding partner, which has been treated with a proteolytic enzyme, such as trypsin. After washing, a short, labeled peptide comprising the binding domain may remain associated with the solid material, which can be isolated and identified by amino acid sequencing. Also, once the gene coding for the intracellular binding partner is obtained, short gene segments can be engineered to express peptide fragments of the protein, which can then be tested for binding activity and purified or synthesized.

For example, and not by way of limitation, an Ob, ObR, or AR gene product can be anchored to a solid material as described, above, by making a GST-Ob, -ObR, or -AR fusion protein and allowing it to bind to glutathione agarose beads. The interactive binding partner can be labeled with a radioactive isotope, such as ³⁵S, and cleaved with a proteolytic enzyme such as trypsin. Cleavage products can then be added to the anchored GST-Ob, -ObR, or -AR fusion protein and allowed to bind. After washing away unbound peptides, labeled bound material, representing the intracellular binding partner binding domain, can be eluted, purified, and analyzed for amino acid sequence by well-known methods. Peptides so identified can be produced synthetically or fused to appropriate facilitative proteins using recombinant DNA technology.

### 5.3.4. Assays for Identification of Compounds that Ameliorate Bone Disease

Compounds, including, but not limited to, compounds identified via assay techniques such as those described, above, in Sections 5.3.1 through 5.3.3, can be tested for the ability to treat bone disease and ameliorate bone disease symptoms. The assays described above can identify compounds which affect Ob, ObR, or AR activity (e.g., leptin receptor or adrenergic agonists or antagonists), and compounds that bind to the natural ligand of the ObR or AR and neutralize ligand activity; or compounds that affect Ob, ObR, or AR gene activity (by affecting Ob, ObR, or AR gene expression, including molecules, e.g., proteins or small organic molecules, that affect or interfere with splicing events so that expression of the full length or the truncated form of the Ob, ObR, or AR can be modulated). However, it should be noted that the assays described can also identify compounds that modulate Ob, ObR, or adrenergic signal transduction (e.g., compounds which affect downstream signaling events, such as inhibitors or enhancers of tyrosine kinase or phosphatase activities which participate in transducing the signal activated by Ob binding to the ObR). Alternatively, the assays described can also identify compounds which modulate the entry of Ob through the blood-brain barrier. The identification and use of such compounds which affect another step in the Ob or ObR signal transduction pathway in which the Ob or ObR gene and/or gene product is involved and, by affecting this same pathway may modulate the effect of Ob or ObR on the development of bone disorders are within the scope of the invention. Similarly, the identification and use of such compounds which affect another step in the adrenergic signal transduction pathway in which the AR gene and/or gene product is involved and, by affecting this same pathway may modulate the effect of catecholamines or AR on the development of bone disorders are within the scope of the invention. Such compounds can be used as part of a therapeutic method for the treatment of bone disease.

Cell-based systems can be used to identify compounds which may act to ameliorate bone disease. Such cell systems can include, for example, recombinant or non-recombinant cells, such as cell lines, which express the Ob, ObR, or AR gene, e.g., NIH3T3L1 cell lines. Further, for example, for ObR, choroid plexus cells, hypothalamus cells, or cell lines derived from choroid plexus or hypothalamus can be used. In addition, expression host cells (e.g., COS cells, CHO cells, fibroblasts) genetically engineered to express a functional Ob or ObR and to respond to activation by the natural Ob ligand, e.g., as measured by a chemical or phenotypic change, induction of another host cell gene, change in ion flux (*e.g.,* Ca⁺⁺), tyrosine phosphorylation of host cell proteins, etc., can be used as an end point in the assay.

In utilizing such cell systems, cells may be exposed to a compound suspected of exhibiting an ability to ameliorate bone disorders, at a sufficient concentration and for a time sufficient to elicit such an amelioration of bone disorders in the exposed cells. After exposure, the cells can be assayed to measure alterations in the expression of the Ob or ObR gene, *e.g.,* by assaying cell lysates for Ob, ObR, or AR mRNA transcripts (e.g., by Northern analysis) or for Ob, ObR, or AR protein expressed in the cell; compounds which regulate or modulate expression of the Ob, ObR, or AR gene are good candidates as therapeutics. Alternatively, the cells are examined to determine whether one or more bone disorder-like cellular phenotypes has been altered to resemble a more normal or more wild type, non-bone disorder phenotype, or a phenotype more likely to produce a lower incidence or severity of disorder symptoms. Still further, the expression and/or activity of components of the signal transduction pathway of which ObR or AR is a part, or the activity of the ObR signal transduction pathway and/or adrenergic signal transduction pathway itself can be assayed.

For example, after exposure, the cell lysates can be assayed for the presence of tyrosine phosphorylation of host cell proteins, as compared to lysates derived from unexposed control cells. The ability of a test compound to inhibit tyrosine phosphorylation of host cell proteins in these assay systems indicates that the test compound inhibits signal transduction initiated by ObR activation. The cell lysates can be readily assayed using a Western blot format; *i.e.*, the host cell proteins are resolved by gel electrophoresis, transferred and probed using a anti-phosphotyrosine detection antibody (e.g., an anti-phosphotyrosine antibody labeled with a signal generating compound, such as radiolabel, fluor, enzyme, etc.) (see, e.g., Glenney et al., 1988, J. Immunol. Methods 109:277-285; Frackelton et al., 1983, Mol. Cell. Biol. 3:1343-1352). Alternatively, an ELISA format could be used in which a particular host cell protein involved in the ObR signal transduction pathway is immobilized using an anchoring antibody specific for the target host cell protein, and the presence or absence of phosphotyrosine on the immobilized host cell protein is detected using a labeled anti-phosphotyrosine antibody (see, e.g., King et al., 1993, Life Sciences 53:1465-1472). In yet another approach, ion flux, such as calcium ion flux, can be measured as an end point for ObR stimulated signal transduction.

In addition, animal-based bone disorder systems, which may include, for example, ob, db and ob/db mice, may be used to identify compounds capable of ameliorating bone disorder-like symptoms. Such animal models may be used as test substrates for the identification of drugs, pharmaceuticals, therapies and interventions which may be effective in treating such disorders. For example, animal models may be exposed to a compound suspected of exhibiting an ability to ameliorate bone disorder symptoms, at a sufficient concentration and for a time sufficient to elicit such an amelioration of bone disorder symptoms in the exposed animals. The response of the animals to the exposure may be monitored by assessing the reversal of disorders associated with bone disorders such as osteoporosis. With regard to intervention, any treatments which reverse any aspect of bone disorder-like symptoms should be considered as candidates for human bone disorder therapeutic intervention. Dosages of test agents may be determined by deriving dose-response curves, as discussed below.

### 5.4. Compounds that Modulate Ob or ObR Expression or Activity

Compounds that interact with (e.g., bind to) Ob or ObR (including, but not limited to, the ECD or CD of ObR), compounds that interact with (e.g., bind to) intracellular proteins that interact with Ob or ObR (including, but not limited to, the TM and CD of ObR), compounds that interfere with the interaction of Ob or ObR with transmembrane or intracellular proteins involved in ObR-mediated signal transduction, and compounds which modulate the activity of Ob or ObR gene expression or modulate the level of Ob or ObR are capable of modulating levels of bone mass. More specifically, compounds which decrease the levels of Ob or ObR, inhibit the transport of Ob across the blood-brain barrier or inhibit binding of Ob to the ObR would cause an increase in bone mass.

Examples of such compounds are leptin and leptin receptor agonists and antagonists. Leptin receptor antagonist, as used herein, refers to a factor which neutralizes or impedes or otherwise reduces the action or effect of a leptin receptor. Such antagonists can include compounds that bind leptin or that bind leptin receptor. Such antagonists can also include compounds that neutralize, impede or otherwise reduce leptin receptor output, that is, intracellular steps in the leptin signaling pathway following binding of leptin to the leptin receptor, *i.e*., downstream events that affect leptin/leptin receptor signaling, that do not occur at the receptor/ligand interaction level. Leptin receptor antagonists may include, but are not limited to proteins, antibodies, small organic molecules or carbohydrates, such as, for example, acetylphenol compounds, antibodies which specifically bind leptin, antibodies which specifically bind leptin receptor, and compounds that comprise soluble leptin receptor polypeptide sequences.

For example, leptin antagonists also include agents, or drugs, which decrease, inhibit, block, abrogate or interfere with binding of leptin to its receptors or extracellular domains thereof; agents which decrease, inhibit, block, abrogate or interfere with leptin production or activation; agents which are antagonists of signals that drive leptin production or synthesis, and agents which prohibit leptin from reaching its receptor, e.g., prohibit leptin from crossing the blood-brain barrier. Such an agent can be any organic molecule that inhibits or prevents the interaction of leptin with its receptor, or leptin production (see, e.g., U.S. Patent No. 5,866,547). Leptin antagonists include, but are not limited to, anti-leptin antibodies, receptor molecules and derivatives which bind specifically to leptin and prevent leptin from binding to its cognate receptor.

A leptin receptor agonist, as used herein, refers to a factor which activates, induces or otherwise increases the action or effect of a leptin receptor. Such agonists can include compounds that bind leptin or that bind leptin receptor. Such agonists can also include compounds that activate, induce or otherwise increase leptin receptor output, that is, intracellular steps in the leptin signaling pathway following binding of leptin to the leptin receptor, *i.e*., downstream events that affect leptin/leptin receptor signaling, that do not occur at the receptor/ligand interaction level. Leptin receptor agonists may include, but are not limited to proteins, antibodies, small organic molecules or carbohydrates, such as, for example, leptin, leptin analogs, and antibodies which specifically bind and activate leptin.

Additional Ob binding proteins include, but are not limited to, inter-alpha-trypsin inhibitor heavy chain-related protein (IHRP); alpha 2-macroglobulin; and OB-BP1 which specifically bind Ob and is thus capable of preventing Ob from binding to the ObR. The specific Ob binding protein further enables modulation of free Ob levels, immobilization and assay of bound/free leptin (see, *e.g*., U.S. Patent No. 5,919,902; Birkenmeier et al., 1998, Eur. J. Endocrin., 139:224-230; and Patel et al., 1999, J. Biol. Chem., 32:22729-22738. Additional Ob binding proteins, such as apolipoproteins, are disclosed in U.S. Patent No. 5,830,450.

Examples of ObR antagonists are acetylphenols, which are known to be useful as antiobesity and antidiabetic compounds. Since acetylphenols are antagonists of the ObR, they prevent binding of Ob to the ObR. Thus, in view of the teachings of the present invention, the compounds would effectively cause an increase in bone mass. For specific structures of acetylphenols which can be used as ObR antagonists, see U.S. Patent No. 5,859,051.

Additional antagonists and agonists of the ObR, and other compounds that modulate ObR gene expression or ObR activity that can be used for diagnosis, drug screening, clinical trial monitoring, and/or the treatment of bone disorders can be found in U.S. Patent Nos. 5,972,621; 5874,535; and 5,912,123.

### 5.5. Compounds that Modulate Catecholamine or Adrenergic Receptor Expression or Activity

Compounds that interact with (e.g., bind to) catecholamines or adrenergic receptors (including, but not limited to, the ECD or CD of adrenergic receptors), compounds that interact with (e.g., bind to) intracellular proteins that interact with catecholamines or adrenergic receptors (including, but not limited to, the TM and CD of adrenergic receptors), compounds that interfere with the interaction of catecholamines or AR with transmembrane or intracellular proteins involved in adrenergic receptor-mediated signal transduction, and compounds which modulate the activity of catecholamines or adrenergic receptor gene expression or modulate the level of catecholamines or adrenergic receptors are capable of modulating levels of bone mass. More specifically, compounds which decrease the levels of catecholamines or adrenergic receptors or inhibit binding of catecholamines to the adrenergic receptor would cause an increase in bone mass.

Examples of such compounds are catecholamine and adrenergic receptor agonists and antagonists. An adrenergic antagonist, as used herein, refers to a factor which neutralizes or impedes or otherwise reduces the action or effect of an adrenergic receptor. Such antagonists can include compounds that bind catecholamines or that bind adrenergic receptors. Such antagonists can also include compounds that neutralize, impede or otherwise reduce catecholamine receptor output, that is, intracellular steps in the adrenergic signaling pathway following binding of catecholamines to the adrenergic receptor, *i.e*., downstream events that affect adrenergic signaling, that do not occur at the receptor/ligand interaction level. Adrenergic antagonists may include, but are not limited to proteins, antibodies, small organic molecules or carbohydrates, such as, for example, antibodies which specifically bind adrenergic receptors, and compounds that comprise soluble adrenergic receptor polypeptide sequences.

Catecholamines are amine-containing derivatives of catechol, 1,2-dihydroxybenzene, such as, but not limited to, norepinephrine and its methyl derivative epinephrine. Norepinephrine is produced by adrenergic nerve endings, while epinephrine is produced by the adrenal medulla.

As described in the background section of U.S. Patent No. 6,313,172, which is hereby incorporated by reference in its entirety, human adrenergic receptors are integral membrane proteins which have been classified into two broad classes, the alpha and the beta adrenergic receptors. Both types mediate the action of the peripheral sympathetic nervous system upon binding of catecholamines. The binding affinity of adrenergic receptors for these compounds forms one basis of the classification: alpha receptors tend to bind norepinephrine more strongly than epinephrine and much more strongly than the synthetic compound isoproterenol. The preferred binding affinity of these hormones is reversed for the beta receptors. In many tissues, the functional responses, such as smooth muscle contraction, induced by a receptor activation are opposed to responses induced by beta receptor binding.

Subsequently, the functional distinction between alpha and beta receptors was further highlighted and refined by the pharmacological characterization of these receptors from various animal and tissue sources. As a result, alpha and beta adrenergic receptors were further subdivided into α₁, and α₂ and β₁, β₂, and β₃ subtypes.

For example, β adrenergic antagonists include, but are not limited to, esmolol, metoprolol, atenolol, or acebutolol (see, e.g., U.S. Patent No. 6,303,573, which is hereby incorporated by reference in its entirety). Other agents that are useful as adrenergic antagonists include, but are not limited to, (i) sympathetic blocking agents which bind to alpha adrenergic receptors and (ii) agents which deplete transmitters. The blocking agents may be reversible alpha receptor antagonists such as phentolamine, tolazoline, prazosin, terzosin, doxazosin, trimazosin, and indoramin or irreversible alpha receptor antagonists such as phenoxybenzamine or dibenzamine. Agents which are depletors of transmitters include guanethidine, guanadrel, reserpine, or metyrosine (see, e.g., U.S. Patent No. 6,009,875, which is hereby incorporated by reference in its entirety).

An adrenergic agonist, as used herein, refers to a factor which activates, induces or otherwise increases the action or effect of an adrenergic receptor. Such agonists can include compounds that bind catecholamines or that bind adrenergic receptors. Such agonists can also include compounds that activate, induce or otherwise increase adrenergic receptor output, that is, intracellular steps in the adrenergic signaling pathway following binding of catecholamines to the adrenergic receptor, *i.e.,* downstream events that affect adrenergic signaling, that do not occur at the receptor/ligand interaction level. Adrenergic agonists may include, but are not limited to proteins, antibodies, small organic molecules or carbohydrates, such as, for example, catecholamines and catecholamine analogs. Examples of β adrenergic agonists include, but are not limited to, isoproterenol, dopamine, and dobutamine (see, *e.g.,* U.S. Patent No. 5,713,367, which is hereby incorporated by reference in its entirety).

### 5.6. Compounds that Modulate NPY or NPY-R Expression or Activity

The present invention also provides for the use of NPY and NPY receptor agonists and antagonists, either alone or in combination with other modulators described herein, for modulating leptin effects and/or sympathetic tone on bone.

Compounds that interact with (e.g., bind to) NPY or NPY-R (including, but not limited to, the ECD or CD of NPY-R), compounds that interact with (*e*.*g*., bind to) intracellular proteins that interact with NPY or NPY-R (including, but not limited to, the TM and CD of NPY-R), compounds that interfere with the interaction of NPY or NPY-R with transmembrane or intracellular proteins involved in NPY-R-mediated signal transduction, and compounds which modulate the activity of NPY or NPY-R gene expression or modulate the level of NPY or NPY-R are capable of modulating levels of bone mass. More specifically, compounds which decrease the levels of NPY or NPY-R, inhibit the transport of NPY across the blood-brain barrier or inhibit binding of NPY to the NPY-R would cause an increase in bone mass.

Examples of such compounds are NPY and NPY receptor agonists and antagonists. An NPY receptor antagonist, as used herein, refers to a factor which neutralizes or impedes or otherwise reduces the action or effect of a NPY receptor. Such antagonists can include compounds that bind NPY or that bind NPY receptor. Such antagonists can also include compounds that neutralize, impede or otherwise reduce NPY receptor output, that is, intracellular steps in the NPY signaling pathway following binding of NPY to the NPY receptor, *i.e*., downstream events that affect NPY/NPY receptor signaling, that do not occur at the receptor/ligand interaction level. NPY receptor antagonists may include, but are not limited to proteins, antibodies, small organic molecules or carbohydrates, such as, for example, acetylphenol compounds, antibodies which specifically bind NPY, antibodies which specifically bind NPY receptor, and compounds that comprise soluble NPY receptor polypeptide sequences.

For example, NPY antagonists also include agents, or drugs, which decrease, inhibit, block, abrogate or interfere with binding of NPY to its receptors or extracellular domains thereof; agents which decrease, inhibit, block, abrogate or interfere with NPY production or activation; agents which are antagonists of signals that drive NPY production or synthesis, and agents which prohibit NPY from reaching its receptor, e.g., prohibit NPY from crossing the blood-brain barrier. Such an agent can be any organic molecule that inhibits or prevents the interaction of NPY with its receptor, or NPY production.

An NPY receptor agonist, as used herein, refers to a factor which activates, induces or otherwise increases the action or effect of a NPY receptor. Such agonists can include compounds that bind NPY or that bind NPY receptor. Additional NPY agonists and analogs include those described in U.S. Patent No. 5,328,899. Such agonists can also include compounds that activate, induce or otherwise increase NPY receptor output, that is, intracellular steps in the NPY signaling pathway following binding of NPY to the NPY receptor, *i.e.*, downstream events that affect NPY/NPY receptor signaling, that do not occur at the receptor/ligand interaction level. NPY receptor agonists may include, but are not limited to proteins, antibodies, small organic molecules or carbohydrates, such as, for example, NPY, NPY analogs, and antibodies which specifically bind and activate NPY.

Numerous NPY antagonists have been described. For example, U.S. Patent No. 5,972,888 describes various compounds which act as NPY antagonists, including, but not limited to, derivatives of naphthalenes, benzofuran, benzothiophenes and indoles; raloxifene; 3-(4-methoxyphenyl)-4-[4-(2-pyrrolidin-1-ylethoxy)benzoyl-1,2-dihydronaphthalene, citrate salt; 3-phenyl-4-[4-(2-pyrrolidin-1-ylethoxy)benzoyl]-7-methoxy-1,2-dihydronaphthalene; 3-phenyl-4-[4-(2-pyrrolidin-1-ylethoxy)benzoyl]-1,2-dihydronaphthalene; 1-[4-(2-pyrrolidin-1-ylethoxy)benzoyl]-2-phenylnaphthalene, citrate salt; 3-(4-methoxyphenyl)-4-[4-[2-(piperidin-1-yl)ethoxy]benzoyl]-1,2-dihydronaphthalene, citrate salt; 3-(4-methoxyphenyl)-4-[4-(2-dimethylaminoethoxy)benzoyl]-1,2-dihydronaphthalene, citrate salt; 3-(4-hydroxyphenyl)-4-(4-[2-(pyrrolidin-1-yl)ethoxy]benzoyl]-1,2-dihydronaphthalene, mesylate salt; 3-(4-methoxyphenyl)-4-[4-[2-(hexamethyleneimin-1-yl)benzoyl]-1,2-dihydronaphthalene, mesylate salt; 3-(4-methoxyphenyl)-4-[4-[2-(piperidin-1-yl)ethoxy]benzoyl]-1,2-dihydronaphthalene, mesylate salt; 3-(4-methoxyphenyl)-4-(4-diethylaminoethoxybenzoyl)-1,2-dihydronaphthalene, mesylate salt; 3-(4-methoxyphenyl)-4-(4-diisopropylaminoethoxybenzoyl)-1,2-dihydronaphthalene, mesylate salt; 3-hydroxy-4-[4-[2-(pyrrolidin-1-yl)ethoxy]benzoyl]-1,2-dihydronaphthalene, sodium salt; 2-(4-methoxyphenyl)-1-[4-[2-(pyrrolidin-1-yl)ethoxy]benzoyl]naphthalene, mesylate salt; 3-(4-methoxyphenyl)-4-[4-[2-(piperidin-1-yl)ethoxy]benzoyl]-7-methoxy-1,2-d ihydronaphthalene, mesylate salt; 3-(4-methoxyphenyl)-4-[4-(2-dimethylaminoethoxy)benzoyl]-1,2-dihydronaphthalene, 2-hydroxy-1,2,3-propanetricarboxylic acid salt; 3-(4-methoxyphenyl)-4-[4-[2-(N-methyl-1-pyrrolidinium)ethoxy]benzoyl]-1,2-d ihydronaphthalene, iodide salt; and 3-(4-mcthoxyphenyl)-4-[4-[2-(pyrrolidin-1-yl)ethoxy]benzoyl]-1,2-dihydronaphthalene, mesylate salt.

Similarly, numerous NPY-R antagonists have been identified. Examples of such antagonists include, but are not limited to, α-alkoxy and α-thioalkoxyamide compositions (see, *e.g.,* U.S. Patent No. 5,939,462); dihydropyridine based compounds (see, *e.g.,* U.S. Patent Nos. 5,554,621, 6,001,836, 5,668,151 and 5,635,503); substituted benzylamine derivatives see, e.g., U.S. Patent Nos. 5,985,873, 5,962,455 and 5,900,415); dihydropyrimidone derivatives (see, *e.g.*, U.S. Patent No. 5,889,016); naphthimidazolyl derivatives (see, *e.g.,* U.S. Patent No. 5,776,931); dimesylate salts (see, *e.g*., U.S. Patent No. 5,914,329); and substituted benzofurans, benzothiophenes or indoles (see, e.g., U.S. Patent No. 5,663,192). Additional NPY-R antagonists are disclosed in U.S. Patent Nos. 5,567,714, 5,504,094, 5,670,482, 5,989,920, and 5,827,853, 5,985,616.

### 5.7. Compounds that Modulate CNTF or CNTF Receptor Expression or Activity

The present invention also provides for the use of CNTF and CNTF receptor agonists and antagonists, either alone or in combination with other modulators described herein, for modulating leptin effects and/or sympathetic tone on bone.

Compounds that interact with (*e.g*., bind to) CNTF or CNTF receptor (including, but not limited to, the ECD or CD of a CNTF receptor polypeptide), compounds that interact with (*e.g*., bind to) intracellular proteins that interact with CNTF or CNTF receptor (including, but not limited to, the TM and CD of CNTF receptor polypeptide), compounds that interfere with the interaction of CNTF or CNTF receptor with transmembrane or intracellular proteins involved in CNTF receptor-mediated signal transduction, and compounds which modulate the activity of CNTF or CNTF receptor gene expression or modulate the level of CNTF or CNTF receptor are capable of modulating levels of bone mass. More specifically, compounds which decrease the levels of CNTF or CNTF receptor or inhibit binding of CNTF to the CNTF receptor would cause an increase in bone mass.

Examples of such compounds are CNTF and CNTF receptor agonists and antagonists. CNTF receptor antagonist, as used herein, refers to a factor which neutralizes or impedes or otherwise reduces the action or effect of a CNTF receptor. Such antagonists can include compounds that bind CNTF or that bind CNTF receptor. Such antagonists can also include compounds that neutralize, impede or otherwise reduce CNTF receptor output, that is, intracellular steps in the CNTF signaling pathway following binding of CNTF to the CNTF receptor, *i.e.,* downstream events that affect CNTF/CNTF receptor signaling, that do not occur at the receptor/ligand interaction level. CNTF receptor antagonists may include, but are not limited to proteins, antibodies, small organic molecules or carbohydrates, such as, for example, mutant CNTF molecules including F152S mutant CNTF or K155A mutant CNTF, antibodies which specifically bind CNTF, antibodies which specifically bind CNTF receptor, and compounds that comprise soluble CNTF receptor polypeptide sequences.

For example, CNTF receptor antagonists also include agents, or drugs, which decrease, inhibit, block, abrogate or interfere with binding of CNTF to its receptors or extracellular domains thereof; agents which decrease, inhibit, block, abrogate or interfere with CNTF production or activation; agents which are antagonists of signals that drive CNTF production or synthesis, and agents which prohibit CNTF from reaching its receptor. Such an agent can be any organic molecule that inhibits or prevents the interaction of CNTF with its receptor, or CNTF production.

CNTF receptor antagonists also include mutant CNTF molecules that interfere with CNTF receptor function (see, *e.g*., U.S. Patent No. 5,846,935). For example, mutation of the lysine residue at position 155 of the primary sequence of CNTF yielded one CNTF antagonist (see, *e.g.*, Inoue et al., 1997, J. Neurochem. 69:95-101; DiMarco et al., 1996, Proc. Natl. Acad. Sci. USA 93:9247-52). U.S. Patent No. 5,723,120 claims CNTF molecules with mutations in the region of amino acids 154-163. CNTF receptor antagonists also include anti-CNTF antibodies, receptor molecules and derivatives which bind specifically to CNTF and prevent CNTF from binding to its cognate receptor.

CNTF receptor antagonists include antagonists of IL-6 receptor function that also inhibit CNTF receptor function, such as gp130 inhibitors based on the structure of GM-CSF and/or gp130 inhibitors based on the structure or IL-6 (see, *e.g.,* U.S. Patent Nos. 5,914,106; 5,891,998; 5,849,283; 5,789,552; 5,591,827; and 5,723,120). CNTF receptor antagonists include antisense oligonucleotides complementary to polynucleotides coding for CNTF receptor polypeptide (see, *e.g*., U.S. Patent Nos. 5,747,470; 5,674,995; and 5,747,470). Other antagonists include those derived from soluble domains or extracellular domains of CNTF receptor polypeptides (see U.S. Patent Nos. 5,844,099 U.S. Patent No. 5,470,952). Finally, the antagonists include antibodies to CNTF receptor polypeptides (see, *e.g*., U.S. Patent Nos. 5,892,003; 5,866,689; and 5,717,073).

CNTF receptor antagonists also include compounds that inhibit the downstream signaling cascades of CNTF activation influence the activity of CNTF. For instance, rapamycin, an inhibitor of the serine kinase mTOR, also inhibits CNTF-induced phosphorylation of STAT3 (see Yokogami et al., 2000, Current Biology 10:47-50). SOCS-3 is a negative regulator of CNTF signal transduction. The serine/threonine kinase inhibitor H7 inhibits CyRE mediated transcription induced by CNTF (see Symes et al., 1997, J. Biol. Chem. 272:9648-9654). The proteasome inhibitor MG 132 and phorbol esters modulate the levels of phosphorylated STAT3 (see, *e.g*., Malek et al., 1999, Cytokine 11:192-199).

CNTF receptor antagonists also include molecules that interfere with and/or prevent the formation of a CNTF/CNTF receptor complex. For example, molecules that bind CNTF or molecules that bind to a component of the CNTF receptor can also prevent the formation of the CNTF/CNTF receptor complex. Examples of such molecules include, but are not limited to, antibodies that recognize one or more CNTF receptor subunits and molecules such as those describe in U.S. Patent Nos. 5,717,073 and 5,866,689, and other molecules that prevent CNTF/CNTF receptor complex formation known to those of skill in the art.

A CNTF receptor agonist, as used herein, refers to a factor which activates, induces or otherwise increases the action or effect of a CNTF receptor. Such agonists can include compounds that bind CNTF or that bind CNTF receptor. Such agonists can also include compounds that activate, induce or otherwise increase CNTF receptor output, that is, intracellular steps in the CNTF signaling pathway following binding of CNTF to the CNTF receptor, *i.e.,* downstream events that affect CNTF/CNTF receptor signaling, that do not occur at the receptor/ligand interaction level. CNTF receptor agonists may include, but are not limited to proteins, antibodies, small organic molecules or carbohydrates, such as, for example, CNTF, CNTF analogs, and antibodies which specifically bind and activate CNTF. In addition, CNTF agonists include mutant CNTF molecules with increased CNTF activity such as DH-CNTF, a superagonist variant of CNTF with mutations of Ser166 to Asp and Gln167 to His (see Saggio et al., 1995, EMBO J. 14:3045-3054). CNTF receptor agonists also include compounds that influence the downstream signaling events of CNTF function. For instance, inhibitors of SOCS-3 might enhance CNTF signal transduction (see Bjorbaek, 1999, Endocrinology 140:2035-2043).

Additional CNTF binding proteins include, but are not limited to, such compounds as an antibody which specifically binds CNTF, a soluble CNTFRα (CNTFRα lacking its glycosyl-phosphatidyl-inositol anchor), and other molecules that bind CNTF such as those described in U.S. Patent No. 5,470,952 and others known to those of skill in the art which specifically bind CNTF and are thus capable of preventing CNTF from binding to the CNTF receptor. The specific CNTF binding protein further enables modulation of free CNTF levels, immobilization and assay of bound/free CNTF.

Additional antagonists and agonists of the CNTF receptor, and other compounds that modulate CNTF receptor gene expression or CNTF receptor activity that can be used for diagnosis, drug screening, clinical trial monitoring, and/or the treatment of bone disorders can be found in U.S. Patent No. 5,846,935; Inoue et al., 1997, J. Neurochem. 69:95-101; DiMarco et al., 1996, Proc. Natl. Acad. Sci. USA 93:9247-52; U.S. Patent No. 5,723,120; and Saggio, 1995, EMBO J 14:3045-54.

### 5.8. Methods for the Treatment or Prevention of Bone Disease

Bone diseases which can be treated and/or prevented in accordance with the present invention include bone diseases characterized by a decreased bone mass relative to that of corresponding non-diseased bone, including, but not limited to osteoporosis, osteopenia, Paget's disease, osteomalacia and renal osteodystrophy. Bone diseases which can be treated and/or prevented in accordance with the present invention also include bone diseases characterized by an increased bone mass relative to that of corresponding non-diseased bone, including, but not limited to osteopetrosis, osteosclerosis and osteochondrosis.

A critical parameter in diseases of low bone mass is susceptibility to fracture. Since susceptibility to fracture cannot be measure directly, measurements of bone mass or bone mineral density provides an indication of how susceptible a bone is to fracture. Although there is a correlation between low bone mass and increased susceptibility to fracture, there is sometimes discordance which can be attributed to variations in bone geometry and trabecular architecture. In general, bone mass (or bone density or bone volume) and bone geometry are used to obtain a static picture of what a bone looks like, from which the mechanical properties of the bone (e.g., strength, rigidity, and stiffness) are inferred and predictions about risk of fracture can be determined by one of skill in the art. In animal models, histomorphometry measures are favored for analyzing bone mass, geometry, and rate of formation. Rate of resorption is harder to characterize because counts of osteoclast number or surface area are not representative of osteoclast activity. The mechanical properties of bone, such as, but not limited to, strength in tension compression and bending, stiffness, and maximal load, can be directly measured. Bone mass and bone geometry can be determined by methods such as, but not limited to, single and dual photon absorptiometry (SPA and DPA), single and dual X-ray absorptiometry (SXA and DXA), quantitative computed tomography (QCT), ultrasound (US) and magnetic resonance imaging (MRI) (see, e.g., Guglielmi et al., 1995, Eur Radiol. 5(2):129-39).

The invention also encompasses bone diseases not related to bone mass. For example, the present invention includes, but is not limited to, diseases of altered mineral content, abnormal matrix compounds (e.g., collagen), or abnormal local outgrowths.

An object of the present invention is the treatment, diagnosis and/or prevention of bone disease through manipulation of sympathetic nervous system pathways. In accordance with one aspect of the present invention, there is a method for treating and preventing one or more symptoms of osteoporosis comprising the administration of β adrenergic antagonists. A specific embodiment of this aspect includes, but is not limited to, the further administration of a leptin antagonist in combination with β adrenergic antagonists. In another embodiment, there is a method for treating and preventing one or more symptoms of osteopetrosis or osteosclerosis comprising the administration of β adrenergic agonists. A further embodiment includes, but is not limited to, the further administration of a leptin agonist in combination with β adrenergic agonists.

Another object of the present invention relates to a method of modulating leptin effects on bone comprising the administration of a therapeutically effective amount of a pharmaceutical composition that alters the sympathetic tone. Another embodiment of the present invention relates to a method of modulating bone mass comprising the administration of a therapeutically effective amount of a pharmaceutical composition that alters sympathetic tone so that bone mass is modulated. Specific embodiments of the pharmaceutical composition include, but are not limited to, leptin antagonists, leptin agonists, sympathetic nervous system antagonists, sympathetic nervous system agonists, and combinations thereof.

Another object of the present invention relates to methods of treating or preventing one or more symptoms of bone disease comprising the administration of a therapeutically effective amount of a pharmaceutical composition that modulates leptin effects in bone by altering sympathetic tone. Specific embodiments of the pharmaceutical composition include, but are not limited to, leptin antagonists, leptin agonists, sympathetic nervous system antagonists, sympathetic nervous system agonists, and combinations thereof.

In one aspect of the invention is a method of treating a bone disease comprising: administering to a mammal in need of said treatment a therapeutically effective amount of a compound that lowers leptin level in blood serum, wherein the bone disease is characterized by a decreased bone mass relative to that of corresponding non-diseased bone. Specific embodiments of some of these compounds and methods include, but are not limited to ones that inhibit or lower leptin synthesis or increase leptin breakdown. Among such compounds are antisense, ribozyme or triple helix sequences of a leptin-encoding polypeptide.

In accordance with another aspect of the present invention, there is a method of treating a bone disease comprising: administering to a mammal in need of said treatment a therapeutically effective amount of a compound that lowers leptin level in cerebrospinal fluid, wherein the bone disease is characterized by a decreased bone mass relative to that of corresponding non-diseased bone. Specific embodiments of some of these compounds and methods include, but are not limited to ones that inhibit or lower leptin synthesis or increase leptin breakdown, and compounds that bind leptin in blood.

Particular embodiments of the methods of the invention include, for example, a method of treating a bone disease comprising: administering to a mammal in need of said treatment a therapeutically effective amount of a compound, wherein the bone disease is characterized by a decreased bone mass relative to that of corresponding non-diseased bone, and wherein the compound is selected from the group consisting of compounds which bind leptin in blood, including, but not limited to such compounds as an antibody which specifically binds leptin, a soluble leptin receptor polypeptide, an inter-alpha-trypsin inhibitor heavy chain related protein and an alpha 2-macroglobulin protein.

In accordance with another aspect of the present invention, there is a method of treating a bone disease comprising: administering to a mammal in need of said treatment a therapeutically effective amount of a compound that lowers the level of phosphorylated Stat3 polypeptide, wherein the bone disease is characterized by a decreased bone mass relative to that of corresponding non-diseased bone. Specific embodiments of some of these compounds and methods include, but are not limited to ones that inhibit or lower leptin synthesis or increase leptin breakdown, compounds that bind leptin in blood, and leptin receptor antagonist compounds, such as acetylphenol compounds, antibodies which specifically bind leptin, antibodies which specifically bind leptin receptor, and compounds that comprise soluble leptin receptor polypeptide sequences.

In accordance with another aspect of the present invention, there is a method of treating or preventing a bone disease comprising: administering to a mammal in need of said treatment or prevention a therapeutically effective amount of a compound that inhibits the activity of dopamine β hydroxylase ("DBH"), the enzyme necessary for converting dopamine to norepinephrine. Specific embodiments of some of these compounds and methods include, but are not limited to ones that inhibit DBH enzyme activity, DBH gene expression, antibodies which specifically bind DBH, and compounds which are involved in the norephinephrine synthesis pathway.

In accordance with another aspect of the present invention, there is a method of treating a bone disease comprising: administering to a mammal in need of said treatment a therapeutically effective amount of a compound that lowers leptin receptor levels in hypothalamus, wherein the bone disease is characterized by a decreased bone mass relative to that of corresponding non-diseased bone. Specific embodiments of some of these compounds and methods include, but are not limited to ones that inhibit or lower leptin receptor synthesis or increase leptin receptor breakdown. Among such compounds are antisense, ribozyme or triple helix sequences of a leptin receptor-encoding polypeptide.

A compound that lowers leptin levels in blood serum or in cerebrospinal fluid is one that lowers leptin levels in the following assay: contacting the compound with a cell from a leptin expressing cell line, preferably a NIH3T3L1 cell line, and determining whether leptin expression and/or synthesis is lowered relative to the level exhibited by the cell line in the absence of the compound. Standard assays such as Northern Blot can be used to determine levels of leptin expression and Western Blot can be used to determine levels of leptin synthesis. An alternate assay comprises comparing the level of leptin in a mammal being treated for a bone disease before and after administration of the compound, such that, if the level of leptin decreases, the compound is one that lowers leptin levels. Likewise, a compound that increases leptin levels in blood serum or in cerebrospinal fluid is one that increases leptin levels via such assays.

A compound that lowers the level of phosphorylated Stat3 polypeptide, a downstream effector of leptin signaling in its target cells (Tartaglia et al., 1995, Cell 83, 1263-1271; Baumann et al., 1996, Proc Natl Acad Sci USA 93, 8374-8378; Ghilardi et al., 1996, Proc Nati Acad Sci USA 93, 6231-6235; Vaisse et al., 1996, Nat Genet 14, 95-97), is one that lowers the level of phosphorylated Stat3 in the following assay: contacting a leptin polypeptide and the compound with a cell that expresses a functional leptin receptor and determining the level of phosphorylated Stat3 polypeptide in the cell. To determine the level of phosphorylation of Stat3 polypeptide, the cells can, for example, be lysed and an appropriate analysis (e.g., Western Blot) can be performed. If the level of phosphorylated Stat3 decreases relative to the level exhibited by the cell line in the absence of the compound, the compound is one that lowers the level of phosphorylated Stat3. Likewise, a compound that increases the level of phosphorylated Stat3 polypeptide in blood serum or in cerebrospinal fluid is one that increases leptin levels via such assays.

A compound that inhibits the activity of dopamine β hydroxylase ("DBH"), the enzyme necessary for converting dopamine to norepinephrine is also provided for in the present invention. The dopamine β-hydroxylase inhibitor properties of test compounds can be determined by an art-recognized in vitro assay which relies upon the DBH-catalyzed conversion of tyramine to octopamine and the inhibition of DBH activity by test compounds. Dopamine-hydroxylase inhibitor properties of test compounds also can be determined by an art-recognized in vivo assay which relies upon dopamine and norepinephrine tissue concentrations and the effect of the test compounds thereon (see. *e.g.,* B. A. Berkowitz et al., 1988 J. Pharm. Exp Ther. 245:850-857). Specific embodiments of some of these compounds and methods include, but are not limited to ones that inhibit DBH enzyme activity, DBH gene expression, antibodies which specifically bind DBH, and compounds which are involved in the norephinephrine synthesis pathway. Examples of known DBH inhibitors, as described in U.S. Patent No. 5,741,503, which is hereby incorporated by reference in its entirety, include, but are not limited to, fusaric acid, disulfiram, 3-phenylpropargylamine and 5-(4'-chlorobutyl)-picolinic acid, diethyidithiocarbamate, beta-chlorophenethylamine, 4-hydroxybenzylcyanide,2-halo-3-(p-hydroxyphenyl)-1 -propene,1-phenyl-1-propyne, 2-phenylallylamine, 2-(2-thienyl)allylamine and derivatives thereof such as 2-thiophene-2-(2-thienyl)allylamine, 3-phenylpropargylamine, 1-phenyl-1(aminoethyl)ethene and derivatives thereof such as N-(trifluoroacetyl)phenyl- (aminoethyl) ethene and 5-picolinic acid derivatives, such as 5-(4'-chlorobutyl)-picolinic acid and other 5-picolinic acids similarly alkyl- or haloalkyl-substituted, e.g., with C₁-C₆ alkyl groups optionally themselves substituted with one or more halogen atoms.

A compound is said to be administered in a "therapeutically effective amount" if the amount administered results in a desired change in the physiology of a recipient mammal, *e.g*., results in an increase or decrease in bone mass relative to that of a corresponding bone in the diseased state; that is, results in treatment, *i.e.,* modulates bone mass to more closely resemble that of corresponding non-diseased bone (that is a corresponding bone of the same type, *e.g*., long, vertebral, *etc*.) in a non-diseased state. With respect to these methods, a corresponding non-diseased bone refers to a bone of the same type as the bone being treated *(e.g.,* a corresponding vertebral or long bone), and bone mass is measured using standard techniques well known to those of skill in the art and described above, and include, for example, X-ray, DEXA and classical histological assessments and measurements of bone mass.

Among the compounds that can be utilized as part of the methods presented herein are those described, for example, in the sections and teached presented herein, as well as compounds identified via techniques such as those described in the sections and teaching presented herein.

Particular techniques and methods that can be utilized as part of the therapeutic and preventative methods of the invention are presented in detail below.

### 5.8.1. Inhibition of Ob or ObR Expression, Levels or Activity to Treat Bone Disease by Increasing Bone Mass

Any method which neutralizes, slows or inhibits Ob or ObR expression (either transcription or translation), levels, or activity can be used to treat or prevent a bone disease characterized by a decrease in bone mass relative to a corresponding non-diseased bone by effectuating an increase in bone mass. Such approaches can be used to treat or prevent bone diseases such as osteoporosis, osteopenia, faulty bone formation or resorption, Paget's disease, bone metastasis, osteomalacia and renal osteodystrophy. Such methods can be utilized to treat states involving bone fractures and broken bones.

For example, the administration of componds such as soluble peptides, proteins, fusion proteins, or antibodies (including anti-idiotypic antibodies) that bind to and "neutralize" circulating Ob, the natural ligand for the ObR, can be used to effectuate an increase in bone mass. Similarly, such compounds as soluble peptides, proteins, fusion proteins, or antibodies (including anti-idiotypic antibodies) can be used to effectuate an increase in bone mass. To this end, peptides corresponding to the ECD of ObR, soluble deletion mutants of ObR, or either of these ObR domains or mutants fused to another polypeptide (*e*.*g*., an IgFc polypeptide) can be utilized. Alternatively, anti-idiotypic antibodies or Fab fragments of antiidiotypic antibodies that mimic the ObR ECD and neutralize Ob can be used. Alternatively, compounds that inhibit ObR homodimerization such that leptin's affinity for the leptin receptor is decreased, also can be used (see, *e.g.,* Devos et al., 1997, J. Biol. Chem., 272:18304-18310). For treatment, such ObR peptides, proteins, fusion proteins, anti-idiotypic antibodies or Fabs are administered to a subject in need of treatment at therapeutically effective levels. For prevention, such ObR peptides, proteins, fusion proteins, anti-idiotypic antibodies or Fabs are administered to a subject at risk for a bone disease, for a time and concentration sufficient to prevent the bone disease.

In an alternative embodiment for neutralizing circulating Ob, cells that are genetically engineered to express such soluble or secreted forms of ObR may be administered to a patient, whereupon they will serve as "bioreactors" *in vivo* to provide a continuous supply of the Ob neutralizing protein. Such cells may be obtained from the patient or an MHC compatible donor and can include, but are not limited to, fibroblasts, blood cells (e.g., lymphocytes), adipocytes, muscle cells, endothelial cells, *etc.* The cells are genetically engineered *in vitro* using recombinant DNA techniques to introduce the coding sequence for the ObR ECD, or for ObR-Ig fusion protein into the cells, *e.g*., by transduction (using viral vectors, and preferably vectors that integrate the transgene into the cell genome) or transfection procedures, including, but not limited to, the use of plasmids, cosmids, YACs, electroporation, liposomes, *etc.* The ObR coding sequence can be placed under the control of a strong constitutive or inducible promoter or promoter/enhancer to achieve expression and secretion of the ObR peptide or fusion protein. The engineered cells which express and secrete the desired ObR product can be introduced into the patient systemically, *e.g*., in the circulation, intraperitoneally, at the choroid plexus or hypothalamus. Alternatively, the cells can be incorporated into a matrix and implanted in the body, e.g., genetically engineered fibroblasts can be implanted as part of a skin graft; genetically engineered endothelial cells can be implanted as part of a vascular graft (see, *e.g*., U.S. Pat. Nos. 5,399,349 and 5,460,959 each of which is incorporated by reference herein in its entirety).

When the cells to be administered are non-autologous cells, they can be administered using well known techniques which prevent the development of a host immune response against the introduced cells. For example, the cells may be introduced in an encapsulated form which, while allowing for an exchange of components with the immediate extracellular environment, does not allow the introduced cells to be recognized by the host immune system.

In an alternate embodiment, bone disease therapy can be designed to reduce the level of endogenous Ob or ObR gene expression, *e.g.*, using antisense or ribozyme approaches to inhibit or prevent translation of Ob or ObR mRNA transcripts; triple helix approaches to inhibit transcription of the Ob or ObR gene; or targeted homologous recombination to inactivate or "knock out" the Ob or ObR gene or its endogenous promoter. Because the ObR gene is expressed in the brain, including the choroid plexus and hypothalamus, delivery techniques should be preferably designed to cross the blood-brain barrier (see, *e.g.*, PCT Publication WO89/10134, which is incorporated by reference herein in its entirety). Alternatively, the antisense, ribozyme or DNA constructs described herein could be administered directly to the site containing the target cells; e.g., the choroid plexus, hypothalamus, adipose tissue, *etc.*

Antisense approaches involve the design of oligonucleotides (either DNA or RNA) that are complementary to Ob or ObR mRNA. The antisense oligonucleotides will bind to the complementary Ob or ObR mRNA transcripts and prevent translation. Absolute complementarity, although preferred, is not required. A sequence "complementary" to a portion of an RNA, as referred to herein, means a sequence having sufficient complementarity to be able to hybridize with the RNA, forming a stable duplex; in the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

The skilled artisan recognizes that modifications of gene expression can be obtained by designing antisense molecules to the control regions of the leptin or leptin receptor genes, *i.e*. promoters, enhancers, and introns, as well as to the coding regions of these genes. Such sequences are referred to herein as leptin-encoding polynucleotides or leptin receptor-encoding polynucleotides, respectively.

Oligonucleotides derived from the transcription initiation site, *e.g*. between -10 and +10 regions of the leader sequence, are preferred. Oligonucleotides that are complementary to the 5' end of the message, *e.g.,* the 5' untranslated sequence up to and including the AUG initiation codon, generally work most efficiently at inhibiting translation. However, sequences complementary to the 3' untranslated sequences of mRNAs have recently shown to be effective at inhibiting translation of mRNAs as well (see generally, Wagner, 1994, Nature 372:333-335). Oligonucleotides complementary to the 5' untranslated region of the mRNA should include the complement of the AUG start codon. Antisense oligonucleotides complementary to mRNA coding regions are less efficient inhibitors of translation but could be used in accordance with the invention. Whether designed to hybridize to the 5'-, 3'- or coding region of Ob or ObR mRNA, antisense nucleic acids should be at least six nucleotides in length, and are preferably oligonucleotides ranging from 6 to about 50 nucleotides in length. In specific aspects the oligonucleotide is at least nucleotides, at least 17 nucleotides, at least 25 nucleotides or at least 50 nucleotides.

Regardless of the choice of target sequence, it is preferred that *in vitro* studies are first performed to quantitate the ability of the antisense oligonucleotide to inhibit gene expression. It is preferred that these studies utilize controls that distinguish between antisense gene inhibition and nonspecific biological effects of oligonucleotides. It is also preferred that these studies compare levels of the target RNA or protein with that of an internal control RNA or protein. Additionally, it is envisioned that results obtained using the antisense oligonucleotide are compared with those obtained using a control oligonucleotide. It is preferred that the control oligonucleotide is of approximately the same length as the test oligonucleotide and that the nucleotide sequence of the oligonucleotide differs from the antisense sequence no more than is necessary to prevent specific hybridization to the target sequence.

The oligonucleotides can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridization, *etc*. The oligonucleotide may include other appended groups such as peptides *(e.g.,* for targeting host cell receptors *in vivo),* or agents facilitating transport across the cell membrane (see, *e.g.,* Letsinger et al., 1989, Proc. Natl. Acad. Sci. U.S.A. 86:6553-6556 and Lemaitre et al., 1987, Proc. Natl. Acad. Sci. 84:648-652; PCT Publication No. WO88/09810) or the blood-brain barrier (see, *e.g.,* PCT Publication No. WO89/10134), hybridization-triggered cleavage agents (see, *e.g.,* Krol et al., 1988, BioTechniques 6:958-976) or intercalating agents (see, *e.g.,* Zon, 1988, Pharm. Res. 5:539-549). To this end, the oligonucleotide may be conjugated to another molecule, *e.g.*, a peptide, hybridization triggered cross-linking agent, transport agent, hybridization-triggered cleavage agent, *etc.*

The antisense oligonucleotide may comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide may also comprise at least one modified sugar moiety selected from the group including but not limited to arabinose, 2-fluoroarabinose, xylulose, and hexose.

In yet another embodiment, the antisense oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an α-anomeric oligonucleotide. An α-anomeric oligonucleotide forms specific double-stranded hybrids with complementary RNA in which, contrary to the usual β-units, the strands run parallel to each other (Gautier et al., 1987, Nucl. Acids Res. 15:6625-6641). The oligonucleotide is a 2'-0-methylribonucleotide (Inoue et al., 1987, Nucl. Acids Res. 15:6131-6148), or a chimeric RNA-DNA analogue (Inoue et al., 1987, FEBS Lett. 215:327-330).

Oligonucleotides of the invention may be synthesized by standard methods known in the art, e.g. by use of an automated DNA synthesizer (such as are commercially available from Biosearch, Applied Biosystems, etc.). As examples, phosphorothioate oligonucleotides may be synthesized by the method of Stein et al., 1988, Nucl. Acids Res. 16:3209, and methylphosphonate oligonucleotides can be prepared by use of controlled pore glass polymer supports (Sarin et al., 1988, Proc. Natl. Acad. Sci. U.S.A. 85:7448-7451).

While antisense nucleotides complementary to the Ob or ObR coding region sequence could be used, those complementary to the transcribed untranslated region are most preferred. For example, antisense oligonucleotides to the ObR coding region having the following sequences can be utilized in accordance with the invention:
(a) 5'-CATCTTACTTCAGAGAA-3' (SEQ ID NO: 1)
(b) 5'-CATCTTACTTCAGAGAAGTACAC-3' (SEQ ID NO: 2)
(c) 5'-CATCTTACTTCAGAGAAGTACACCCATAA-3' (SEQ ID NO: 3)
(d) 5'-CATCTTACTTCAGAGAAGTACACCCATAATCCTCT-3' (SEQ ID NO: 4)
(e) 5'-AATCATCTTACTTCAGAGAAGTACACCCATAATCC-3' (SEQ ID NO: 5)
(f) 5'-CTTACTTCAGAGAAGTACACCCATAATCC-3' (SEQ ID NO: 6)
(g) 5'-TCAGAGAAGTACACCCATAATCC-3' (SEQ ID NO: 7)
(h) 5'-AAGTACACCCATAATCC-3' (SEQ ID NO: 8)

The antisense molecules should be delivered to cells which express the Ob or ObR *in vivo.* A number of methods have been developed for delivering antisense DNA or RNA to cells; *e.g.,* antisense molecules can be injected directly into the tissue site, or modified antisense molecules, designed to target the desired cells (e.g., antisense linked to peptides or antibodies that specifically bind receptors or antigens expressed on the target cell surface) can be administered systemically.

A preferred approach for achieving intracellular concentrations of the antisense sufficient to suppress translation of endogenous mRNAs utilizes a recombinant DNA construct in which the antisense oligonucleotide is placed under the control of a strong pol III or pol II promoter. The use of such a construct to transfect target cells in the patient will result in the transcription of sufficient amounts of single stranded RNAs that will form complementary base pairs with the endogenous Ob or ObR transcripts and thereby prevent translation of the Ob or ObR mRNA, respectively. For example, a vector can be introduced *in vivo* such that it is taken up by a cell and directs the transcription of an antisense RNA. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in mammalian cells. Expression of the sequence encoding the antisense RNA can be by any promoter known in the art to act in mammalian, preferably human cells. Such promoters can be inducible or constitutive. Such promoters include but are not limited to: the SV40 early promoter region (Bemoist & Chambon, 1981, Nature 290:304-310), the promoter contained in the 3' long terminal repeat of Rous sarcoma virus (Yamamoto et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al., 1982, Nature 296:39-42), etc. Any type of plasmid, cosmid, YAC or viral vector can be used to prepare the recombinant DNA construct which can be introduced directly into the tissue site; *e.g*., the choroid plexus or hypothalamus. Alternatively, viral vectors can be used which selectively infect the desired tissue; (*e.g.*, for brain, herpesvirus vectors may be used), in which case administration may be accomplished by another route (e.g., systemically).

Ribozyme molecules-designed to catalytically cleave Ob or ObR mRNA transcripts can also be used to prevent translation of Ob or ObR mRNA and expression of Ob or ObR (see, *e.g.*, PCT International Publication WO90/11364 and Sarver et al., 1990, Science 247:1222-1225). While ribozymes that cleave mRNA at site specific recognition sequences can be used to destroy Ob or ObR mRNAs, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Haseloff & Gerlach, 1988, Nature, 334:585-591. There are hundreds of potential hammerhead ribozyme cleavage sites within the nucleotide sequence of human Ob and ObR cDNA (see, *e.g.,* U.S. Patent No. 5,972,621). Preferably, the ribozyme is engineered so that the cleavage recognition site is located near the 5' end of the Ob or ObR mRNA; *i.e.,* to increase efficiency and minimize the intracellular accumulation of non-functional mRNA transcripts.

For example, hammerhead ribozymes directed to ObR mRNA having the following sequences can be utilized in accordance with the invention:
(a) 
5'-ACAGAAUUUUUGACAAAUCAAAGCAGANNNNUCUGAGNAGUCCUUAC
UUCAGAGAA-3' (SEQ ID NO: 9);
(b) 
5'-GGCCCGGGCAGCCUGCCCAAAGCCGGNNNNCCGGAGNAGUCGCCAGA
CCGGCUCGUG-3' (SEQ ID NO: 10);
(c) 
5'-UGGCAUGCAAGACAAAGCAGGNNNNCCUGAGNAGUCCUUAAAUCUCC
AAGGAGUAA-3' (SEQ ID NO: 11);
(d) 
5'-UAUAUGACAAAGCUGUNNNNACAGAGNAGUCCUUGUGUGGUAAAGAC
ACG-3' (SEQ ID NO: 12);
(e) 
5'-AGCACCAAUUGAAUUGAUGGCCAAAGCGGGNNNNCCCGAGNAGUCAA
CCGUAACAGUAUGU-3' (SEQ ID NO: 13);
(f) 
5'-UGAAAUUGUUUCAGGCUCCAAAGCCGGNNNNCCGGAGNAGUCAAGAA
GAGGACCACAUGUCACUGAUGC-3' (SEQ ID NO: 14);
(g) 
5'-GGUUUCUUCAGUGAAAUUACACAAAGCAGCNNNNGCUGAGNAGUCAG
UUAGGUCACACAUC-3' (SEQ ID NO: 15);
(h)

5'-ACCCAUUAUAACACAAAGCUGANNNUCAGAGNAGUCAUCUGAAGGU
UUCUUC -3' (SEQ ID NO: 16).

The ribozymes of the present invention also include RNA endoribonucleases (hereinafter "Cech-type ribozymes") such as the one which occurs naturally in *Tetrahymena thermophila* (known as the IVS, or L-19 IVS RNA) and which has been extensively described (Zaug et al., 1984, Science, 224:574-578; Zaug & Cech, 1986, Science, 231:470-475; Zaug et al., 1986, Nature, 324:429-433; PCT Publication No. WO 88/04300; Been & Cech, 1986, Cell, 47:207-216). The Cech-type ribozymes have an eight base pair active site which hybridizes to a target RNA sequence whereafter cleavage of the target RNA takes place. The invention encompasses those Cech-type ribozymes which target eight base-pair active site sequences that are present in Ob and ObR.

As in the antisense approach, the ribozymes can be composed of modified oligonucleotides (*e.g.* for improved stability, targeting, *etc.)* and should be delivered to cells which express Ob and ObR *in vivo.* A preferred method of delivery involves using a DNA construct "encoding" the ribozyme under the control of a strong constitutive pol III or pol II promoter, so that transfected cells will produce sufficient quantities of the ribozyme to destroy endogenous Ob or ObR messages and inhibit translation. Because ribozymes, unlike antisense molecules, are catalytic, a lower intracellular concentration is required for efficiency.

Similarly, leptin or leptin receptor inhibition can be achieved by using "triple helix" base-pairing methodology. Triple helix pairing compromises the ability of the double helix to open sufficiently for the binding of polymerases, transcription factors, or regulatory molecules. Techniques for utilizing triple helix technology are well known to those of skill in the art (see generally, Helene, 1991, Anticancer Drug Des. 6(6):569-84; Helene, 1992, Ann. N.Y. Acad. Sci. 660:27-36; and Maher, 1992, Bioassays 14(12):807-15).

Endogenous Ob or ObR gene expression can also be reduced by inactivating or "knocking out" the Ob or ObR gene or its promoter using targeted homologous recombination (see, *e.g.,* Smithies et al., 1985, Nature 317:230-234; Thomas & Capecchi, 1987, Cell 51:503-512; and Thompson et al., 1989 Cell 5:313-321; each of which is incorporated by reference herein in its entirety). For example, a mutant, non-functional Ob or ObR (or a completely unrelated DNA sequence) flanked by DNA homologous to the endogenous Ob or ObR gene (either the coding regions or regulatory regions) can be used, with or without a selectable marker and/or a negative selectable marker, to transfect cells that express Ob or ObR *in vivo.* Insertion of the DNA construct, via targeted homologous recombination, results in inactivation of the Ob or ObR gene. Such approaches are particularly suited in the agricultural field where modifications to ES (embryonic stem) cells can be used to generate animal offspring with an inactive ObR (see, e.g., Thomas & Capecchi 1987 and Thompson 1989, *supra*). However, this approach can be adapted for use in humans provided the recombinant DNA constructs are directly administered or targeted to the required site *in vivo* using appropriate viral vectors, *e.g.,* herpes virus vectors for delivery to brain tissue; *e.g.,* the hypothalamus, choroid plexus, or adipose tissue.

Alternatively, endogenous Ob or ObR gene expression can be reduced by targeting deoxyribonucleotide sequences complementary to the regulatory region of the Ob or ObR gene (*i.e.*, promoters and/or enhancers) to form triple helical structures that prevent transcription of the Ob or ObR gene in target cells in the body (see generally, Helene, C. 1991, Anticancer Drug Des., 6(6):569-84; Helene, C., et al., 1992, Ann, N.Y. Accad. Sci., 660:27-36; and Maher, L. J., 1992, Bioassays 14(12):807-15).

In yet another embodiment of the invention, the activity of Ob or ObR can be reduced using a "dominant negative" approach to effectuate an increase in bone mass. To this end, constructs which encode defective Ob or ObRs can be used in gene therapy approaches to diminish the activity of the Ob or ObR in appropriate target cells. For example, nucleotide sequences that direct host cell expression of ObRs in which the CD or a portion of the CD is deleted or mutated can be introduced into cells in the choroid plexus or hypothalamus (either by *in vivo* or *ex vivo* gene therapy methods described above). Alternatively, targeted homologous recombination can be utilized to introduce such deletions or mutations into the subject's endogenous ObR gene in the hypothalamus or choroid plexus. The engineered cells will express non-functional receptors (*i.e.*, an anchored receptor that is capable of binding its natural ligand, but incapable of signal transduction). Such engineered cells present in the choroid plexus or hypothalamus should demonstrate a diminished response to the endogenous Ob ligand, resulting in an increase in bone mass.

An additional embodiment of the present invention is a method to decrease leptin levels by increasing breakdown of leptin protein, *i.e.*, by binding of an antibody such that the leptin protein is targeted for removal. An alternative embodiment of the present invention is a method to decrease leptin receptor levels by increasing the breakdown of leptin receptor protein, *i.e.,* by binding of an antibody such that the leptin receptor protein is targeted for removal. Another embodiment is to decrease leptin levels by increasing the synthesis of a soluble form of the leptin receptor, which binds to free leptin.

Another embodiment of the present invention is a method to administer compounds which affect leptin receptor structure, function or homodimerization properties. Such compounds include, but are not limited to, proteins, nucleic acids, carbohydrates or other molecules which upon binding alter leptin receptor structure, function, or homodimerization properties, and thereby render the receptor ineffectual in its activity.

### 5.8.2. Restoration or Increase in Ob or ObR Expression or Activity to Decrease Bone Mass

With respect to an increase in the level of normal Ob or ObR gene expression and/or gene product activity, Ob or ObR nucleic acid sequences can be utilized for the treatment of bone disorders. Where the cause of the disorder is a defective Ob or ObR, treatment can be administered, for example, in the form of gene replacement therapy. Specifically, one or more copies of a normal Ob or ObR gene or a portion of the Ob or ObR gene that directs the production of an Ob or ObR gene product exhibiting normal function, may be inserted into the appropriate cells within a patient or animal subject, using vectors which include, but are not limited to, adenovirus, adeno-associated virus, retrovirus and herpes virus vectors, in addition to other particles that introduce DNA into cells, such as liposomes.

Because the ObR gene is expressed in the brain, including the choroid plexus and hypothalamus, such gene replacement therapy techniques involving ObR should be capable of delivering ObR gene sequences to these cell types within patients. Thus, the techniques for delivery of the ObR gene sequences should be designed to readily cross the blood-brain barrier, which are well known to those of skill in the art (see, e.g., PCT publication No. WO89/10134, which is incorporated herein by reference in its entirety) or, alternatively, should involve direct administration of such ObR gene sequences to the site of the cells in which the ObR gene sequences are to be expressed. Alternatively, targeted homologous recombination can be utilized to correct the defective endogenous Ob or ObR gene in the appropriate tissue. In animals, targeted homologous recombination can be used to correct the defect in ES cells in order to generate offspring with a corrected trait.

Additional methods which may be utilized to increase the overall level of Ob or ObR gene expression and/or activity include the introduction of appropriate Ob or ObR-expressing cells, preferably autologous cells, into a patient at positions and in numbers which are sufficient to ameliorate the symptoms of bone disorders associated with increased bone mass. Such cells may be either recombinant or non-recombinant. Among the cells which can be administered to increase the overall level of Ob or ObR gene expression in a patient are normal cells, preferably choroid plexus cells, or hypothalamus cells which express the ObR gene, or adipocytes, which express the Ob gene. The cells can be administered at the anatomical site in the brain or in the adipose tissue, or as part of a tissue graft located at a different site in the body. Such cell-based gene therapy techniques are well known to those skilled in the art (see, *e.g.,* U.S. Pat. Nos. 5,399,349 and 5,460,959).

Finally, compounds, identified in the assays described above, that stimulate or enhance the signal transduced by activated ObR, e.g., by activating downstream signaling proteins in the ObR cascade and thereby by-passing the defective ObR, can be used to achieve decreased bone mass. The formulation and mode of administration will depend upon the physico-chemical properties of the compound. The administration should include known techniques that allow for a crossing of the blood-brain barrier.

### 5.8.3. Gene Therapy Approaches to Controlling Ob and ObR Activity and Treating or Preventing Bone Disease

The expression of Ob and ObR can be controlled *in vivo* (*e.g.* at the transcriptional or translational level) using gene therapy approaches to regulate Ob and ObR activity and treat bone disorders. Certain approaches are described below.

With respect to an increase in the level of normal Ob and ObR gene expression and/or Ob and ObR gene product activity, Ob and ObR nucleic acid sequences can be utilized for the treatment of bone diseases. Where the cause of the bone disease is a defective Ob or ObR gene, treatment can be administered, for example, in the form of gene replacement therapy. Specifically, one or more copies of a normal Ob or ObR gene or a portion of the gene that directs the production of a gene product exhibiting normal function, may be inserted into the appropriate cells within a patient or animal subject, using vectors which include, but are not limited to adenovirus, adeno-associated virus, retrovirus and herpes virus vectors, in addition to other particles that introduce DNA into cells, such as liposomes.

Because the ObR gene is expressed in the brain, including the cortex, thalamus, brain stem and spinal cord and hypothalamus, such gene replacement therapy techniques should be capable of delivering ObR gene sequences to these cell, types within patients. Thus, the techniques for delivery of the ObR gene sequences should be designed to readily cross the blood-brain barrier, which are well known to those of skill in the art (see, e.g., PCT publication No. WO89/10134, which is incorporated herein by reference in its entirety), or, alternatively, should involve direct administration of such ObR gene sequences to the site of the cells in which the ObR gene sequences are to be expressed.

Alternatively, targeted homologous recombination can be utilized to correct the defective endogenous Ob or ObR gene in the appropriate tissue; e.g., adipose and brain tissue, respectively. In animals, targeted homologous recombination can be used to correct the defect in ES cells in order to generate offspring with a corrected trait.

Additional methods which may be utilized to increase the overall level of Ob or ObR gene expression and/or activity include the introduction of appropriate Ob or ObR-expressing cells, preferably autologous cells, into a patient at positions and in numbers which are sufficient to ameliorate the symptoms of bone disorders, including, but not limited to, osteopetrosis, osteosclerosis and osteochondrosis. Such cells may be either recombinant or non-recombinant. Among the cells which can be administered to increase the overall level of Ob or ObR gene expression in a patient are normal cells, or adipose or hypothalamus cells which express the Ob or ObR gene, respectively. The cells can be administered at the anatomical site in the adipose tissue or in the brain, or as part of a tissue graft located at a different site in the body. Such cell-based gene therapy techniques are well known to those skilled in the art, see, e.g., U.S. Pat. Nos. 5,399,349 and 5,460,959.

### 5.8.4. Combination Therapy for Modulation of Bone

One aspect of the present invention is a method for treating and preventing osteoporosis comprising the administration of β adrenergic antagonists, such as, but not limited to, β₁, β₂, and β₃ antagonists. A specific embodiment of this aspect includes, but is not limited to, the further administration of a leptin antagonist in combination with β adrenergic antagonists.

In another embodiment, there is a method for treating and preventing osteopetrosis or osteosclerosis comprising the administration of β adrenergic agonists such as, but not limited to, β₁, β₂, and β₃ agonists. A further embodiment includes, but is not limited to, the further administration of a leptin agonist in combination with β adrenergic agonists.

Another object of the present invention relates to a method of modulating leptin effects on bone comprising the administration of a therapeutically effective amount of a pharmaceutical composition that alters the sympathetic tone. Specific embodiments of the pharmaceutical composition include, but are not limited to, leptin antagonists, leptin agonists, sympathetic nervous system antagonists, sympathetic nervous system agonists, and combinations thereof.

Another object of the present invention relates to methods of treating or preventing a bone disease comprising the administration of a therapeutically effective amount of a pharmaceutical composition that modulates leptin effects in bone by altering sympathetic tone. Specific embodiments of the pharmaceutical composition include, but are not limited to, leptin antagonists, leptin agonists, sympathetic nervous system antagonists, sympathetic nervous system agonists, and combinations thereof.

The invention encompasses combinations of the compounds described in Sections 5.4, 5.5, 5.6, and 5.7. In a preferred embodiment, the combinations of the compounds results in a synergistic effect. The term "synergistic" as used herein refers to a combination which is more effective than the additive effects of any two or more single agents. A determination of a synergistic interaction between compounds that modulate sympathetic tone, such as but not limited to β antagonists, and another therapeutic agent may be based on the results obtained from X-ray and histomorphometry analysis, as described in Section 6.2. *infra.* The results of these assays may be analyzed by any method known in the art, including Chou and Talalay's combination method and dose-effect analysis with microcomputers' software, in order to obtain a Combination Index (Chou and Talalay, 1984, Adv. Enzyme Regul. 22:27-55 and Chou and Chou, 1987, software and manual, Elsevier Biosoft, Cambridge, UK, pp. 19-64). Combination Index values < 1 indicates synergy, values > 1 indicate antagonism and values equal to 1 indicate additive effects.

In another embodiment, the combinations of any of the compounds described in Sections 5.4, 5.5, 5.6, and 5.7 are administered sequentially. For example, but not by limitation, a leptin antagonist can be administered in combination with an adrenergic antagonist wherein an leptin antagonist is administered first, followed by an adrenergic antagonist, or vice versa. Similarly, by example and not by limitation, an adrenergic agonist can be administered in combination with a leptin agonist wherein an adrenergic agonist is administered first, followed by a leptin agonist, or vice versa. In another non-limiting example, a DBH antagonist can be administered in combination with a leptin antagonist and/or an adrenergic antagonist. The sequential addition of compounds can involve two or more compounds. One skilled in the art can determine the necessary sequence of compounds to exert the desired effect.

### 5.9. Pharmaceutical Formulations and Methods of Treating Bone Disorders

The compounds of this invention can be formulated and administered to inhibit a variety of bone disease states by any means that produces contact of the active ingredient with the agent's site of action in the body of a mammal. They can be administered by any conventional means available for use in conjunction with pharmaceuticals, either as individual therapeutic active ingredients or in a combination of therapeutic active ingredients. They can be administered alone, but are generally administered with a pharmaceutical carrier selected on the basis of the chosen route of administration and standard pharmaceutical practice.

The dosage administered will be a therapeutically effective amount of the compound sufficient to result in amelioration of symptoms of the bone disease and will, of course, vary depending upon known factors such as the pharmacodynamic characteristics of the particular active ingredient and its mode and route of administration; age, sex, health and weight of the recipient; nature and extent of symptoms; kind of concurrent treatment, frequency of treatment and the effect desired.

### 5.9.1. Dose Determinations

Toxicity and therapeutic efficacy of such compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀ /ED₅₀. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ *(i.e.,* the concentration of the test compound which achieves a half-maximal inhibition of symptoms) as determined in cell culture. Such information can be used to more accurately determine useful doses in humans. Levels in plasma may be measured, for example, by high performance liquid chromatography.

Specific dosages may also be utilized for antibodies. Typically, the preferred dosage is 0.1 mg/kg to 100 mg/kg of body weight (generally 10 mg/kg to 20 mg/kg), and if the antibody is to act in the brain, a dosage of 50 mg/kg to 100 mg/kg is usually appropriate. If the antibody is partially human or fully human, it generally will have a longer half-life within the human body than other antibodies. Accordingly, lower dosages of partially human and fully human antibodies is often possible. Additional modifications may be used to further stabilize antibodies. For example, lipidation can be used to stabilize antibodies and to enhance uptake and tissue penetration (e.g., into the brain). A method for lipidation of antibodies is described by Cruikshank et al., 1997, J. Acquired Immune Deficiency Syndromes and Human Retrovirology 14:193.

A therapeutically effective amount of protein or polypeptide *(i.e.,* an effective dosage) ranges from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight.

Moreover, treatment of a subject with a therapeutically effective amount of a protein, polypeptide or antibody can include a single treatment or, preferably, can include a series of treatments. In a preferred example, a subject is treated with antibody, protein, or polypeptide in the range of between about 0.1 to 20 mg/kg body weight, one time per week for between about 1 to 10 weeks, preferably between 2 to 8 weeks, more preferably between about 3 to 7 weeks, and even more preferably for about 4, 5 or 6 weeks.

The present invention further encompasses agents which modulate expression or activity. An agent may, for example, be a small molecule. For example, such small molecules include, but are not limited to, peptides, peptidomimetics, amino acids, amino acid analogs, polynucleotides, polynucleotide analogs, nucleotides, nucleotide analogs, organic or inorganic compounds (*i*.*e*,. including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

It is understood that appropriate doses of small molecule agents depends upon a number of factors known to those or ordinary skill in the art, e.g., a physician. The dose(s) of the small molecule will vary, for example, depending upon the identity, size, and condition of the subject or sample being treated, further depending upon the route by which the composition is to be administered, if applicable, and the effect which the practitioner desires the small molecule to have upon the nucleic acid or polypeptide of the invention. Exemplary doses include milligram or microgram amounts of the small molecule per kilogram of subject or sample weight (e.g., about 1 microgram per kilogram to about 500 milligrams per kilogram, about 100 micrograms per kilogram to about 5 milligrams per kilogram, or about 1 microgram per kilogram to about 50 micrograms per kilogram.

### 5.9.2. Formulations and Use

Pharmaceutical compositions for use in accordance with the present invention may be formulated in conventional manner using one or more physiologically acceptable carriers or excipients.

Thus, the compounds and their physiologically acceptable salts and solvates may be formulated for administration by inhalation or insufflation (either through the mouth or the nose) or oral, buccal, parenteral or rectal administration.

For oral administration, the pharmaceutical compositions may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (*e*.*g*., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound.

For buccal administration the compositions may take the form of tablets or lozenges formulated in conventional manner.

For administration by inhalation, the compounds for use according to the present invention are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

The compounds may be formulated for parenteral administration by injection, e.g., by bolus injection or continuous infusion. Formulations for injection may be presented in unit dosage form, *e.g.,* in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use. In general, water, a suitable oil, saline, aqueous dextrose (glucose), and related sugar solutions and glycols such as propylene glycol or polyethylene glycols are suitable carriers for parenteral solutions. Solutions for parenteral administration contain preferably a water soluble salt of the active ingredient, suitable stabilizing agents and, if necessary, buffer substances. Antioxidizing agents such as sodium bisulfate, sodium sulfite or ascorbic acid, either alone or combined, are suitable stabilizing agents. Also used are citric acid and its salts and sodium ethylenediaminetetraacetic acid (EDTA). In addition, parenteral solutions can contain preservatives such as benzalkonium chloride, methyl- or propyl-paraben and chlorobutanol. Suitable pharmaceutical carriers are described in *Remington's Pharmaceutical Sciences,* a standard reference text in this field.

The compounds may also be formulated in rectal compositions such as suppositories or retention enemas, e.g., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

Additionally, standard pharmaceutical methods can be employed to control the duration of action. These are well known in the art and include control release preparations and can include appropriate macromolecules, for example polymers, polyesters, polyamino acids, polyvinyl, pyrolidone, ethylenevinylacetate, methyl cellulose, carboxymethyl cellulose or protamine sulfate. The concentration of macromolecules as well as the methods of incorporation can be adjusted in order to control release. Additionally, the agent can be incorporated into particles of polymeric materials such as polyesters, polyamino acids, hydrogels, poly (lactic acid) or ethylenevinylacetate copolymers. In addition to being incorporated, these agents can also be used to trap the compound in microcapsules.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration.

Useful pharmaceutical dosage forms, for administration of the compounds of this invention can be illustrated as follows:
Capsules: Capsules are prepared by filling standard two-piece hard gelatin capsulates each with the desired amount of powdered active ingredient, 175 milligrams of lactose, 24 milligrams of talc and 6 milligrams magnesium stearate.
Soft Gelatin Capsules: A mixture of active ingredient in soybean oil is prepared and injected by means of a positive displacement pump into gelatin to form soft gelatin capsules containing the desired amount of the active ingredient. The capsules are then washed and dried.
Tablets: Tablets are prepared by conventional procedures so that the dosage unit is the desired amount of active ingredient. 0.2 milligrams of colloidal silicon dioxide, 5 milligrams of magnesium stearate, 275 milligrams of microcrystalline cellulose, 11 milligrams of cornstarch and 98.8 milligrams of lactose. Appropriate coatings may be applied to increase palatability or to delay absorption.
Injectable: A parenteral composition suitable for administration by injection is prepared by stirring 1.5% by weight of active ingredients in 10% by volume propylene glycol and water. The solution is made isotonic with sodium chloride and sterilized.
Suspension: An aqueous suspension is prepared for oral administration so that each *5* millimeters contain 100 milligrams of finely divided active ingredient, 200 milligrams of sodium carboxymethyl cellulose, 5 milligrams of sodium benzoate, 1.0 grams of sorbitol solution U.S.P. and 0.025 millimeters of vanillin.
Gene Therapy Administration: Where appropriate, the gene therapy vectors can be formulated into preparations in solid, semisolid, liquid or gaseous forms such as tablets, capsules, powders, granules, ointments, solutions, suppositories, injections, inhalants, and aerosols, in the usual ways for their respective route of administration. Means known in the art can be utilized to prevent release and absorption of the composition until it reaches the target organ or to ensure timed-release of the composition. A pharmaceutically acceptable form should be employed which does not ineffectuate the compositions of the present invention. In pharmaceutical dosage forms, the compositions can be used alone or in appropriate association, as well as in combination, with other pharmaceutically active compounds.

Accordingly, the pharmaceutical composition of the present invention may be delivered via various routes and to various sites in an animal body to achieve a particular effect (see, e.g., Rosenfeld et al., 1991, *supra;* Rosenfeld et al 1991, Clin. Res., 39(2), 311A; Jaffe et al., *supra;* and Berkner, *supra).* One skilled in the art will recognize that although more than one route can be used for administration, a particular route can provide a more immediate and more effective reaction than another route. Local or systemic delivery can be accomplished by administration comprising application or instillation of the formulation into body cavities, inhalation or insufflation of an aerosol, or by parenteral introduction, comprising intramuscular, intravenous, peritoneal, subcutaneous, intradermal, as well as topical administration.

The composition of the present invention can be provided in unit dosage form wherein each dosage unit, e.g., a teaspoonful, tablet, solution, or suppository, contains a predetermined amount of the composition, alone or in appropriate combination with other active agents. The term "unit dosage form" as used herein refers to physically discrete units suitable as unitary dosages for human and animal subjects, each unit containing a predetermined quantity of the compositions of the present invention, alone or in combination with other active agents, calculated in an amount sufficient to produce the desired effect, in association with a pharmaceutically acceptable diluent, carrier, or vehicle, where appropriate. The specifications for the unit dosage forms of the present invention depend on the particular effect to be achieved and the particular pharmacodynamics associated with the pharmaceutical composition in the particular host.

A composition of the present invention can also be formulated as a sustained and/or timed release formulation. Such sustained and/or timed release formulations may be made by sustained release means or delivery devices that are well known to those of ordinary skill in the art, such as those described in U.S. Patent Nos.: 3,845,770; 3,916,899; 3,536,809; 3,598,123; 4,008,719; 4,710,384; 5,674,533; 5,059,595; 5,591,767; 5,120,548; 5,073,543; 5,639,476; 5,354,556; and 5,733,566, the disclosures of which are each incorporated herein by reference. The pharmaceutical compositions of the present invention can be used to provide slow or sustained release of one or more of the active ingredients using, for example, hydropropylmethyl cellulose, other polymer matrices, gels, permeable membranes, osmotic systems, multilayer coatings, microparticles, liposomes, microspheres, or the like, or a combination thereof to provide the desired release profile in varying proportions. Suitable sustained release formulations known to those of ordinary skill in the art, including those described herein, may be readily selected for use with the pharmaceutical compositions of the invention. Thus, single unit dosage forms suitable for oral administration, such as, but not limited to, tablets, capsules, gelcaps, caplets, powders, and the like, that are adapted for sustained release are encompassed by the present invention.

Accordingly, the present invention also provides a method of transferring a therapeutic gene to a host, which comprises administering the vector of the present invention, preferably as part of a composition, using any of the aforementioned routes of administration or alternative routes known to those skilled in the art and appropriate for a particular application: The "effective amount" of the composition is such as to produce the desired effect in a host which can be monitored using several end-points known to those skilled in the art. Effective gene transfer of a vector to a host cell in accordance with the present invention to a host cell can be monitored in terms of a therapeutic effect (e.g. alleviation of some symptom associated with the particular disease being treated) or, further, by evidence of the transferred gene or expression of the gene within the host (*e.g.,* using the polymerase chain reaction in conjunction with sequencing, Northern or Southern hybridizations, or transcription assays to detect the nucleic acid in host cells, or using immunoblot analysis, antibody-mediated detection, mRNA or protein half-life studies, or particularized assays to detect protein or polypeptide encoded by the transferred nucleic acid, or impacted in level or function due to such transfer).

These methods described herein are by no means all-inclusive, and further methods to suit the specific application will be apparent to the ordinary skilled artisan. Moreover, the effective amount of the compositions can be further approximated through analogy to compounds known to exert the desired effect.

Furthermore, the actual dose and schedule can vary depending on whether the compositions are administered in combination with other pharmaceutical compositions, or depending on interindividual differences in pharmacokinetics, drug disposition, and metabolism. Similarly, amounts can vary in *in vitro* applications depending on the particular cell line utilized (e.g., based on the number of adenoviral receptors present on the cell surface, or the ability of the particular vector employed for gene transfer to replicate in that cell line). Furthermore, the amount of vector to be added per cell will likely vary with the length and stability of the therapeutic gene inserted in the vector, as well as also the nature of the sequence, and is particularly a parameter which needs to be determined empirically, and can be altered due to factors not inherent to the methods of the present invention (for instance, the cost associated with synthesis). One skilled in the art can easily make any necessary adjustments in accordance with the exigencies of the particular situation.

The following examples are offered by way of example, and are not intended to limit the scope of the invention in any manner.

### 6. EXAMPLE: RELATIONSHIP BETWEEN LEPTIN AND BONE MASS

### 6.1. Generation Characterization and Treatment of Animals

Breeders and mutant mice (C57BL/6J Lep^{ob}, C5 7BL/6J Lepr^{db}, C57BL/6J A^{y}/a) were purchased from the Jackson Laboratory. Generation of A-ZIP/F-I transgenic mice has been previously reported. (Moitra et al., 1998, Genes Dev 12, 3168-3181) Genotyping was performed according to established protocols (Chua et al., 1997, Genomics 45, 264-270; Moitra et al., 1998, Genes Dev 12, 3168-3181; and Namae et al., 1998, Lab Animal Sci 48, 103-104). Animals were fed a regular diet (Purina #5001) or, when indicated, a high fat/high carbohydrate diet (Bio-serv # F3282). Bone specimens were processed as described (Ducy et al., 1999, Genes Dev 13, 1025-1036).

### 6.2. Demonstration of Bone Mass Phenotype in ob/ob and db/db Mice

Hypogonadism induces an increase in osteoclast number and in bone resorption activity which leads to a low bone mass phenotype (Riggs & Melton, 1986, N Engl J Med 314, 1676-1678). Thus, the ob/ob mice that have a hypogonadism of hypothalamic origin should have a lower bone mass than wild-type littermates (Ahima et al., 1996, Nature 382, 250-252; Chehab et al., 1996, Nat Genet 12, 318-320; Ahima et al., 1997, J Clin Invest 99, 391-395). To determine if the obesity of the ob/ob mice could affect their expected low bone mass phenotype, X-ray analysis of vertebrae and long bones of 6-month-old wild-type and ob/ob mice was performed using a Faxitron (Phillips). Surprisingly, the bones of the ob/ob mice appeared much denser than those of their wild-type littermates (FIG. 1A), demonstrating the presence of a higher amount of mineralized bone matrix. Given the poor sensitivity of X-rays to quantify bone mass abnormalities, the increase in bone density was an indication of a major change in bone architecture (i.e. affecting more than 30% of the bone matrix).

Histologic analysis was performed on undecalcified sections stained with the von Kossa reagent and counterstained with Kemechtrot. In 3 and 6 month-old mice the presence of many more thick trabeculae in the bones of ob/ob mice compared to those of wild-type mice was observed (FIG. 1B). The cortical bone was not affected. Histomorphometric quantification according to standard techniques were performed (Parfitt et al., 1987, J Bone Min Res 2, 595-610) using the Osteomeasure Analysis System (Osteometrics, Atlanta). Statistical differences between groups (n=4 to 6) were assessed by Student's test. The experiments showed a nearly 2-fold increase in trabecular bone volume in long bones and vertebrae of ob/ob mice compared to wild-type littermates (FIG. 1C). This phenotype was observed in both sexes. The functional consequences of this increase in bone mass were analyzed by comparing the biomechanical properties of long bones of 6 month-old ob/ob mice, wild-type mice, and wild-type mice that have been ovariectomized (wild-type-ovx) for 4 months to mimic the hypogonadic state of the ob/ob mice. An assay in which femora were tested to failure by three-point bending on a servo-hydrolic testing machine (Zwick GmbH & Co.) at a constant displacement rate of 10 mm/min was used to determine failure load, which is a measure of the strength of the bones. Failure load of the bones from wild-type and ob/ob mice were undistinguishable but significantly higher than the one observed in the bones of wild-type-ovx mice (F1G. 1D). This result indicates that leptin deficiency has a beneficial effect on the biomechanical properties of the bones. Analysis of the bones of the db/db mice that have an inactivating mutation of the leptin receptor was also performed (Tartaglia et al., 1995, Cell 83, 1263-1271). Like the ob/ob mice, the db/db mice are obese and hypogonadic. There was an increase in the number of trabeculae in both long bones and vertebrae similar to that observed in ob/ob mice (FIG. 1E) resulting in a 3-fold increase in bone volume compared to wild-type mice (FIG. 1F). This latter result demonstrates genetically that leptin signals through its known receptor to affect bone mass.

### 6.3. The High Bone Mass Phenotype of the ob/ob and db/db Mice is Secondary to the Absence of Leptin Signaling and Not to Obesity

The high bone mass phenotype of the ob/ob and ob/db mice could be secondary either to a lack of leptin signaling or to the obesity of these mice. To distinguish between these two possibilities, several additional groups of mutant mice were analyzed for high bone mass phenotype as in Example 6.2. First, a low fat diet which postpones the appearance of obesity in ob/ob mice was fed to ob/ob mice, and they had a normal weight at one month of age. However, the mice already had a high bone mass phenotype at that age (FIG. 2A). Second, heterozygote leptin-deficient mice (ob/+) that are not obese were analyzed. These animals also had a high bone mass phenotype (FIG. 2B). Third, the bones of other mouse models of obesity that are not primarily related to leptin signaling were observed. Another genetic model of obesity, the Agouti yellow (A^{y}/a) mice (Herberg and Coleman, 1977, Metabolism 26, 59-99), had a normal bone mass, as did wild-type mice fed with a high fat diet (FIGS. 2C and 2D).

Thus, the existence of a high bone mass phenotype in ob/ob mice prior to the appearance of obesity and in ob/+ mice that are not obese, and its absence in leptin-unrelated models of obesity demonstrate that it is the absence of leptin signaling, not the obesity, that causes this high bone mass phenotype.

### 6.4. Increased Osteoblast Function in ob/ob and db/db Mice

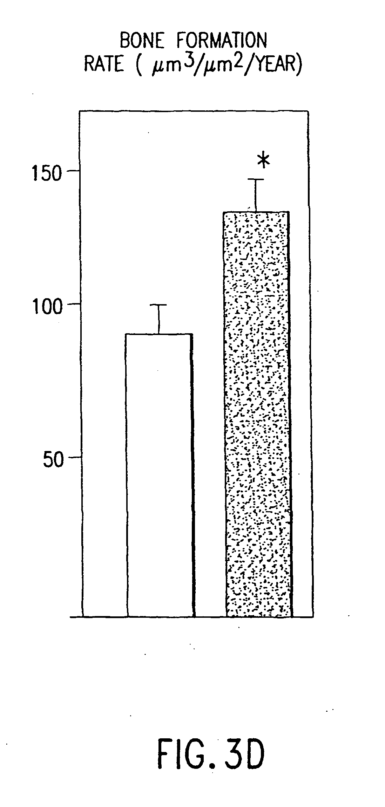

The increase in bone mass could be due to an increase in osteoblastic bone formation, to a decrease in osteoclastic bone resorption, or to a combination of both abnormalities. To study osteoblast function *in vivo,* the rate of bone formation was quantified following double labeling with calcein, a marker of newly formed bone (FIG. 3A) as described (Ducy et al., 1999, Genes Dev 13:1025-1036). Calcein was injected twice at 8 day intervals and animals were sacrificed two days later. In 3 month-old, and 6 month-old ob/ob mice there was a 70% and 60%, respectively, increase of the bone formation rate compared to the one of wild-type littermates (FIG. 3B). This result demonstrated that the high bone mass phenotype of the ob/ob mice was due, at least in part, to an increase in bone formation activity. Remarkably, considering the massive increase of the bone formation rate, the surface of the osteoblasts as well as the osteoblast number were not increased in ob/ob mice, indicating that leptin deficiency affects the function of the osteoblasts and not their differentiation after birth (FIG. 3C). Likewise, calcein labeling of the db/db mice showed an increase in the rate of bone formation in both long bones and vertebrae in the face of a normal number of osteoblasts (FIGS. 3D and 3E). As expected given the existence of the hypogonadism, the number of osteoclasts was increased in both mutant mouse strains (FIG. 3F) suggesting that the high bone mass phenotype of the ob/ob and db/db mice may have developed despite an increase in bone resorption.

The specificity of the effect of the absence of leptin signaling on osteoblast function using the same groups of control animals as above was determined. One month-old ob/ob animals fed a low fat diet and heterozygote leptin-deficient mice, both of which were lean, also had a significant increase in their rate of bone formation (FIG. 3G). In contrast, Ar/a mutant mice as well as wild-type mice fed a high fat diet, two leptin-unrelated models of obesity, had normal bone formation parameters (FIG. 3H).

### 6.5. Analysis of Osteoclast Function in ob/ob Mice

The coexistence of a high bone mass phenotype and of hypogonadism was so exceptional that it raised the hypothesis that bone resorption might be defective in these mice. The increased urinary elimination of deoxypyridinoline crosslinks (Dpd), a biochemical marker of bone resorption (Eyre et al., 1988, Biochem 252, 494-500), in the ob/ob mice argued against this hypothesis (wild-type: 10.5 ± 2.5 nM Dpd/mM creatinine; ob/ob: 24.0 b 4.0 nM Dpd/mM creatinine). Deoxypyridinoline crosslinks were measured in morning urines using the Pyrilinks-D immunoassay kit (Metra Biosystem). Creatinine values were used for standardization between samples (Creatinine kit, Metra Biosystem). Circulating concentration of 17β-estradiol and leptin were quantified by radioimmunoassays, using the third generation estradiol kit (Diagnostic system laboratories) and the mouse leptin RIA kit (Linco), respectively. Estradiol is an estrogen analog and also a Selective Estrogen Receptor Modulator (SERM). Coadministration of an estrogen analog with an Ob or ObR inhibitor further increases the high bone mass phenotype.

Nevertheless, to address this point more thoroughly the hypogonadism of the ob/ob mice was exploited. Hypogonadism normally leads to an increase in osteoclasts number and in bone resorptive activity. Thus, if the osteoclasts of the ob/ob mice were functional, correcting the hypogonadism of these mice should decrease their rate of bone resorption by diminishing the number of osteoclasts and thereby should further increase their bone mass. On the other hand, if the osteoclasts of the ob/ob mice were not functioning properly, correcting their hypogonadism should not affect the severity of their high bone mass phenotype. To determine which of these two possibilities was correct, 17 β-estradiol or placebo pellets (Innovative Research of America) were implanted subcutaneously in 2-month-old female ob/ob mice to maintain a serum concentration of 250 pg/mL. These animals were analyzed after a 3-month treatment period.

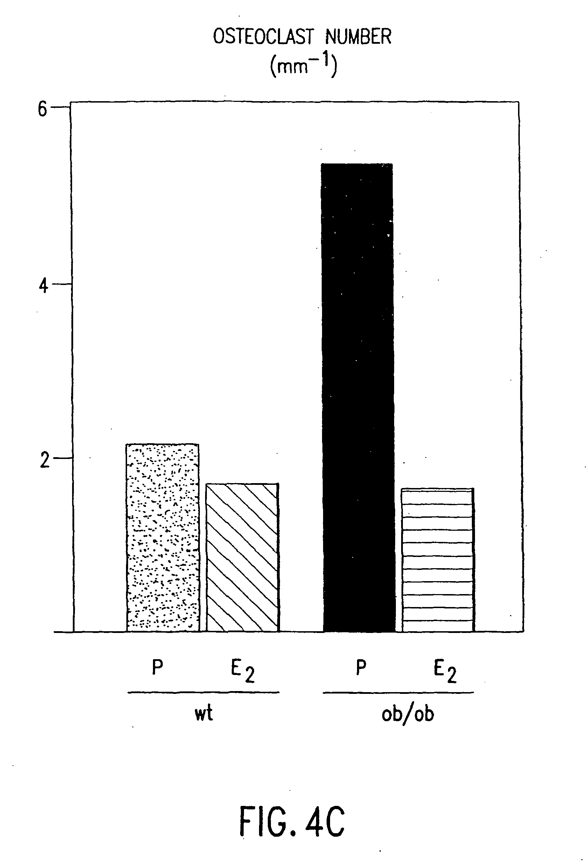

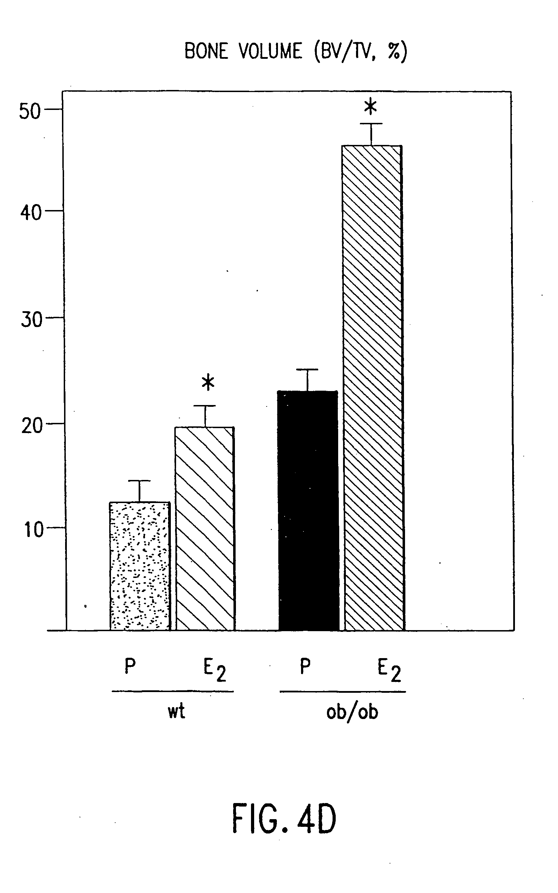

As expected, the estradiol treatment corrected their hypogonadism as judged by the aspect of their uteri and their levels of estradiol in blood (FIG. 4A), and furthermore resulted in a normalization of the osteoclast number (FIG. 4C). It also led to a further increase of the high bone mass phenotype of these mice compared to placebo treated ob/ob mice, thus eliminating a defect of bone resorption as the origin of the high bone mass phenotype in ob/ob mice (FIG. 4B). Estradiol-treated ob/ob mice had a 50% increase in bone volume compared to estradiol-treated wild-type mice and a 3-fold increase compared to untreated wild-type mice at the end of the treatment period (FIG. 4D). Similar results were obtained in male ob/ob mice treated with testosterone implants. Finally, the function of the osteoclasts of ob/ob and db/db mice was studied *in vitro* in an assay using hematopoietic progenitor cells from wild type, ob/ob, or db/db mice and growth factors known to induce the differentiation of these cells into functional osteoclasts. Mouse osteoclasts were generated *in vitro* according to protocols previously reported (Simonet et al., 1997, Cell 89, 309-319; Quinn et al., 1998, Endocrinology 139, 4424-4427). Bone-marrow cells of wt, ob/ob, and db/db mice were cultured at an initial density of 106 cells/well on 24-well tissue culture plates or dentin chips in α-MEM (Sigma) containing 10% FBS (Hicione), murine M-CSF 40 ng/mL (Sigma), RANKL/ODF 25 ng/mL (Peprotech), 10⁻⁸ M dexamethasone (Sigma) and 10⁻⁸ 1,25 dihydroxy vitamin D3. Medium was changed every other day. After 6 days of culture, cells were fixed in 3.7% formalin. Osteoclasts formed on plastic plates were stained for tartrate resistant acid phosphatase. Cells on dentin chips were removed by treatment with sodium hypochloride solution. Dentin chips were then stained with toluidine blue to visualize resorption pits.

As shown in FIG. 4E, wild-type, ob/ob, and db/db hematopoietic progenitor cells differentiated equally well into osteoclasts able to resorb a matrix.

Thus, these experiments demonstrate that there is no detectable functional defect of the osteoclasts in ob/ob and db/db mice and establish that the high bone mass phenotype of these mouse mutant strains results exclusively from the increase in bone formation secondary to the absence of leptin signaling.

### 6.6. Absence of Leptin Signaling in Osteoblasts

The previous analyses indicate that leptin is an inhibitor of osteoblastic bone formation. Moreover, the existence of a high bone mass phenotype in db/db mice demonstrates that leptin must bind to its known receptor to fulfill this function. In theory, leptin could either act directly on osteoblasts, indirectly through the release of a second factor present in fat, or by using a hypothalamic pathway as it does for the control of body weight. These three possible mechanisms of action were tested.

Expression of leptin in osteoblasts was studied in subconfluent primary osteoblast cultures from wild-type mice which were maintained for 4 days in 10% FBS mineralization medium and then subsequently switched to 0.5% for 2 days and replaced daily. Medium was replaced 2 hours before a 20 min treatment with 80 ng/mL leptin (Sigma) or 40 ng/mL Oncostatin M (R&D Diagnostics) or vehicle. RNA extractions were performed using Triazol (Gibco). Northern blots were performed with 15 µg of total RNA according to methods well known in the art.

Expression of leptin in osteoblasts could not be detected even after a long film exposure, indicating that an autocrine regulation was unlikely (FIG. 5A). Leptin expression could also not be detected in whole bone samples, providing an indirect argument against a paracrine regulation of osteoblast function by leptin (FIG. 5A). In any case, a paracrine and/or an endocrine regulation of osteoblast function by leptin would require that functional leptin receptors are present on osteoblasts. There are several transcripts of the leptin receptor, but only one, *Ob-Rb,* is thought to have signal transduction ability (Tartaglia et al., 1995, Cell 83, 1263-1271; Chen et al., 1996, Cell 84, 491-495; Lee et al., 1996, Nature 379, 632-635). The expression of this transcript of leptin receptor is highly, although not strictly, hypothalamus-specific. RT-PCR experiments were performed to search for *Ob-Rb* transcripts in primary osteoblasts and whole bone samples. RT-PCR analysis (27 cycles) of *Ob-Rb* expression was performed on random-primed cDNAs using the following primers: 5'-TGGATAAACC CTTGCTCTTCA-3' (SEQ ID NO: 17), and 5'-ACACTGTTAATTTCACACCAGAG-3' (SEQ ID NO: 18) (Friedman & Halaas, 1998, Nature 395, 763-770). Amplification of Hprt was used as an internal control for cDNA quality using the following primers:

5'-GTTGAGAGATCATCTCCACC-3' (SEQ ID NO: 19), and 5'-AGCGATGATG
AACCAGGTTA-3' (SEQ ID NO: 20).

In several experiments using a number of amplification cycles necessary to detect *Ob-*Rb transcripts in hypothalamus, there was no detection of *Ob-Rb* expression in calvaria, long bone, and primary osteoblast cultures (FIG. 5B).

To determine whether or not leptin could transduce its signal in osteoblasts, serum-starved primary osteoblasts isolated from wild-type mice were treated with leptin. Primary osteoblast cultures from calvaria of newborn wild-type or db/db mice were established as previously described (Ducy et al., 1999, Genes Dev 13, 1025-1036) and maintained in mineralization medium (αMEMI 0.1 mg/mL ascorbic acid; 5 mM α-glycerophosphate) supplemented with 10% PBS. Cultures of mutant cells were maintained in this medium for 15 days before analysis. Cultures derived from wild-type mice were maintained for 10 days in this medium then the percentage of serum was reduced to 0.5% and the medium supplemented with 1.2 µg/mL leptin (Sigma) or vehicle for 5 days.

The phosphorylation of Stat3, a downstream effector of leptin signaling in its target cells (Tartaglia et al., 1995, Cell 83, 1263-1271; Baumann et al., 1996, Proc Natl Acad Sci USA 93, 8374-8378; Ghilardi et al., 1996, Proc Natl Acad Sci USA 93, 6231-6235; Vaisse et al., 1996, Nat Genet 14, 95-97), was monitored, in addition to the expression of two immediate early genes whose transcription is increased following leptin treatment of target cells (Elmquist et al., 1997, Endocrinology 138, 839-842). As a positive control oncostatin-M that induces Stat3 phosphorylation and activates the expression of the same two immediate early genes in osteoblasts (Levy et al., 1996, Endocrinology 137, 1159-1165) was utilized. Western blot analysis was performed to determine Stat3 phosphorylation as follows. Cells were lysed, protein extracts were separated on a 7.5% SDS-PAGE and blotted on nitrocellulose (Biorad) for immunoblotting assay by methods well known in the art. Analysis of Stat3 phosphorylation was performed using the PhosphoPlus Stat3 (Tyr7O5) Antibody kit (New England Biolabs) according to the manufacturer's instructions.

As shown in FIG. 5C, treatment of osteoblasts with Oncostatin-M did induce Stat3 phosphorylation while leptin treatment did not. Several doses of leptin, physiologic and supraphysiologic, were used in this experiment, yet all of them failed to induce Stat3 phosphorylation. Similarly, expression of *Tis11* and *c-fos,* analyzed by standard Northern analysis methods, was quickly and transiently activated by oncostatin-M but not by leptin (FIG. 5D). Finally, the effect of a long-term leptin treatment of primary osteoblast cultures from wild-type mice on extracellular matrix synthesis and bone matrix mineralization was determined. The presence of a collagen-rich extracellular matrix and of mineralization nodules was assessed by whole-mount staining of the cultures by the van Gieson and von Kossa reagent, respectively. No difference was observed when assessing collagen synthesis or mineralization nodule formation between control and leptin-treated cultures (FIG. 5E).

Finally, if there is no functional leptin receptor on osteoblasts, then wild-type and db/db osteoblasts should be undistinguishable in *ex vivo* culture. Primary cultures of osteoblasts from db/db and wild-type mice were analyzed for their ability to generate a bona fide bone extracellular matrix and to mineralize it. For all the parameters analyzed, which were alkaline phosphatase staining, type I collagen production and formation of mineralization nodules, there was no difference between wild type and db/db primary osteoblast cultures (FIG. 5F). Taken together, these results indicate that leptin action on bone formation in the entire animal does not require leptin binding to a receptor located on the osteoblasts.

### 6.7. High Bone Mass in Absence of Fat Tissue

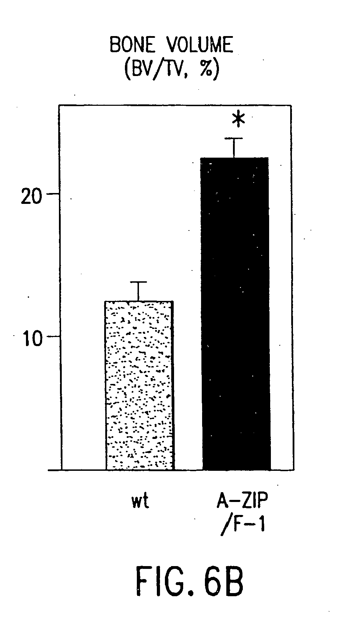

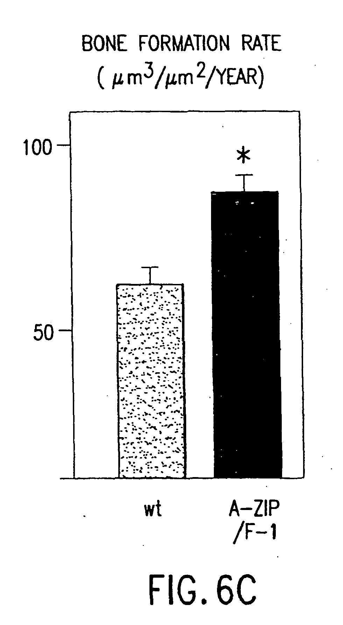

To address the possibility that this action of leptin could require the presence of fat, a transgenic mouse model expressing, exclusively in adipocytes, a dominant negative protein termed A-ZIP was utilized (Moitra et al., 1998, Genes Dev 12, 3168-3181). This dominant negative protein abolishes the DNA-binding ability of most B-ZIP transcription factors, a class of transcription factors critical for adipocyte differentiation. As a result the A-ZIP/F-1 transgenic mice have no white adipose tissue, which is the type of fat regulated by leptin signaling, and dramatically reduced amounts of inactive brown adipose tissue. They also have a 20-fold reduction in leptin synthesis (Moitra et al., 1998, Genes Dev 12, 3168-3181). In A-ZIP/F-I transgenic mice the same high bone mass phenotype due to an increase in osteoblast function was observed as is seen in ob/ob and db/db mice (FIG. 6).

This experiment has two implications. First, it confirms that leptin deficiency, not high fat index, is responsible for the high bone mass phenotype of the ob/ob and db/db mice. Second, it demonstrates that fat tissue is not a necessary relay for the action of leptin on bone formation.

### 6.8. Intracerebroventricular Infusion of Leptin Corrects the High Bone Mass Phenotype of the ob/ob Mice

Lastly, the issue of whether leptin binding to its hypothalamic receptor could correct the high bone mass phenotype of the ob/ob mice as it can rescue their obesity phenotype was addressed. To that end pumps delivering either PBS or leptin (8 ng/hr) in the third ventricle of ob/ob mice were inserted as follows. Animals were anesthetized with avertin and placed on a stereotaxic instrument (Stoelting). The Calabria was exposed and a 0.7 mm hole was drilled upon bregma. A 28-gauge cannula (Brain infusion kit U, Alza) was implanted into the third ventricle according to the following coordinates: midline, -0.3 AP, 3 mm ventral (0 point bregma). The cannula was secured to the skull with cyanoacrylate, and attached with Tygon tubing to an osmotic pump (Alza) placed in the dorsal subcutaneous space of the animal. The rate of delivery was 0.25 µl/hour (8 ng/hr of leptin (Sigma)) or PBS for 28 days. The dosage has been previously shown to have no effect when administered systemically (Halaas et al., 1997, Proc Nati Acad Sci USA 94:8878-8883). To be as close as possible to the biological situation of the ob/ob mice, the animals in which pumps were inserted were ovariectomized to avoid any artificial increase in bone mass due to the correction of their hypogonadism. The pumps were left in place for 28 days and double-labeling with calcein was performed to measure the bone formation parameters.

Classical histology showed that the bone of leptin-treated mice but not of the PBS-treated mice had regained a normal appearance (FIG. 7A). They had fewer trabeculae and these trabeculae looked more regular than in the PBS-treated ob/ob mice. Bone volume, trabeculae thickness and bone formation rates were all significantly decreased in leptin-treated mice (FIG. 7A). No leptin in the serum of these animals was detected using a specific radioimmunoassay. The rescue of the bone phenotype by leptin intracerebroventricular infusion, together with the absence of measurable circulating leptin, demonstrates that control of bone formation is a neuroendocrine leptin-dependent function.

### 7. EXAMPLE: RELATIONSHIP BETWEEN LEPTIN, BONE MASS, AND SYMPATHETIC TONE

Leptin inhibits bone formation and intracerebral ventricular (ICV) infusion of leptin in leptin-deficient (ob/ob) mice corrects their high bone mass (HBM) phenotype, revealing the central nature of this regulation. Cross-circulation between ob/ob mice followed by ICV infusion of leptin in one animal of each pair demonstrates that the efferent signal in this regulatory loop is not of humoral nature. Analysis of mutant mice deficient in melanocortin signaling, a mediator of leptin action on body weight, failed to identify any bone abnormalities, suggesting that other pathways mediate the bone formation regulating function of leptin. Leptin deficiency reduces activity of the sympathetic nervous system, and mice deficient in the enzyme necessary for catecholamine synthesis have a HBM phenotype. Moreover, treatment of ob/ob mice with a sympathomimetic agent corrects their HBM phenotype. This example describes the identification of the sympathetic nervous system as an effector of leptin regulation of bone formation and indicates that sympathetic nervous system modulators can be used in treating bone diseases.

Immunocytochemistry. Tibias from 3 day old C57BL6 mice were dissected and directly frozen in OCT compound. Twelve micron sections were cut with a Micron cryostat. The sections were fixed, blocked in 5% serum and incubated with primary antibody directed to β1, β2, and β3 adrenergic receptors (Santa Cruz Biotechnology). After washing, the antigen-antibody complex was detected by incubation with a secondary antibody coupled to peroxidase for visualization.

Cross Circulation (Parabiosis) Procedure. Following anesthesia by standard procedures, the surgery is made under adequate anesthesia and aseptic techniques. A longitudinal incision is made along one side on each mouse and skin is loosened from connective tissue. The ventral edges of the incision are joined by suture. Abdominal cavity of each mouse is opened and connected to make coelio anastomosis. Finally, the dorsal edges of the skin incision are joined. Parabiosed mice are housed in separate cages subsequently. Mice used for this procedure are syngenic to avoid immune reactions. Bone histology and histomorphometry are performed at the end of the treatment period to determine bone mass.

Isoproterenol, a beta agonist, was injected into 4 weeks old ob/ob mice at a dose of 30mg/kg i.p. per day. After 6 weeks treatment, the mice were dissected and the bone was analyzed by X-ray and histomorphometry, as described in Section 6.2.

*In vivo,* leptin inhibits bone formation by osteoblasts and as a result ob/ob, db/db and lipodystrophic mice and humans that all share absent or deficient leptin signaling have a high bone mass (HBM) phenotype regardless of their body weight. The HBM phenotype of the ob/ob and db/db mice coexists with hypogonadism and, to date, leptin deficiency is the only known condition resulting in the coexistence of high bone mass and hypogonadism. This last feature underlies the importance of this regulation in bone biology. Central infusion of leptin decreased bone mass and bone formation parameters in both leptin-deficient and wildtype mice, thereby revealing the existence of a central component in the regulation of bone formation.

To determine whether the signal(s) emanating from the brain and controlling bone formation was/were of humoral nature, cross circulation (parabiosis) experiments were performed between ob/ob mice. Four weeks after establishing and verifying the cross circulation, a pump delivering leptin ICV (8 ng/h) was installed in one mouse of each parabiosed pair. The absence of leakage through the blood brain barrier was verified by the absence of measurable leptin in the plasma of these animals. Body weight and bone histology of the ipsilateral and contralateral mouse of each pair was analyzed four weeks later, reasoning that if leptin was using humoral means to control body weight and bone mass, both mice in each pair should respond equally well to the leptin infusion. As previously shown, body weight and bone volume dropped considerably in the ob/ob mice receiving leptin ICV. In contrast, body weight or bone volume loss in the contralateral mouse of all the parabiosed pairs was never observed. These results suggest that the efferent signal(s) in the leptin-dependent loop controlling bone formation is/are not of humoral nature.

Leptin activation of pro-opiomelanocortin (POMC) neurons contribute to its function in regulating appetite and body weight. To determine whether POMC neurons contribute also to the bone formation regulating function of leptin, we studied lethal agouti (Ay) mice that have a defect in POMC signaling in the brain leading to late onset obesity and resistance to the anorexigenic function of leptin. Ay mice have abnormal bone mass and ICV infusion of leptin in these mice leads to a significant decrease of their bone mass without affecting their body weight. This result indicates that leptin can affect bone mass in the absence of melanocortin signaling. Next, mice deficient in melanocortin 4 receptor (MC4-R), a brain specific receptor for melanocortin, were studied. MC4-R-deficient mice that are obese and hyperinsulinemic have a normal bone mass and normal bone formation parameters at I and 3 month of age. Taken together, the analysis of these two mutant mouse strains indicate that POMC neurons are not a major relay in the regulation of bone formation by leptin, suggesting that other mediators are involved to control bone mass. The analysis of these mutant mice addressed also another aspect of the central regulation of bone formation. Indeed, a legitimate concern raised by the existence of a HBM phenotype in absence of leptin signaling was that it could be explained by the hyperinsulinism that is observed in these animals. The study of the Ay and MC-4R-deficient mice shows that it is unlikely to be the case since these two mutant mouse models are characterized by the existence of a severe hyperinsulinism and yet have a normal bone mass.

To determine whether sympathetic neurons were involved in regulating bone formation, mice deficient in dopamine β hydroxylase (DBH), the enzyme necessary for converting dopamine to norepinephrine were studied. As shown in FIG. 8, DBH-deficient mice have a HBM phenotype. Similarly to what is observed in the absence of leptin signaling, this phenotype become more severe over time and is secondary to an increase in bone formation parameters. Urinary excretion of deoxypridinoline, a biomarker of bone resorption, was normal in DBH-deficient mice indicating that a defect in bone resorption is causing their HBM phenotype.

### 8. EXAMPLE: NEURONAL REGULATION OF BONE FORMATION BY THE SYMPATHETIC NERVOUS SYSTEM

In this Example, leptin-dependent antiosteogenic hypothalamic networks show that neuropeptides mediating leptin anorexigenic function do not affect bone formation and that peripheral mediators of leptin antiosteogenic function may be neuronal. Leptin deficiency results in low sympathetic tone, genetic and pharmacologic ablation of adrenergic signaling in mice leads to a leptin-resistant high bone mass and β-adrenergic receptors on osteoblasts which regulate their proliferation and function. Accordingly, a β-adrenergic agonist decreases bone mass in leptin-deficient and wildtype mice whereas a β-adrenergic antagonist increases bone mass in wildtype and ovariectomized mice. None of these manipulations affects body weight. This study presented herein demonstrates a leptin-dependent neuronal regulation of bone formation that has therapeutic implications for osteoporosis.

### 8.1. Introduction

In vertebrates, bone mass is maintained constant through the interplay of 2 functions: bone resorption by osteoclasts and bone formation by osteoblasts. The concerted action of these 2 cell types defines bone remodeling. That osteoporosis, the most frequent bone remodeling disease, is also the most frequent degenerative disease in developed countries (see, *e.g.*, Cooper & Melton, 1996, In Osteoporosis, R. Marcus, D. Feldman, and J. Kelsey, eds., San Diego, Academic Press, pp. 419-434) explains why identifying molecular regulators of bone remodeling is such an important question of bone biology.

The precision of the recovery following inducible osteoblast ablation in adult mice led to the postulation of the existence of a systemic control of bone formation (see, *e.g.*, Corral et al., 1998, Proc Natl Acad Sci USA 95:13835-13840). The high incidence of osteoporosis following gonadal failure (see, *e.g*., Riggs et al., 1998, J Bone Miner Res 13:763-773) and its low incidence in obese people (see, e.g., Felson et al., 1993, J Bone Miner Res 8:567-573 and Tremollieres et al., 1993, J Clin Endocrinol Metab 77:683-686.) suggested a hypothesis whereby bone mass, body weight and reproduction would be controlled by the same hormone(s). Testing this hypothesis revealed that leptin, a hormone regulating body weight and gonadal function, is also a powerful inhibitor of bone formation, i.e. an antiosteogenic factor (see, *e.g.,* Ducy et al., 2000, Cell 100:197-207). Leptin-deficient (*ob*/*ob*), leptin receptor-deficient and lipodystrophic mice that have in common decreased leptin signaling have the same high bone mass (HBM) phenotype. These three mutant mouse strains, characterized by a great disparity in body weight, which displayed the same bone phenotype suggested that it is leptin signaling, not body weight, that controls bone mass. Infusion of leptin into the third ventricle (ICV) of *ob*/*ob* or wildtype (wt) mice decreased bone mass and bone formation parameters establishing the existence of a central component in the control of bone formation (see, *e.g.,* Ducy et al., 2000, Cell 100:197-207). These results are in agreement with the initial hypothesis since obese people that are protected from osteoporosis are resistant to leptin central action (see, *e.g.,* Ahima & Flier, 2000, Annu Rev Physiol 62:413-437). The importance of leptin antiosteogenic function is underscored by the fact that leptin deficiency is the only known condition resulting in the coexistence of HBM and hypogonadism, a condition that otherwise favors bone loss.

Much progress has been made in identifying mechanisms whereby leptin exerts its anorexigenic function. Chemical lesioning, molecular elucidation of mouse mutant strains and generation of neuropeptide-deficient mice have identified hypothalamic neurons synthesizing orexigenic or anorexigenic molecules that are targets of leptin anorexigenic action (see, *e.g.,* Elias et al., 1999, Neuron 23:775-786; Cowley et al., 2001, Nature 411:480-484; and DeFalco et al., 2001, Science 291:2608-2613). In contrast, the cellular and molecular bases of leptin antiosteogenic function remain unknown.

To decipher the bases of leptin antiosteogenic function, chemical lesioning, genetic, physiologic and molecular analyses were used. The studies presented in this Example suggest that the anorexigenic and antiosteogenic modes of leptin action are distinct, identify a neuronal regulation of bone formation and point toward a therapeutically useful way of manipulating this pathway.

### 8.2. Experimental procedures

### 8.2.1. Animals, treatments and surgical procedures

Wildtype (C57BL/6J) and mutant (C57BL/6J *A^{y}*/*a,* C57BL/6J *ob*/*ob*) mice were obtained from the Jackson laboratory and adrenal medullectomized mice from Harlan Tecklad laboratory. *Dbh-*/*-* mice were rescued as previously described (Thomas et al., 1998, Neurochem 70:2468-2476). Isoproterenol (Sigma) was injected intraperitoneally (ip) once daily for 6 weeks at 30 mg/kg (wt) or 3 mg/kg (*ob*/*ob*). Propranolol (Sigma) was added to the drinking water at a concentration of 0.5g/l. For ICV infusion, a 28-gauge cannula (Brain infusion kit II, Alza) was implanted into the third ventricle as previously described infusing human leptin (Sigma) at 8ng/hr or MT-II at 125ng/hr (Phoenix Pharmaceuticals) for 28 days (see, *e.g.,* Ducy et al., 2000, Cell 100:197-207). The cannula was connected to an osmotic pump (Alza) placed in the dorsal subcutaneous space of the animal. For parabiosis longitudinal incisions were made in the skin and in the peritoneal cavity along one side on each mouse. Edges of the incisions were connected by suture to induce coelioanastomosis. Cross-circulation was quantified by injecting 1 ml/kg 0.25% Evans blue into the tail vein of one mouse of the parabiosed pair. After 30 min. blood was collected from both mice by retroorbital bleeding and the blood exchange rate was calculated according to standard techniques (see, *e.g.,* Harris & Martin, 1984, Am J Physiol 247:R380-386). All parabiosed pairs had an hourly exchange rate above 2%. Two weeks after parabiosis a pump delivering leptin ICV in one animal of each pair was implanted. For GTG lesioning, 4-week-old C57BL/6J mice were given a single ip injection of either PBS or GTG (0.5mg/g). For MSG lesioning, 2 day-old C57BL/6J pups were injected daily subcutaneously with either PBS or MSG (2 mg/g) for 10 days.

### 8.2.2. Generation of transgenic mice and molecular studies

The_*α1 (I) leptin* transgene was generated by cloning the mouse leptin cDNA downstream of the 2.3kb osteoblast-specific fragment of the *al(I)collagen* promoter. Transgenic founders were generated by standard techniques (see, e.g., Ausubel, 1995, Current Protocols in Molecular Biology, New York). Genotypes were determined by PCR. Progenies of two lines expressing the transgene were analyzed. Northern blot analyses were performed using total RNA or polyA+ RNA according to standard protocols. RT-PCR analysis of adrenergic receptor expression was performed on random-primed cDNA for 27 cycles.

### 8.2.3. Histological procedures, immunocytochemistry and proliferation

Specimens were embedded in paraffin and sectioned at 6 µm. Brains were stained with 0.1% cresyl violet using standard procedures. Immunohistochemistry was performed according to standard protocols (see, e.g., Ausubel, 1995, Current Protocols in Molecular Biology, New York). *In vivo* osteoblast proliferation assays were performed on newborn mice treated daily for 5 days with 40 µg isoproterenol, 4 µg dexamethasone or vehicle. BrdU (0.4mg) was injected ip at day 5, mice were sacrificed 2 hours later. BrdU incorporation was detected by immunohistochemistry using Zymed BrdU staining kit (Zymed Laboratories Inc.). Four pups per treatment were analyzed and 10 calvariae sections were counted per animal. NovaRED was used as a chromogenic peroxidase substrate. Sections were counterstained with hematoxylin.

Histological analyses were performed on undecalcified sections stained by von Kossa and counterstained by von Gieson (see, *e.g.,* Ducy et al., 2000, Cell 100:197-207). Static and dynamic histomorphometric analyses were performed according to standard protocols (see, *e*.*g*., Parfitt et al., 1987, J Bone Miner Res 6:595- 610) using the Osteomeasure Analysis System (Osteometrics, Atlanta). Six to 12 animals were analyzed for each group. Statistical significance was assessed by Student's t test.

### 8.2.4. Cell cultures and bioassays

Primary osteoblast cultures were established as previously described (see, *e.g.,* Ducy et al., 2000, Cell 100:197-207) and maintained in αMEM/0.1 mg/ml ascorbic acid supplemented with 10% FBS. SaOS-2 were grown in MEM/10% FBS. For cAMP assay confluent cultures were incubated with serum free medium containing 100 µM IBMX (3-isobutyl-1-methylxanthine, Sigma) for 8 minutes before PBS, PTH(1-34) (Bachem), isoproterenol, norepinephrine, phenylephrine (Sigma) or propranolol were added for 5 min. Intracellular cAMP concentration was measured by immunoassay (R&D Systems). For gene expression analyses, primary osteoblasts were treated for 72 hours with appropriate drugs in 0.1% FBS. Hormone serum levels were quantified using immunoassays kits from Peninsula Laboratories (Insulin) or Alpco Diagnostics (leptin). Deoxypyridinoline cross-links and Creatinine were measured in morning urines using the Quidel kits. α1(I) leptin bioactivity was verified by cotransfection of 293 cells expressing *ObRb* with a STAT3-responsive-luc reporter construct, pSVβgal plasmid and α1(I) leptin expression vector or mock. Twenty-four hours later luciferase and β-galactosidase activities were measured. Data represents ratios of luciferase/β-galactosidase activities, and values are mean of 6 independent transfection experiments.

### 8.3. Results

### 8.3.1. Identification of hypothalamic antiosteogenic areas

Two hypothalamic nuclei, the ventromedial hypothalamic nucleus (VMH) and arcuate nucleus (ARC) have the highest density of neurons expressing *ObRb,* the signal transducing form of the leptin receptor; both nuclei play a critical role in mediating leptin anorexigenic function (see, *e.g.,* Tartaglia et al., 1995, Cell 83:1263-1271 and Fei et al., 1997, Proc Natl Acad Sci U S A 94:7001-7005.). The potential involvement of neurons of these nuclei in leptin antiosteogenic function was first assessed by chemical lesion followed or not by leptin ICV infusion.

Newborn pups were first treated with monosodium glutamate (MSG), which damages circumventricular neurons expressing the glutamate receptor (see, *e.g.,* Olney, 1969, Science 164:719-721). MSG treatment markedly affected ARC structures as illustrated by the near absence of neurons synthesizing neuropeptide Y (NPY) (FIG. 9A). In contrast, the preserved expression of steroidogenic factor 1 (SF1), a marker of VMH neurons (see, *e.g.,* Ikeda et al., 1995, Mol Endocrinol 9:478-486 and Dellovade et al., 2000, J Comp Neurol 423:579-589), indicated that the majority of these neurons were not affected by MSG treatment. No lesions outside the hypothalamus were observed. Twelve week-old MSG-treated mice had a normal bone mass as determined histologically by measurement of their bone volume (FIG. 9B). To further address the role of MSG-sensitive neurons in leptin antiosteogenic function, a leptin ICV infusion was performed in *ob*/*ob* mice treated with MSG. MSG treatment blocked the ability of leptin ICV infusion to decrease body weight but not to decrease bone mass (FIG. 9C). The normal bone mass of MSG-treated mice along with the efficacy of leptin ICV infusion despite MSG-induced lesions in *ob*/*ob* mice indicates that MSG-sensitive neurons are dispensable for leptin antiosteogenic action.

Next, 4-week-old wt mice were treated with gold thioglucose (GTG), a compound that destroys neurons of the VMH and of the more dorso-lateral part of the ARC although it affects other neurons (see, *e.g.,* Debons et al., 1962, Am J Physiol 4:743-750). In each mouse analyzed the deleterious effect of GTG treatment on VMH was revealed by a dense scar distorting the anatomy of the ventral hypothalamus. Immunohistochemical studies in GTG-treated mice showed a marked perturbation of VMH neurons expressing SF1 whereas NPY-expressing neurons located in the ARC were spared (FIG. 9D). These observations suggest that GTG and MSG differentially affected neurons of the ARC and VMH. Bone histologic analysis revealed that 12 week-old GTG-treated mice displayed a HBM whose severity was nearly identical to that of *ob*/*ob* mice (FIG. 9E). As with *ob*/*ob* mice, this HBM was due to an increase in bone formation defined by an increase in the bone formation rate (FIG. 9E). Urinary elimination of deoxypyridinoline (dpd), a collagen breakdown product indicative of osteoclast activity, and osteoclast numbers were normal indicating that bone resorption was not overtly affected by GTG (data not shown). These findings established that neurons sensitive to GTG are involved in the control of bone mass.

To determine whether GTG-sensitive neurons were implicated in leptin antiosteogenic function, a leptin ICV infusion was performed in GTG-treated *ob*/*ob* mice. Leptin ICV infusion decreased body weight of these animals indicating that it was effective (FIG. 9F). However and despite the extreme sensitivity of *ob*/*ob* mice to leptin, this infusion failed to decrease their bone mass (FIG. 9F). Taken together, these data indicate that GTG-sensitive neuronal networks are necessary for leptin antiosteogenic function. Moreover, the observation that GTG or MSG treatment affected differentially leptin antiosteogenic and anorexigenic functions suggests that these two functions are executed, at least partly, by distinct neuronal pathways.

### 8.3.2. Leptin antiosteogenic function does not require known anorexigenic neuropeptides

To go beyond chemical lesions a genetic approach was used. It has been demonstrated that binding of α-melanocyte stimulating hormone (αMSH), produced by ARC neurons, to neurons expressing melanocortin 4 receptor (MC4-R) and melanocortin 3 receptor (MC3-R) is required for leptin anorexigenic function (see, *e.g.,* Huszar et al., 1997; Vaisse et al., 1998; Yeo et al., 1998; Cowley et al., 2001, Nature 411:480-484). To assess the role of melanocortin signaling in leptin antiosteogenic function mutant mouse strains were analyzed with disrupted melanocortin signaling and *ob*/*ob* mice treated with a melanocortin receptor agonist.

*A^{y}*/*a* mice have decreased melanocortin signaling due to the binding to melanocortin receptors of the agouti protein, a competitive antagonist of melanocortin receptor signaling with a high affinity for MC4-R (see, e.g., Miller et al., 1993; Lu et al., 1994; Fan et al., 1997). As a result *A^{y}*/*a* mice develop a late-onset obesity accompanied by a central resistance to leptin anorexigenic function (see, e.g., Halaas et al., 1997, Proc Natl Acad Sci U S A 94:8878-8883) that resembles the phenotype observed in *Mc4*-*r*-deficient mice (see, *e.g.,* Huszar et al., 1997, Cell 88:131-141). In contrast, multiple lines of evidence indicate that leptin antiosteogenic function does take place when melanocortin signaling is disrupted. First, *A^{y}*/*α* mice have a normal bone mass (see, *e.g.,* Ducy et al., 2000, Cell 100:197-207). Second, *A^{y}*/*a* mice are not resistant to leptin antiosteogenic function as long-term ICV leptin infusion decreased bone volume comparably in *A^{y}*/*a* mice and wt mice, secondary to a decrease in the bone formation rate (FIG. 10A). Third, *Mc4*-*r*-deficient mice have a normal bone mass (FIG. 10B). Fourth, ICV infusion of MTII, a MC4-R/MC3-R agonist (Fan et al., 1997) in *ob*/*ob* mice, did not affect their bone mass while it significantly decreased their body weight (FIGS. 10C and 10D).
Another anorexigenic polypeptide whose expression is regulated by leptin is cocaine amphetamine related transcript (CART) (see, e.g., Kristensen et al., 1998, Nature 393:72-76). Although CART regulates body weight in mice (see, *e.g.,* Asnicar et al., 2001, Endocrinology 142:4394-4400), *Cart*-deficient mice did not display HBM (data not shown). Taken together these experiments indicate that melanocortin and CART signaling pathways, which are critical for leptin anorexigenic action, are not required for its antiosteogenic function. These results are consistent with the observation that MSG-sensitive neurons, which are the main hypothalamic source of αMSH, are dispensable for leptin antiosteogenic action.

The analysis of Mc4-r-deficient mice addressed another concern that could not be studied in mice deficient in leptin signaling. The HBM observed in absence of leptin signaling raised the possibility that it could be secondary to the hyperinsulinism created by this condition. The normal bone mass in Mc4-r-deficient mice despite their elevated plasma insulin levels argues that hyperinsulinism does not lead to HBM (Table1). Three other lines of evidence presented below further dissociate plasma insulin levels and bone mass regulation.

### 8.3.3. Peripheral mediation of leptin antiosteogenic function

The next question was whether the signal(s) emanating from the hypothalamic antiosteogenic network was/were of humoral or of neuronal nature. To that end, cross-circulation (parabiosis) experiments between *ob*/*ob* animals (that have no circulating leptin) were relied upon. Parabiosis experiments were performed as described in Experimental procedures and dye injection confirmed effective cross-circulation with >95% equilibrium between the parabiotic animals after 2 hours. Two weeks after parabiosis, in each parabiosed pair, a pump infusing leptin ICV was implanted. The absence of measurable leptin in serum of all the animals analyzed ruled out a leakage of leptin into the general circulation (data not shown). Four weeks later the animals were sacrificed and analyzed. As expected, bone mass dropped significantly in the *ob*/*ob* mouse receiving leptin ICV; in contrast, no modification of bone mass was observed in the contralateral mouse (FIG. 11A). Although it does not rule out the existence of a short-lived humoral mediator, this experiment raised the possibility of a neuronal mediation of leptin antiosteogenic function.

None of the experiments presented above excluded the possibility that leptin could also affect osteoblast function by acting locally. To test this hypothesis, two transgenic mouse lines were generated using the osteoblast-specific fragment of the *α1(I) collagen* promoter (see, *e.g.,* Rossert et al., 1995, J Cell Biol 129:1421-1432.) to drive *Leptin* expression in osteoblasts [*α1(I)-leptin]* (FIG. 11B). Northern blot analysis and immunocytochemistry demonstrated a high level of leptin synthesis by osteoblasts (FIGS. 11B, 11C) that resulted in a slight increase in plasma leptin levels although it remained within the normal range (wt: 3.2ng/ml± 0.4 vs *α1*(*I*) leptin: 5.6±0.8, n=7 per genotype). The bioactivity of leptin transcribed by this transgene was established by showing that following DNA transfection leptin increased the activity of a Stat3-dependent luciferase reporter construct in 293 cells expressing *ObRb* (FIG. 11D). Despite this high local level of bioactive leptin the bone mass of the transgenic mice was undistinguishable from that of wt mice at any age including 1 year (FIG. 11E). This result indicates that the primary basis of leptin antiosteogenic function is not a direct action on osteoblasts.

### 8.3.4. Sympathetic regulation of bone formation

A well-characterized consequence of leptin deficiency is a reduced activity of the sympathetic nervous system (SNS) (see, *e.g.,* Bray & York, 1998, Recent Prog Horm Res 53, 95-117). Moreover, it has been proposed that the VMH mediates leptin-induced increase in catecholamine secretion (see, *e.g*., Ruffin & Nicolaidis, 1999, Brain Res 846:23-29 and Satoh et al., 1999, Diabetes 48:1787-1793). These observations as well as the findings presented herein led to the exploration of the role of the SNS in the control of bone formation.

As SNS function is mediated through adrenergic receptors, mutant mice deficient in dopamine β-hydroxylase (DBH), an enzyme necessary to produce norepinephrine and epinephrine, the catecholamine ligands for adrenergic receptors, were studied. Histologic examination revealed the existence of a HBM in *Dbh*-deficient mice albeit less severe than the one observed in *ob*/*ob* mice (FIG. 12A). This finding was significant since *Dbh*-deficient mice have an increase in serum corticosterone and in dopamine levels (see, e.g., Alaniz et al., 1999, Proc Natl Acad Sci U S A 96:2274-2278), two conditions favoring low bone mass (see, *e.g*., Adachi et al., 1993, Semin Arthritis Rheum 22:375-384; Alaniz et al., 1999, Proc Natl Acad Sci U S A 96:2274-2278; and *et al.,* 2000, Bone 26:15-19). This HBM was secondary to an increase in the bone formation rate and in the number of osteoblasts while markers of bone resorption were normal (FIGS. 12A-12D and data not shown). The HBM observed in *Dbh*-deficient mice was not associated with hyperinsulinism or with other hormonal perturbations besides the high corticosterone level (Table 1 and data not shown). Catecholamines are released from two main sources: the sympathetic nerves and the adrenal glands. To determine whether the adrenal production of catecholamines is involved in the regulation of bone mass, wt mice in which the adrenal medulla, the main source of circulating epinephrine (see, e.g., Young & Landsberg, 1998, The Adrenal. In Williams Textbook of Endocrinology, J. D. Wilson, D. W. Foster, H. Kronenberg, and P. R. Larsen, eds. (Philadelphia, W. B. Saunders Co.), pp. 665-682), had been surgically removed 4 weeks previously were analyzed. Histologic analysis showed that removal of the adrenal medulla did not affect bone mass (FIG. 12E). These results establish the existence of a neuronal regulation of bone formation.

It was next asked whether there was a direct link between leptin central antiosteogenic function and the SNS regulation of bone formation. To that end, leptin ICV infusion in *Dbh-*deficient mice was performed. This infusion led to a near disappearance of the gonadal fat pad in all *Dbh*-deficient mice treated indicating that centrally delivered leptin can affect body weight regulation in absence of norepinephrine and epinephrine (FIG. 12F). However, leptin failed to decrease bone mass of *Dbh*-deficient mice (FIG. 12G) demonstrating that leptin antiosteogenic function is dependent on a functional SNS.

**Table 1. Serum Insulin Levels and Bone Mass**

| | Insulin (ng/ml) | Bone Volume |
|---|---|---|
| Wild-type | 0.8 ± 0.3 | Normal |
| MC4-R*^{-l-}* | 23.0 ± 6.0* | Normal |
| Dbh*^{-l-}* | 1.0 ± 0.4 | High |
| ob/ob | 19.6 ± 0.8 | High |
| ob/ob + isoproterenol | 4.4 ± 0.4 | Low |
| Wild-type + propranalol | 0.8 ± 0.1 | High |

| | | |
|---|---|---|
| * (Huszar et al., 1997, Cell 88:131-141) | | |

### 8.3.5. Functional adrenergic receptors on osteoblasts

For a sympathetic regulation of bone formation to exist several requirements have to be fulfilled. The first one is that functional adrenergic receptors are present on osteoblasts. Gene expression analysis by RT-PCR and Northern blot showed the presence of β₂ adrenergic receptor transcripts, but of no other transcripts for adrenergic receptors, in primary mouse osteoblast cultures (FIG. 13A). Immunohistochemical analysis of long bones from transgenic mice expressing *LacZ* under the control of the osteoblast-specific fragment of the *α1(I) collagen* promoter verified the presence of β2-adrenergic receptors on osteoblasts (FIG. 13B). No other adrenergic receptor subtype could be detected. Moreover, axons immunoreactive with anti-neurofilament and anti-tyrosine hydroxylase antibodies were observed in the vicinity of osteoblasts (FIGS. 13C and 13D). Electron micrographs confirmed the presence of unmyelinated peripheral nerve axons coursing through the marrow adjacent to bone trabeculae and to osteoblasts (FIG. 13E). β-adrenergic receptors are G-coupled receptors that signal through the cAMP pathway (see, *e.g.*, Benovic et al., 1988, Annu Rev Cell Biol 4:405-428). Thus, to assess the biologic relevance of the presence of β₂-adrenergic receptors on osteoblasts, these cells were treated with isoproterenol, a β-adrenergic agonist, alone or in the presence of propranolol, a β-adrenergic receptor antagonist; with norepinephrine, the natural ligand of β-adrenergic receptors; with phenylephrine, an α-adrenergic agonist or with vehicle and measured cAMP production. As a positive control, parathyroid hormone (PTH) that binds to another G-coupled receptor present in osteoblasts was used (Gardella & Juppner, 2001, Trends Endocrinol Metab 12:210-217.). Isoproterenol or norepinephrine but not phenylephrine treatment increased cAMP production to a similar extent as PTH. These effects were abolished by propranolol treatment. The same results were obtained using mouse and human osteoblasts (FIG. 13F).

### 8.3.6. Decreased bone mass but persistent obesity in sympathomimetic-treated ob/ob mice

A second requirement is that treatment of *ob*/*ob* mice with sympathomimetic agents should decrease their bone mass. To address this point one month-old *ob*/*ob* mice were treated for 6 weeks with the β-adrenergic agonist isoproterenol or vehicle. As shown in FIG. 14A long-term isoproterenol treatment (3 mg/kg/day) resulted in a massive bone loss in the vertebrae and long bones of *ob*/*ob* mice. This was secondary to a marked decrease in the bone formation rate and in the number of osteoblasts per bone surface (FIG. 14B) while bone resorption parameters were unaffected (data not shown). Identical results were obtained when using 10 mg/kg/day of isoproterenol (data not shown). Two aspects of this experiment are of particular importance. First, this low bone mass developed while *ob*/*ob* mice remained hyperinsulinemic further dissociating hyperinsulinism and bone mass regulation (Table 1). Second, at these two doses isoproterenol decreased bone mass without affecting body weight demonstrating the existence of a range of doses in which isoproterenol affects selectively bone mass (FIGS. 14A and 14C). To determine the role of the SNS in animals that have none of the metabolic and neurological abnormalities caused by leptin deficiency, this experiment was repeated in wt mice. Isoproterenol again significantly decreased bone mass, bone formation rate, and osteoblast number without affecting their body weight (FIGS. 14D-14F). The increased expression of *uncoupling protein 1 (Ucp1)* in brown adipose tissue indicated that isoproterenol mimicked an increase in sympathetic activity in both *ob*/*ob* and wt mice (Scarpace & Matheny, 1998, Am J Physiol 275:E259-264) (FIG. 14G). Taken together these data identify the SNS, acting through β₂ adrenergic receptors, as a regulator of bone formation independently of the effect it may have on body weight.

To elucidate the bases of the antiosteogenic effect of isoproterenol *in vivo,* osteoblast proliferation, gene expression and apoptosis were studied. Following bromodeoxyuridine (BrdU) labeling *in vivo,* calvariae of isoproterenol-treated wt mice showed a nearly two-fold reduction in the number of positive cells compared to calvariae of control mice indicating that isoproterenol reduces osteoblast proliferation (FIG. 14H). As a control, dexamethasone that severely reduced osteoblast proliferation was used. Osteoblast gene expression studies revealed that isoproterenol treatment decreased the expression of *Cbfa1,* a transcription factor controlling osteoblast function (see, e.g., Ducy et al., 1999, Genes Dev 13:1025-1036) and *α1(I) collagen,* a gene encoding the main component of the bone extracellular matrix (FIG. 14I). Treatment with propranolol, a β-adrenergic antagonist, reversed the inhibitory effect of isoproterenol on gene expression (FIG. 14I). To study apoptosis, *Caspase* 3 expression and Annexin-V staining levels were examined and did not observe any change in isoproterenol-treated versus control osteoblasts (data not shown). These results indicate that the antiosteogenic function of the SNS is secondary to an inhibition of osteoblast proliferation and function.

### 8.3.7. A β-adrenergic antagonist increases bone mass in wt and ovariectomized mice

The most important biomedical requirement for regulation of bone formation by the SNS is that β-adrenergic antagonists (β blockers) should increase bone mass by increasing bone formation. To address this point, wt mice were treated for 5 weeks with propranolol (0.4mg/day). This treatment resulted in a significant increase in bone mass in vertebrae and long bones (FIG. 15A). In some propranolol-treated mice the bone volume matched what is observed in *ob*/*ob* mice. This increase in bone mass was secondary to an increase of both the bone formation rate and the number of osteoblasts (FIG. 15B). The change in bone mass induced by propranolol occurred while the body weight and fat pad weight of the animals remained normal (FIG. 15C and data not shown). Propranolol did not cause hyperinsulinism or other hormonal modifications (Table 1 and data not shown). Next, a possible link between leptin antiosteogenic function and the effect of β blocker on bone mass was examined. To that end, a leptin ICV infusion in propranolol-treated mice was done. Centrally delivered leptin led to a disappearance in fat pads of propranolol treated mice but did not affect their bone mass (FIGS. 15D, 15E). This experiment is a second argument supporting that leptin antiosteogenic function requires sympathetic activity.

This effect of propranolol on bone formation in wt mice suggested that it might mitigate the osteoporosis observed following estrogen depletion. To test if it was the case ovariectomy in 6 week-old wt mice was performed and the mice were treated for 7 weeks with propranolol or vehicle. Estrogen depletion by ovariectomy decreased bone mass in vehicle-treated mice. In contrast propranolol-treated ovariectomized mice had a normal bone mass (FIG. 15F). This preventative effect of propranolol treatment was due to a striking increase in the bone formation rate and in the osteoblast number that were both significantly higher than in control ovariectomized mice (FIG. 15G). This latter result underscores the physiologic and therapeutic importance of the sympathetic regulation of bone formation.

### 8.4. Discussion

Neuronal pathways required for leptin antiosteogenic function have been identifed and it has been demonstrated that the SNS is a negative regulator of bone formation. This inhibitory role of the SNS on bone formation could be modulated without affecting body weight. Besides providing a molecular basis for leptin antiosteogenic function, these findings establish the neuronal regulation of bone remodeling. The finding that a β-adrenergic antagonist could overcome the deleterious effect of ovariectomy on bone without affecting body weight has major implications for treatment of osteoporosis.

### 8.4.1. Specificity of leptin antiosteogenic function

These results demonstrate the importance of the hypothalamus in leptin antiosteogenic function and implies, with all the inherent limitations of lesioning studies, the VMH as a site of leptin action in this pathway. However, these chemical lesioning studies do not identify precisely subgroups of neurons in the hypothalamus required for leptin antiosteogenic function nor do they rule out that other neuronal populations elsewhere in the brain may be involved in this function. To address rigorously these issues further studies using region-specific inactivation of the leptin receptor in the brain will be needed (see, *e.g.,* Cohen et al., 2001, J. Clin Invest108: 1113-1121).

Genetically modified mouse strains were used to address the specificity of leptin antiosteogenic function. One neuropeptide implicated in leptin anorexigenic function is αMSH which is produced by ARC neurons and that binds to the melanocortin receptors. This signaling pathway is disrupted in *A^{y}*/*a* and *Mc4-r*-deficient mice that do not display bone mass abnormality. Moreover, *A^{y}*/*a* mice are not resistant to leptin antiosteogenic function whereas treatment of *ob*/*ob* mice with a melanocortin receptor agonist decreased their body weight without affecting their bone mass. These results, indicating that melanocortin agonists are not major regulators of bone formation, are consistent with the absence of an overt effect of MSG-induced damage on leptin antiosteogenic function. CART is another anorexigenic neuropeptide whose expression in the hypothalamus is regulated by leptin (see, *e.g*., Kristensen et al., 1998, Nature 393:72-76). *Cart*-deficient mice develop, on a high fat diet, a mild obesity (see, *e.g.*, Asnicar et al., 2001, Endocrinology 142:4394-4400) yet they do not have a HBM. The ability to differentially modulate the anorexigenic and the antiosteogenic effect of leptin within the hypothalamus as observed in MSG or GTG-treated mice and in mutant mouse strains suggests that these two functions are executed at least in part by distinct neuronal populations.

### 8.4.2. Sympathetic activity and regulation of bone formation

Several lines of evidence, including parabiosis experiments, analysis of *Dbh*-deficient mice, and treatment of *ob*/*ob,* wild type or ovariectomized mice with sympathetic agonists or antagonists establish that the SNS is a major regulator of bone formation. This anabolic effect of β blocker on bone formation could account for their beneficial effects in bone fracture in rats (see, *e.g.*, Minkowitz et al., 1991, J Orthop Res 9:869-875). In these experiments, the effects of the SNS on bone mass are independent of changes in body or fat weight. In addition, the failure of leptin ICV infusion to decrease bone mass in *Dbh*-deficient and in propranolol-treated mice uncovers a functional link between leptin antiosteogenic function and sympathetic activity.

Other mouse genetic observations also support the existence of a sympathetic regulation of bone formation. Dopamine transporter deficient-mice, in which rapid uptake of dopamine into presynaptic terminals does not occur, are osteopenic (see, *e.g*., Bliziotes et al., 2000, Bone 26:15-19). This latter observation suggests that the increased level of circulating dopamine observed in *Dbh*-deficient mice (see, *e.g.*, Alaniz et al., 1999; Proc Natl Acad Sci U S A 96:2274-2278) may have limited rather than created or amplified their bone phenotype. The cross-circulation experiments in parabiosed mice do not exclude the possibility of a short-lived humoral mediator of leptin antiosteogenic function. However, the result of the adrenalectomy experiment would argue against such a mechanism. Likewise, the low bone mass of panhypopituitarism patients does not support this notion (see, e.g., Kaufman et al., 1992, J Clin Endocrinol Metab 74:118-123).

### 8.4.3. Leptin as a master hormone

The pleiotropic functions of leptin that include control of body weight, reproduction, and bone formation are reminiscent of the many functions of hormones such as cortisol, estrogen, thyroid hormone and insulin. By analogy with the notion that master genes orchestrate cell differentiation programs during development it was proposed that leptin along with these other hormones defines a group of master hormones involved in the coordinated control of important homeostatic functions. These multiple functions of master hormones may make use of different modes of action. Indeed, analysis of glucocorticoid receptor mutants has revealed that cortisol, the hormone binding to this receptor, uses different pathways to achieve different functions (see, *e.g.,* Reichardt et al., 1998, Cell 93:531-541 and Karst et al., 2000, Nat Neurosci 3:977-978). The evidence presented here distinguishing its anorexigenic and antiosteogenic mode of action suggests that the same may be true in the case of leptin. The ability of master hormones to use different pathways to fulfill multiple functions in vertebrates may have been a way to integrate the increasing complexity of homeostasis in large animals during evolution.

### 8.4.3. Biomedical implications

One line of evidence indicates that a sympathetic regulation of bone mass exists in human and plays an important role. Reflex sympathetic dystrophy is a human disease characterized by manifestations of hyperadrenergic activity whose manifestations include osteoporosis. Treatment of patients affected by this disease with P blockers corrects most manifestations including the osteoporosis (see, e.g., Schwartzman, 2000, N Engl J Med 343:654-656). The existence of an osteoporosis in a human disorder characterized by excessive sympathetic activity and the disappearance of the osteoporosis with β blockers treatment is entirely consistent with the findings presented in this example. Although many drugs can effectively stop bone destruction in this disease, there is a need for drugs that would enhance bone formation. The observation presented herein that propranolol, a widely used drug with no major deleterious effects, can significantly increase bone formation and bone mass without affecting body weight provides evidence that β adrenergic antagonists, or derivatives thereof, may be used to treat osteoporosis.

All patents and other publications mentioned in the specifications are indicative of the levels of those skilled in the art to which the invention pertains. All patents and other publications are herein incorporated by reference to the same extent as if each individual publication was specifically and individually indicated to be incorporated by reference.

One skilled in the art readily appreciates that the patent invention is well adapted to carry out the objectives and obtain the ends and advantages mentioned as well as those inherent therein. Leptin, leptin receptor, leptin antibodies, leptin analogs, leptin agonists and antagonists, agonists and antagonists, pharmaceutical compositions, treatments, methods, procedures and techniques described herein are presently representative of the preferred embodiments and are intended to be exemplary and are not intended as limitations of the scope. Changes therein and other uses will occur to those skilled in the art which are encompassed within the spirit of the invention or defined by the scope of the pending claims.

## Claims

1. Use of a β adrenergic antagonist and a leptin antagonist for the preparation of a pharmaceutical composition for treating or preventing a symptom of osteoporosis.

2. The use of claim 1, wherein the leptin antagonist is selected from the group consisting of acetylphenol, an antibody that binds leptin, and an antibody that binds leptin receptor.

3. The use of claim 1, wherein the β adrenergic antagonist is selected from the group consisting of β₁, β₂ and β₃ antagonist.

4. Use of a composition that alters sympathetic tone in a mammal for the preparation of a pharmaceutical composition for the modulation of leptin effects on bone, for the modulation of bone mass or for treating or preventing a symptom of bone disease, wherein the pharmaceutical composition comprises a combination of a leptin antagonist and a β adrenergic antagonist.

5. A pharmaceutical composition for use in a mammal comprising a therapeutically effective amount of a β adrenergic antagonist and a leptin antagonist.

6. The composition of claim 5, further comprising a pharmaceutically acceptable carrier.

7. A β adrenergic antagonist and a leptin antagonist for use in treating or preventing a symptom of osteoporosis.

8. The β adrenergic antagonist and a leptin antagonist of claim 7, wherein the leptin antagonist is selected from the group consisting of acetylphenol, an antibody that binds leptin, and an antibody that binds leptin receptor.

9. The β adrenergic antagonist and a leptin antagonist of claim 7, wherein the β adrenergic antagonist is selected from the group consisting of β₁, β₂ and β₃ antagonist.

10. A composition that alters sympathetic tone in a mammal for use in the modulation of leptin effects on bone, in the modulation of bone mass or in treating or preventing a symptom of bone disease, wherein the pharmaceutical composition comprises a combination of a leptin antagonist and a β adrenergic antagonist.
